Europäisches Patentamt
European Patent Office
Office européen des brevets

(11) Veröffentlichungsnummer: **0 353 187 A2**

# EUROPÄISCHE PATENTANMELDUNG

(21) Anmeldenummer: **89810543.2**

(22) Anmeldetag: **18.07.89**

(51) Int. Cl.5: **C 07 D 213/61**
A 01 N 43/40, C 07 D 213/50,
C 07 D 213/70,
C 07 D 213/71,
C 07 D 213/65,
C 07 D 213/85,
C 07 D 213/78, C 07 D 213/79

(30) Priorität: **25.07.88 CH 2825/89 05.01.89 CH 29/89**

(43) Veröffentlichungstag der Anmeldung:
**31.01.90 Patentblatt 90/05**

(84) Benannte Vertragsstaaten:
**AT BE CH DE ES FR GB GR IT LI LU NL SE**

(71) Anmelder: **CIBA-GEIGY AG**
**Klybeckstrasse 141**
**CH-4002 Basel (CH)**

(72) Erfinder: **Brunner, Hans-Georg, Dr.**
**Wannenstrasse 14**
**CH-4415 Lausen (CH)**

(54) **Neue Herbizide.**

(57) Die Erfindung betrifft neue, herbizid wirksame Cyclohexandione der Formel I oder I'

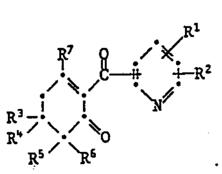

oder

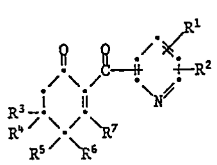

worin
$R^1$ und $R^2$ unabhängig voneinander Wasserstoff; Halogen; Nitro; Cyano; $C_1$-$C_4$-Alkyl; $C_1$-$C_4$-Alkoxy; $C_1$-$C_4$-Alkyl-$S(O)_n$-; $COR^8$; $C_1$-$C_4$-Halogenalkoxy; oder $C_1$-$C_4$-Halogenalkyl;
$R^3$, $R^4$ und $R^5$ unabhängig voneinander Wasserstoff; $C_1$-$C_4$-Alkyl; oder gegebenenfalls bis zu dreifach gleich oder verschieden durch Halogen, Nitro, Cyano, $C_1$-$C_4$-Alkyl, $C_1$-$C_4$-Alkoxy, $C_1$-$C_4$-Alkyl-$S(O)_n$-, $C_1$-$C_4$-Halogenalkyl, $C_1$-$C_4$-Halogenalkyl-$S(O)_n$- oder $C_1$-$C_4$-Halogenalkoxy substituiertes Phenyl oder Benzyl;
$R^6$ Wasserstoff; $C_1$-$C_4$-Alkyl, $C_1$-$C_4$-Alkoxycarbonyl; oder Cyano;
$R^7$ OH; oder $O^{\ominus}M^{\oplus}$;
$R^8$ OH; $C_1$-$C_4$-Alkoxy; $NH_2$; $C_1$-C4-Alkylamino; oder di-$C_1$-$C_4$-Alkylamino;
n 0, 1 oder 2;
$M^{\oplus}$ ein Kationenäquivalent eines Metallions oder eines gegebenenfalls bis zu dreifach durch $C_1$-$C_4$-Alkyl, $C_1$-$C_4$-Hydroxyalkyl-, oder $C_1$-$C_4$-Alkoxy-$C_1$-$C_4$-alkyl-gruppen substituierten Ammoniumions
bedeutet, herbizide Mittel, Verfahren zur Herstellung neuer Verbindungen sowie neue Zwischenprodukte und deren Herstellung.

EP 0 353 187 A2

**Beschreibung**

## Neue Herbizide

Die vorliegende Erfindung betrifft neue Cyclohexandione mit herbizider Wirkung, agrochemische Mittel, die diese Cyclohexandione enthalten, deren Verwendung zur Bekämpfung unerwünschten Pflanzenwachstums sowie Verfahren zur Herstellung der erfindungsgemässen Verbindungen. Ferner betrifft die Erfindung auch neue Zwischenprodukte und Verfahren zu deren Herstellung.

Es sind bereits zahlreiche substituierte Cyclohexan-1,3-dione mit herbizider Wirkung bekannt geworden. Diese Verbindungen befriedigen nicht immer im Hinblick auf Wirkungsstärke, Wirkdauer, Selektivität und Anwendbarkeit. Demgegenüber wurde überraschenderweise gefunden, dass die neuen Cyclohexan-1,3-dione der allgemeinen Formel I bzw. I′ gut herbizid wirksam sind.

Die Erfindung betrifft die neuen Cyclohexan-1,3-dione der Formel I oder I′

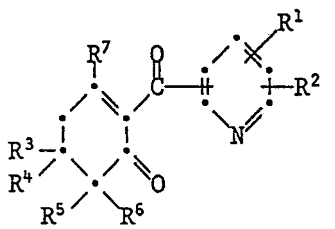

oder

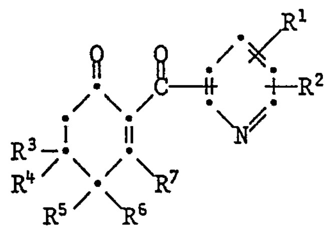

worin

$R^1$ und $R^2$ unabhängig voneinander Wasserstoff; Halogen; Nitro; Cyano; $C_1$-$C_4$-Alkyl; $C_1$-$C_4$-Alkoxy; $C_1$-$C_4$-Alkyl-$S(O)_n$-; $COR^8$; $C_1$-$C_4$-Halogenalkoxy; oder $C_1$-$C_4$-Halogenalkyl;

$R^3$, $R^4$ und $R^5$ unabhängig voneinander Wasserstoff; $C_1$-$C_4$-Alkyl; oder gegebenenfalls bis zu dreifach gleich oder verschieden durch Halogen, Nitro, Cyano, $C_1$-$C_4$-Alkyl, $C_1$-$C_4$-Alkoxy, $C_1$-$C_4$-Alkyl-$S(O)_n$-, $C_1$-$C_4$-Halogenalkyl, $C_1$-$C_4$-Halogenalkyl-$S(O)_n$- oder $C_1$-$C_4$-Halogenalkoxy substituiertes Phenyl oder Benzyl;

$R^6$ Wasserstoff; $C_1$-$C_4$-Alkyl, $C_1$-$C_4$-Alkoxycarbonyl; oder Cyano;

$R^7$ OH; oder $O^{\ominus}M^{\oplus}$;

$R^8$ OH; $C_1$-$C_4$-Alkoxy; $NH_2$; $C_1$-$C_4$-Alkylamino; oder di-$C_1$-$C_4$-Alkylamino;

n 0, 1 oder 2;

$M^{\oplus}$ ein Kationenäquivalent eines Metallions oder eines gegebenenfalls bis zu dreifach durch $C_1$-$C_4$-Alkyl, $C_1$-$C_4$-Hydroxyalkyl-, oder $C_1$-$C_4$-Alkoxy-$C_1$-$C_4$-alkyl-gruppen substituierten Ammoniumions

bedeutet.

In den in dieser Beschreibung verwendeten Definitionen umfassen die angegebenen generischen Begriffe, sowie die durch Kombination einzelner Unterbegriffe erhältlichen Substituenten, beispielsweise die folgenden spezifischen Einzelsubstituenten, wobei diese Aufzählung keine Einschränkung der Erfindung darstellt:

Alkyl: Methyl, Ethyl, n-Propyl, i-Propyl, n-Butyl, sek-Butyl, i-Butyl und tert-Butyl; vorzugsweise Methyl, Ethyl und i-Propyl.

Halogen: Fluor, Chlor, Brom und Jod; vorzugsweise Fluor, Chlor und Brom; besonders bevorzugt (für $R^1$ und $R^2$) Fluor und Chlor und Brom.

Halogenalkyl: Fluormethyl, Difluormethyl, Trifluormethyl, Chlormethyl, Dichlormethyl, Trichlormethyl, 2,2,2-Trifluorethyl, 2-Fluorethyl, 2-Chlorethyl und 2,2,2-Trichlorethyl; vorzugsweise Trichlormethyl, Difluorchlormethyl und Dichlorfluormethyl.

Alkoxy: Methoxy, Ethoxy, Propyloxy, i-Propyloxy, n-Butyloxy, i-Butyloxy, s-Butyloxy und t-Butyloxy; vorzugsweise Methoxy.

Halogenalkoxy: Fluormethoxy, Difluormethoxy, Trifluormethoxy, 2,2,2-Trifluorethoxy, 1,1,2,2-Tetrafluorethoxy, 2-Fluorethoxy, 2-Chlorethoxy und 2,2,2-Trichlorethoxy; vorzugsweise Difluormethoxy, 2-Chlorethoxy und Trifluormethoxy.

Alkoxycarbonyl: Methoxycarbonyl, Ethoxycarbonyl, 4-Propyloxycarbonyl, i-Propyloxycarbonyl und n-Butyloxycarbonyl; vorzugsweise Methoxycarbonyl und Ethoxycarbonyl.

Alkylthio: Methylthio, Ethylthio, Propylthio, Isopropylthio, n-Butylthio, i-Butylthio, s-Butylthio oder t-Butylthio; vorzugsweise Methylthio und Ethylthio.

Alkylsulfinyl: Methylsulfinyl, Ethylsulfinyl, n-Propylsulfinyl, i-Propylsulfinyl, n-Butylsulfinyl, sec-Butylsulfinyl, i-Butylsulfinyl; vorzugsweise Methylsulfinyl und Ethylsulfinyl.

Alkylsulfonyl: Methylsulfonyl, Ethylsulfonyl, n-Propylsulfonyl, i-Propylsulfonyl, n-Butylsulfonyl, sec-Butylsulfonyl, i-Butylsulfonyl; vorzugsweise Methyl- und Ethylsulfonyl.

Im Hinblick auf ihre chemische Struktur können die Verbindungen der Formel I als in Position 2 acylierte 1,3-Cyclohexandione angesehen werden. Von dieser Grundstruktur sind zahlreiche tautomere Formen ableitbar. Die Erfindung umfasst sämtliche Tautomere.

Die jeweils durch ein Semikolon voneinander abgetrennten Einzelbedeutungen der Substituenten $R^1$ bis $R^7$ sind als Untergruppen dieser Substituenten anzusehen. Die Erfindung umfasst auch die durch Streichung einer oder mehrerer dieser Untergruppen erhältlichen Definitionen der Verbindungen der Formel I.

Die Verbindungen der Formel I und I′ liegen in einem Gleichgewicht beider Formen gemäss nachstehender Gleichung vor:

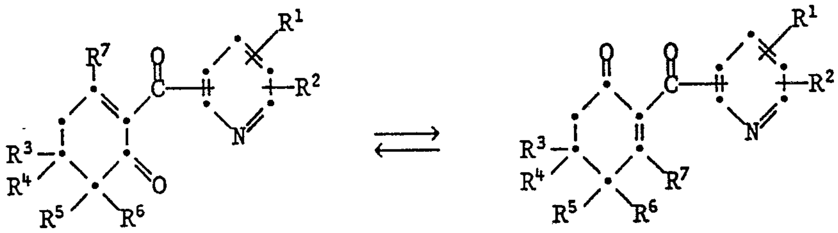

I I'

Im Falle der Hydroxyverbindungen ($R^7$ = OH) kann neben den drei Enolformen Ia, Ia‴ bzw. Ia′ auch die Triketoform Ia″ gemäss nachstehendem Tautomeriegleichgewicht auftreten:

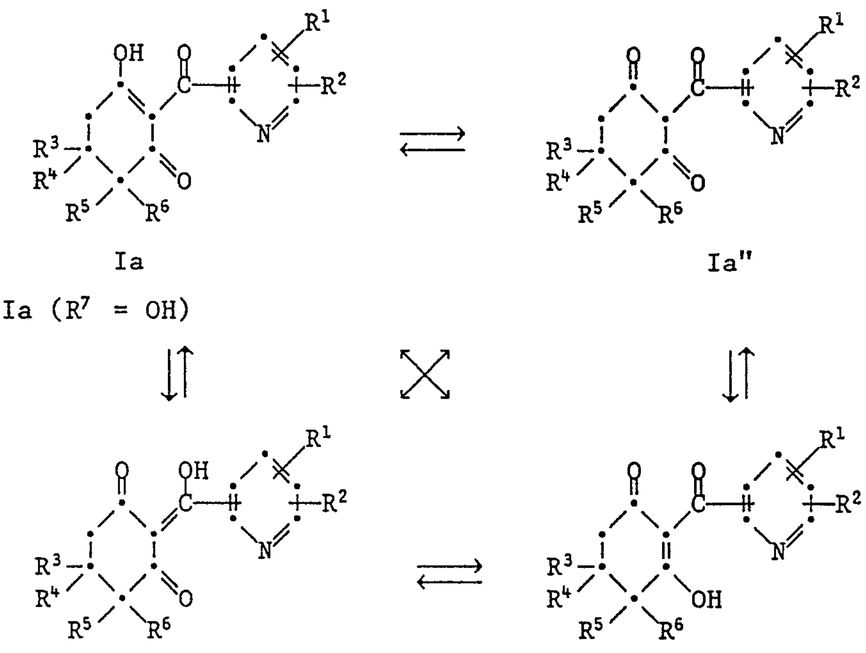

Ia Ia"

(Ia ($R^7$ = OH)

Ia''' Ia'

Die Erfindung umfasst sämtliche aus der Grundstruktur I bzw. I′ herleitbaren tautomeren Strukturen und deren Salze ($R^7 = O^{\ominus}M^{\oplus}$).

Darüber hinaus können die Verbindungen der Formel I bzw. I′ (insbesondere durch die Reste $R^3$ bis $R^6$ am Cyclohexansystem) unsymmetrisch substituiert sein. Die Erfindung umfasst sowohl das Racemat als auch die angereicherten und optisch reinen Formen der jeweiligen Stereoisomeren.

Die unsymmetrisch substituierten Verbindungen der Formel I fallen, sofern nicht chirale Edukte verwendet werden, im allgemeinen bei den in dieser Anmeldung beschriebenen Verfahren als Racemate an. Die Stereoisomeren können sodann nach an sich bekannten Methoden, wie etwa fraktionierter Kristallisation nach Salzbildung mit optisch reinen Basen, Säuren oder Metallkomplexen, oder aber durch chromatographische Verfahren aufgrund physikochemikalischer Eigenschaften aufgetrennt werden.

Sowohl das Racemat als auch die stereoisomeren Formen werden von der Erfindung umfasst.

Hervorzuheben sind die Verbindungen der Formel I oder I′

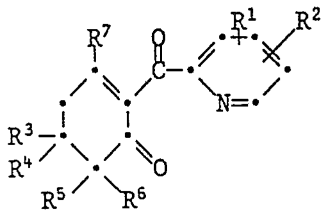

I

oder

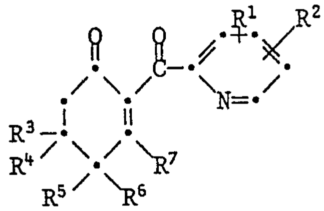

I'

worin der Pyridincarbonylrest über die Position 2 des Pyridinsystems gebunden ist.

Hervorzuheben sind weiterhin die Verbindungen der Formel I oder I′

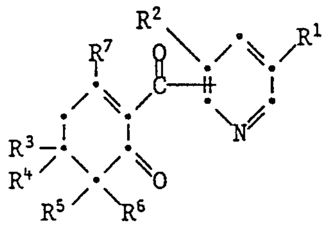

I

oder

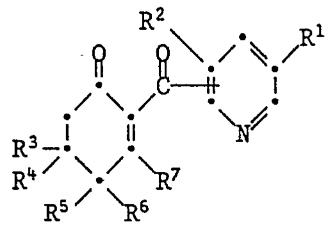

I'

worin die Reste $R^1$ und $R^2$ in den Positionen 3 und 5 des Pyridinsystems gebunden sind.

Bevorzugt sind die Verbindungen der Formel I oder I′

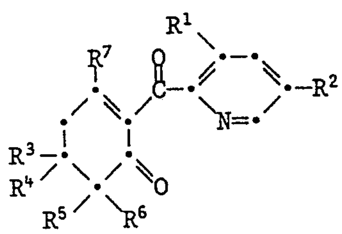

I

oder

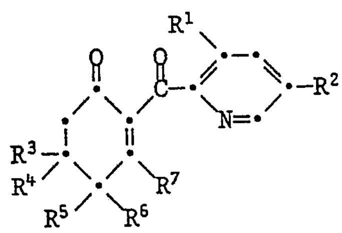

I'

worin die Reste $R^1$ und $R^2$ in den Positionen 3 und 5 des Pyridinringes und das Pyridincarbonylsystem über die Position 2 des Pyridinringes gebunden sind.

Insbesondere bevorzugt sind die Verbindungen der Formel I oder I′

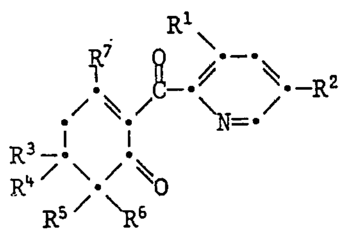

I

oder

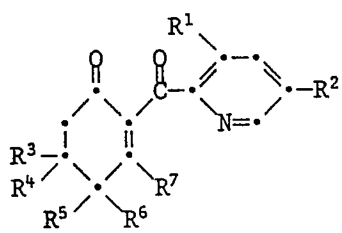

I'

worin

$R^1$ Wasserstoff; Halogen; Nitro; Cyano; $C_1$-$C_4$-Alkyl; $C_1$-$C_4$-Alkoxy; $C_1$-$C_4$-Alkyl-$S(O)_n$-; $COR^8$; $C_1$-$C_4$-Halogenalkoxy; oder $C_1$-$C_4$-Halogenalkyl;

$R^2$ Wasserstoff; Halogen; Nitro; Cyano; $C_1$-$C_4$-Alkyl; $C_1$-$C_4$-Alkoxy; $C_1$-$C_4$-Halogenalkoxy; $C_1$-$C_4$-Alkyl-$S(O)_n$-; oder $C_1$-$C_4$-Halogenalkyl;

$R^3$, $R^4$ und $R^5$, unabhängig voneinander; Wasserstoff; $C_1$-$C_4$-Alkyl; oder gegebenenfalls bis zu dreifach gleich oder verschieden durch Halogen, Nitro, Cyano, $C_1$-$C_4$-Alkyl, $C_1$-$C_4$-Alkoxy, $C_1$-$C_4$-Alkyl-$S(O)_n$-, $C_1$-$C_4$-Halogenalkyl, $C_1$-$C_4$-Halogenalkyl-$S(O)_n$- oder $C_1$-$C_4$-Halogenalkoxy substituiertes Phenyl oder Benzyl;

$R^6$ Wasserstoff; $C_1$-$C_4$-Alkoxycarbonyl; oder Cyano;

$R^7$ OH; oder $O^{\ominus}M^{\oplus}$

$R^8$ OH; $C_1$-$C_4$-Alkoxy; $NH_2$; $C_1$-$C_4$-Alkylamino; oder di-$C_1$-$C_4$-Alkylamino;

n 0, 1 oder 2;

$M^{\oplus}$ ein Kationenäquivalent eines Alkali-, Erdalkali- oder Ammoniumions; Mono-$C_1$-$C_4$-alkylammonium;

Di-$C_1$-$C_4$-alkylammonium-; Tri-$C_1$-$C_4$-alkylammonium-; oder Triethanolammoniumions; bedeutet.

Besonders hervorzuheben sind bei den vorgenannten Verbindungen der Formel I oder I′ im Umfang der breitesten generischen Bedeutung wie auch bei den hervorgehobenen, bevorzugten und besonders generischen Definitionen jeweils nachstehend genannte Untergruppen:

a) Verbindungen der Formel I oder I′, in denen mindestens einer der Reste $R^3$ bis $R^6$ Wasserstoff bedeutet,

b) Verbindungen der Formel I oder I′, in denen mindestens zwei der Reste $R^3$ bis $R^6$ Wasserstoff bedeuten,

c) Verbindungen der Formel I oder I′, in denen $R^6$ Cyan und $R^5$ Wasserstoff bedeutet,

d) Verbindungen der Formel I oder I′, in denen $R^6$ Cyan, $R^5$ Wasserstoff und $R^3$ und $R^4$ unabhängig voneinander Wasserstoff oder $C_1$-$C_4$-Alkyl bedeutet,

e) Verbindungen der Formel I oder I′ in denen $R^6$ $C_1$-$C_4$-Alkoxycarbonyl bedeutet,

f) Verbindungen der Formel I oder I′, in denen $R^7$ OH bedeutet,

g) Verbindungen der Formel I oder I′, in denen $R^7$ $O^{\ominus}M^{\oplus}$ bedeutet,

h) Verbindungen der Formel I oder I′, in denen $R^1$ Wasserstoff, Chlor, Fluor, Nitro, Trifluormethyl, Methoxy, Brom, Methylthio, Methylsulfonyl, Carboxy, Trichlormethyl oder Methyl bedeutet,

i) Verbindungen der Formel I und I′, in denen $R^2$ Wasserstoff, Chlor, Nitro, Methylthio, Methylsulfinyl, Methylsulfonyl, Methyl, Fluor, Trifluormethyl oder Trichlormethyl bedeutet.

Insbesondere sind hervorzuheben Kombinationen der Untergruppen a) bis e) mit h) und i) sowohl als freie Säure (Gruppe f)) als auch als Salze (Gruppe g)).

Besonders bevorzugt sind die Verbindungen der Formel I oder I′ worin

$R^1$ Wasserstoff; Fluor; Chlor; Brom; Nitro; Cyano; Methyl; Trifluormethyl; Trichlormethyl; Methoxy; Methylthio; Methylsulfinyl; Methylsulfonyl Carboxy; Carbamoyl; Methoxycarbonyl; oder Ethoxycarbonyl;

$R^2$ Wasserstoff; Fluor; Chlor; Nitro; Trifluormethyl; Trichlormethyl; Methylthio; Methylsulfinyl; oder Methylsulfonyl;

$R^3$ Wasserstoff; $C_1$-$C_3$-Alkyl, Phenyl; Benzyl; oder Chlorphenyl;

$R^4$ Wasserstoff; oder Methyl;

$R^5$ Wasserstoff; oder Methyl;

$R^6$ Wasserstoff; Cyan; Methyl; oder $C_1$-$C_2$-Alkoxycarbonyl;

$R^7$ OH; oder $O^{\ominus}M^{\oplus}$

$M^{\oplus}$ ein Kationenäquivalent des Natrium-, Lithium-, Calcium-, Trimethylammonium- oder Triethanolammoniumions

bedeuten.

Als Einzelverbindungen zu nennen sind 2-(3-Chlor-5-trifluormethyl-pyridin-2-yl-carbonyl)-cyclohex-1-en-1-ol-3-on

und

2-(3-Chlor-5-methylsulfonyl-pyridin-2-yl-carbonyl)-cyclohex-1-en-1-ol-3-on.

Die Verbindungen der Formel Ia oder Ia′, worin die Reste $R^1$ bis $R^6$ wie zuvor definiert sind und $R^7$ OH bedeutet können hergestellt werden durch

a) Umsetzung von Cyclohexandionen der Formel II, worin die Reste $R^3$ bis $R^6$ wie zuvor definiert sind, mit einem Pyridin der Formel III, worin $R^1$ und $R^2$ wie zuvor definiert sind und X Halogen, vorzugsweise Chlor oder Brom, den Rest

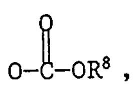

oder

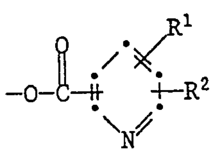

und $R^8$ $C_1$-$C_4$-Alkyl, Phenyl oder Benzyl bedeutet, in Gegenwart einer Base umsetzt

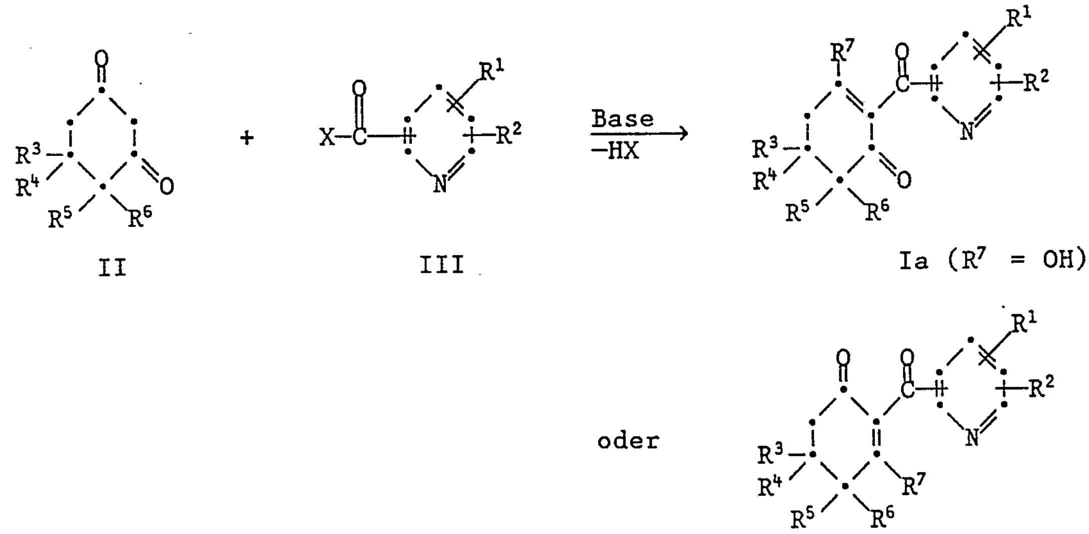


II III Ia ($R^7$ = OH)

oder

Ia' ($R^7$ = OH) .

oder

b) thermische Umlagerung eines Esters der Formel IV oder IV′, worin die Reste $R^1$ bis $R^6$ wie zuvor definiert sind

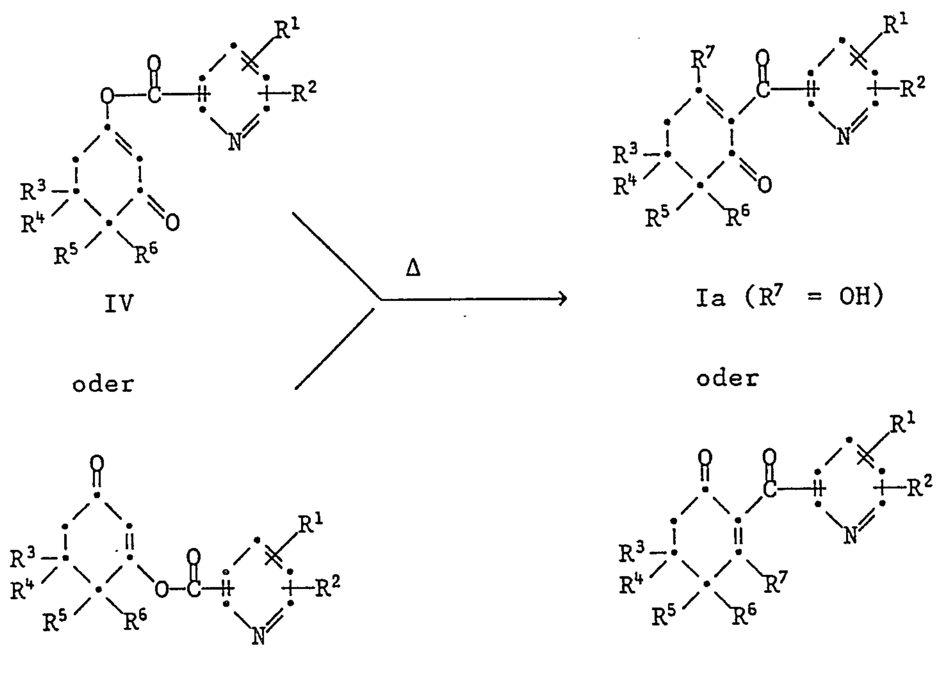


IV

oder

Ia ($R^7$ = OH)

oder

IV'

Ia' ($R^7$ = OH)

vorzugsweise in Gegenwart von Cyanid.

Die Salze der Formel Ib bzw. Ib′, worin die Reste $R^1$ bis $R^6$ wie zuvor definiert sind, und $R^7$ $O^{\ominus}M^{\oplus}$ bedeutet, können hergestellt werden durch

c) Umsetzung eines Cyclohexandions Ia oder Ia′, worin $R^1$ bis $R^6$ wie zuvor definiert sind, und $R^7$ OH bedeutet mit einer Base V, worin B $OH^{\ominus}M^{\oplus}$ bedeutet und $M^{\oplus}$ wie zuvor definiert ist

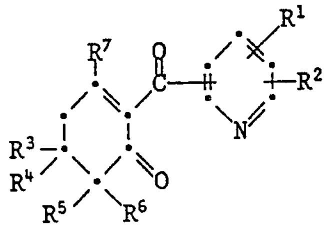

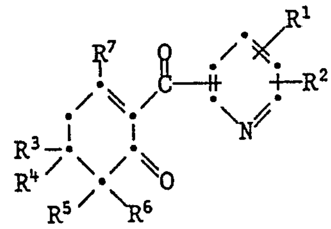

Ia ($R^7$ = OH) + B V Ib ($R^7$ = $O^{\ominus}M^{\oplus}$)

oder

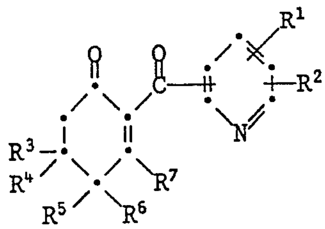

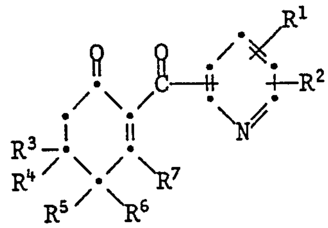

Ia' ($R^7$ = OH) Ib' ($R^7$ = $O^{\ominus}M^{\oplus}$)

Die Ester der Formel IV bzw. IV' sind wertvolle Zwischenverbindungen zur Herstellung der herbiziden Endprodukte Ia bzw. Ia' gemäss Verfahrensvariante b). Die Erfindung betrifft somit auch die neuen Ester IV oder IV'. Die Ester IV oder IV' können aber auch als Nebenprodukte bei der Acylierung gemäss Verfahrensvariante a) entstehen.

d) Weiterhin können die Verbindungen der Formel Ic oder Ic' worin einer oder mehrere der Reste $R^1$ bis $R^6$ für $C_1$-$C_4$-Alkyl-$S(O)_n$- mit n = 1 oder 2 steht, und die übrigen Reste wie zuvor definiert sind, hergestellt werden durch Oxidation eines Thioethers der Formel Id oder Id', worin der zu oxidierende Rest aus der Gruppe $R^1$ bis $R^6$ $C_1$-$C_4$-Alkyl-$S(O)_n$- mit n = O bedeutet und die übrigen Reste wie zuvor definiert sind

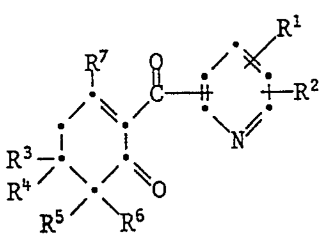

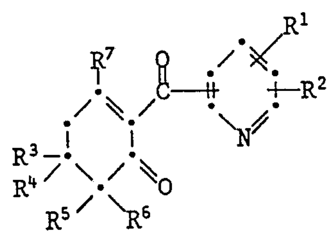

Id (Thioether mit n = 0) Oxidation Ic (Sulfinyl- oder Sulfonylverbindung mit n = 1 oder 2)

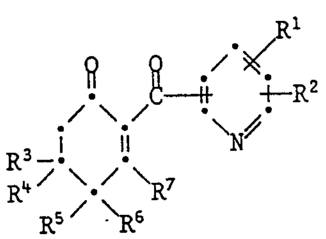

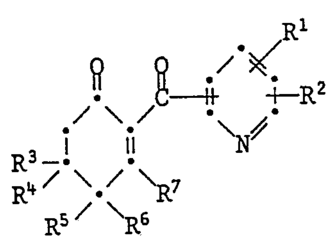

Id' (Thioether mit n = 0) Ic' (Sulfinyl- oder Sulfonylverbindung mit n = 1 oder 2)

Derartige Oxidationen sind dem Fachmann geläufig (z.B. Methodicum Chimicum, Ed. F. Korte, 9. Thieme Verlag Stuttgart 1976, Bd. 7 Seiten 696-698 und die dort genannten Literaturstellen für die Oxidation zu

Sulfenen und Bd. 7, Seiten 751-755 und die dort genannte Literatur für die Oxidation zu Sulfonen).

Bevorzugt ist die Oxidation mit $H_2O_2$ und mit Persäuren, insbesondere mit 3-Chlorperbenzoesäure.

Durch geeignete Wahl von Basen, Lösungsmittel sowie weiterer Reaktionsparameter, wie Temperatur, Konzentration etc. kann die O-Acylierung gemäss nachstehendem Schema zur Hauptreaktion werden:

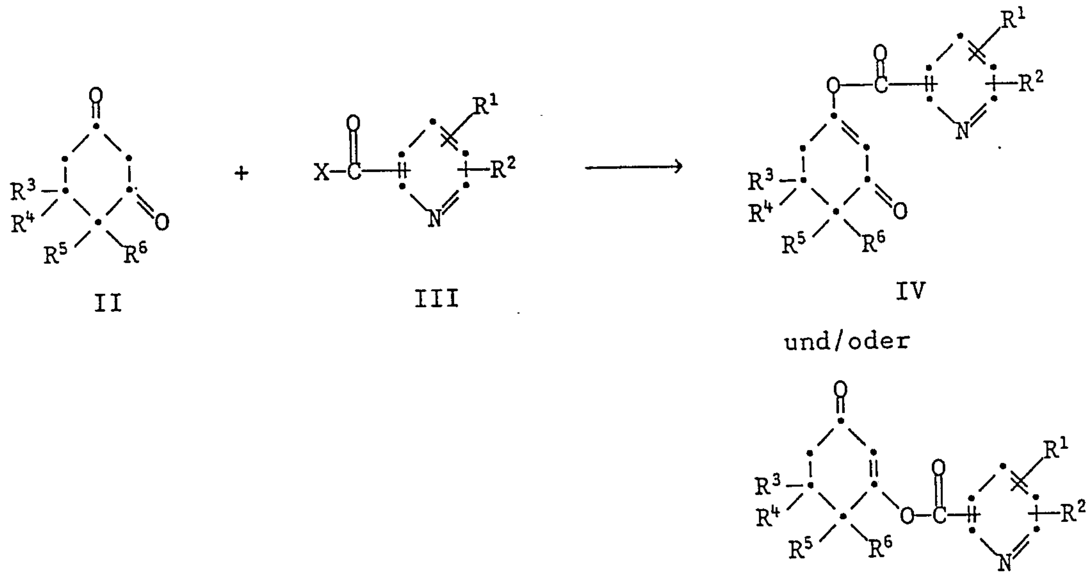

II

III

IV

und/oder

IV'

Bei der C-Acylierung gemäss Verfahrensvariante a) hat es sich als vorteilhaft erwiesen, die Umsetzung in Gegenwart geringer Mengen von Cyanid zu arbeiten. Eine geringe Cyanidkonzentration kann beispielsweise durch die Zugabe von Acetoncyanhydrin sichergestellt werden.

Die Umlagerung der Ester IV bzw. IV′ kann ebenfalls in vorteilhafter Weise unter Einwirkung von Cyanidionen sowie in Gegenwart einer Base erfolgen.

Obwohl die als Reaktion a) skizzierte Synthese der Verbindungen der Formel Ia bzw. Ia′ ein Verfahren beschreibt, mit welchem prinzipiell sämtliche von Formel Ia bzw. Ia′ umfassten Verbindungen herstellbar sind, kann es aus ökonomischen oder verfahrenstechnischen Gründen sinnvoll sein, bestimmte Verbindungen der Formel Ia bzw. Ia′ in andere, von Formel I bzw. I′ umfasste Derivate, zu überführen. Beispiele für derartige Umwandlungen sind, neben der Reaktion c) und d), zum Beispiel Verfahren, bei denen $R^6$ für Ester-, Halogen- oder Cyanoradikal steht. Diese Radikale können analog zu den nachstehend in Schema 1 gezeigten Umsetzungen (IIa → IIb, IIc oder IId) auch noch auf der Stufe der Verbindungen der Formel I durchgeführt werden. Derartige Derivatisierungsreaktionen sind dem Fachman geläufig.

Mit Vorteil führt man die obigen Umsetzungen in einem reaktionsinerten Lösungsmittel aus. Dabei kommen als inerte Lösungsmittel, Kohlenwasserstoffe, wie Benzol, Toluol oder Xylol; Ether, wie Diethylether, Methylisopropylether, Glyme, Diglyme; cyclische Ether, wie Tetrahydrofuran und Dioxan; Ketone, wie Aceton, Methylethylketon; Amide, wie Dimethylformamid, N-Methylpyrrolidon; Sulfoxide, wie Dimethylsulfoxid; oder chlorierte Kohlenwasserstoffe, wie Dichlormethan, Trichlormethan, Tetrachlormethan oder Tetrachlorethan, Alkohole, wie Methanol, Ethanol, Isopropanol, Propanol, Butanol etc. in Betracht.

Auch können mit Vorteil in einigen Fällen Lösungsmittelgemische als organische Lösungsmittel in Mischung mit Wasser Verwendung finden.

Die Reaktionstemperatur kann in weiten Grenzen variiert werden. Geeignete Reaktionstemperaturen liegen beispielsweise zwischen -20°C und der Rückflusstemperatur des Reaktionsgemisches. Vorzugsweise wird die Umsetzung bei einer Temperatur zwischen 0°C und 100°C durchgeführt.

Im Fall der Reaktionen a) und b) ist es vorteilhaft unter Basenzusatz zu arbeiten. Geeignete Basen sind u.a. Natrium-, Kalium- und Calciumhydroxid, Alkali- und Erdalkalicarbonate, Amine, wie etwa Triethylamin oder Heterocyclen, wie Pyridin, 4-Dimethylaminopyridin, DABCO sowie Alkalimetallhydride.

Die Reaktion a) und b) können auch vorteilhaft unter Phasentransfer-Bedingungen in Zweiphasensystemen durchgeführt werden. Derartige Reaktionen sind dem Fachman geläufig (z.B. beschrieben in Dehmlow und Dehmlow, Phase Transfer Catalysis, Verlag Chemie, Weinheim 1983; W.E. Keller, Phase Transfer Reactions Vol. 1 und Vol. 2, G. Thieme Verlag, Stuttgart 1986, 1987).

Die Cyclohexandione der Formel II sind entweder bekannt oder sie können analog zu literaturbekannten Verfahren hergestellt werden.

Einen generellen Zugang zu den Cyclohexandionen II ermöglicht nachstehende Malonestersynthese, in der zunächst gemäss nachstehendem Schema aus einem Aldehyd oder Keton VI und Aceton die spezifisch substituierten Cyclohexandione IIa, IIb oder IIc herstellbar;

Schema 1

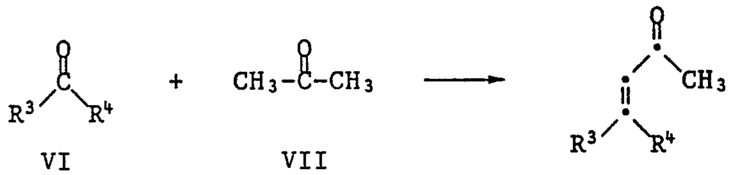

VI VII VIII

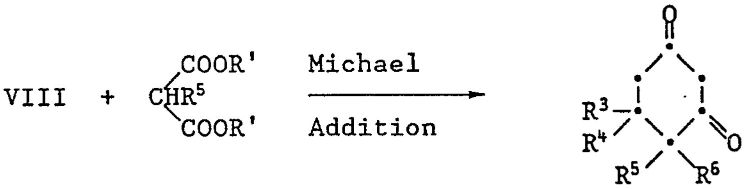


IIa ($R^6$ = COOR', R' = $C_1$-$C_4$-Alkyl)

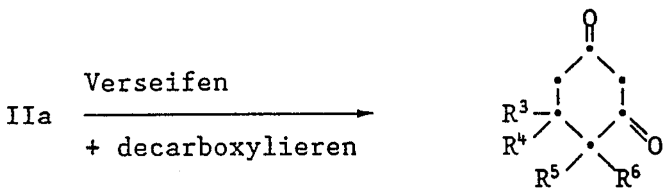


IIb ($R^6$ = H)

Die Verbindungen der Formel IIc, in denen die Reste $R^3$ bis $R^5$ wie zuvor definiert sind und $R^6$ Cyan bedeutet, können in Abwandlung des Reaktionsschema 1 durch Michael-Addition von Cyanessigsäureester XIII, worin $R^5$ wie zuvor definiert ist und R′ $C_1$-$C_4$-Alkyl bedeutet an das Keton VIII, worin $R^3$ und $R^4$ wie zuvor definiert sind, hergestellt werden.

Schema 2

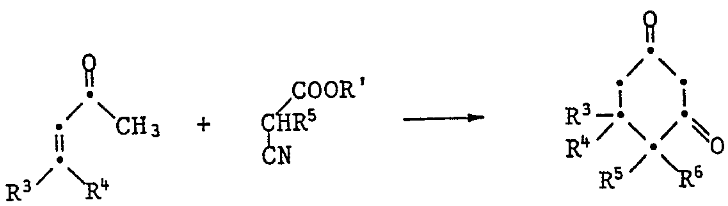

VIII XIII IIc ($R^6$ = CN)

Bei den Pyridincarbonsäurederivaten III sind insbesondere die Säurechloride bevorzugt.

Das Pyridin-2-carbonsäurechlorid IIIa kann in vorteilhafter Weise durch eine Pd-katalysierte Carbonylierungsreaktion gemäss nachstehendem Reaktionsschema 3 hergestellt werden:

Schema 3

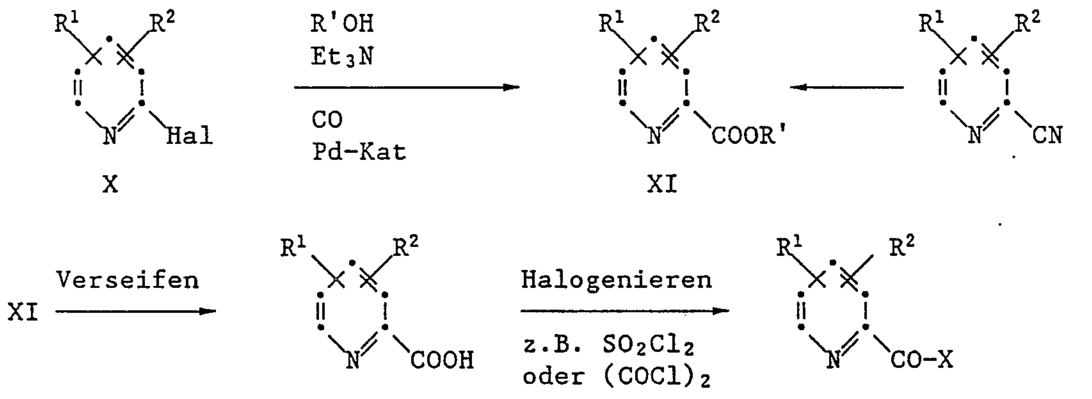


X, XI, XIV, IIIa (X = Halogen)

In obigem Schema ist Hal Halogen (in erster Linie Chlor); R′ $C_1$-$C_4$-Alkyl und Pd-Kat ist vorzugsweise $PdCl_2(TPP)_2$ ein Triphenylphoshpinkomplex des Palladiums.

Die in Schema 3 genannten Picolinsäurederivate XI, IIIa und XIV sind wertvolle Zwischenprodukte für die Synthese der erfindungsgemässen Cyclohexandione I. Zum überwiegenden Teil sind diese Verbindungen neu.

Die Erfindung betrifft somit auch die neuen Picolinsäurederivate der Formel XV

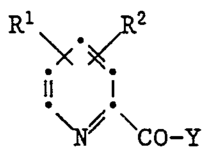

XV,

worin
Y OH; $C_1$-$C_4$-Alkoxy; oder Halogen; und
$R^1$ und $R^2$ unabhängig voneinander Halogen, Nitro; Cyano; $C_1$-$C_4$-Halogenalkyl; $C_1$-$C_4$-Alkyl; $C_1$-$C_4$-Alkoxy; oder $C_1$-$C_4$-Alkyl-$S(O)_n$-; und
n 0; 1; oder 2;
bedeutet,
mit der Massgabe, dass wenn Y Chlor bedeutet und der Rest $R^1$ in Position 3 und der Rest $R^2$ in Position 5 gebunden ist,
$R^1$ und $R^2$ nicht beide zusammen Chlor oder beide zusammen Methyl oder wenn $R^1$ für Nitro steht $R^2$ nicht Methyl bedeutet.

Bevorzugt sind die Picolinsäurederivate der Formel XV′,

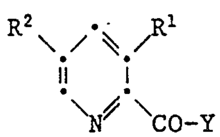

XV′,

worin
Y OH; $C_1$-$C_4$-Alkoxy; oder Halogen; und
$R^1$ und $R^2$ unabhängig voneinander Halogen, Nitro; Cyano; $C_1$-$C_4$-Halogenalkyl; $C_1$-$C_4$-Alkyl; $C_1$-$C_4$-Alkoxy; oder $C_1$-$C_4$-Alkyl-$S(O)_n$-; und
n 0; 1; oder 2;
bedeutet,
mit der Massgabe, dass wenn Y Chlor bedeutet, $R^1$ und $R^2$ nicht beide zusammen Chlor oder beide zusammen Methyl oder $R^1$ Nitro und $R^2$ Methyl bedeutet.

Besonders bevorzugt sind die Verbindungen der Formel XV′, worin
Y OH; $C_1$-$C_4$-Alkoxy; oder Halogen;
$R^1$ Wasserstoff; und
$R^2$ $C_1$-$C_4$-Alkoxy, $C_1$-$C_4$-Halogenalkyl; oder Cyano bedeutet.

Ebenfalls besonders bevorzugt sind die Verbindung der Formel XV′, worin
Y OH; $C_1$-$C_4$-Alkoxy; oder Halogen;
$R^1$ $C_1$-$C_4$-Alkoxy; $C_1$-$C_4$-Halogenalkyl; oder Cyano; und
$R^2$ Waserstoff
bedeutet.

Hervorzuheben sind bei den Verbindungen der Formel XV sowie den als bevorzugt und besonders bevorzugt genannten Verbindungen XV′, die Säurechloride, das heisst diejenigen Verbindungen, in denen Y Chlor bedeutet.

Die Verbindungen der Formel I sind hochaktive Pflanzenwirkstoffe, welche sich bei geeigneten Aufwandmengen hervorragend als Selektivherbizide zur Unkrautbekämpfung in Nutzpflanzenkulturen eignen. Das heisst, bei diesen Aufwandmengen zeichnen sich die Wirkstoffe der Formel I durch gute selektiv-hebizide Eigenschaft gegen Unkräuter aus. Insbesondere Getreide, wie Roggen, Gerste, Hafer, Weizen und Mais aber auch andere Kulturpflanzen, wie Hirse, Reis, Baumwolle, Zuckerrohr oder Soja oder auch Dauerkulturen (wie etwa Reben oder Plantagen) bleiben bei niedrigen Aufwandmengen praktisch ungeschädigt. Bei gesteigerten Aufwandmengen werden die Kulturpflanzen nur geringfügig in ihrem Wachstum beeinflusst. Werden sehr hohe Aufwandmengen appliziert, entfalten die Substanzen der Formel I totalherbizide Eigenschaften. Die Aufwandmengen betragen in der Regel 0,001 bis 4 kg vorzugsweise 0,005 bis 2 kg Aktiosubstanz je Hektar.

Bei hohen Aufwandmengen können die Verbindungen der Formel I auch als Totalherbicide eingesetzt werden. Sie sind insbesondere geeignet zur Unkrautbekämpfung auf Wegen, Plätzen, Geleisanlagen oder sonstiger Flächen, auf denen eine vollständige Abtötung der dort wachsenden Pflanzen gewünscht ist.

Die selektiv-herbizide Wirkung der erfindungsgemässen Verbindungen wird sowohl bei der preemergenten als auch der postemergenten Anwendung festgestellt. Diese Wirkstoffe können daher im Vorauflaufverfahren und im Nachauflaufverfahren zur selektiven Unkrautbekämpfung gleichermassen mit gutem Erfolg verwendet werden.

In vorteilhafter Weise können die erfindungsgemässen Wirkstoffe oder Mittel auch auf das Vermehrungsgut der Kulturpflanze aufgebracht werden. Besonders zu erwähnen ist hier die Samenbeizung. Vermehrungsgut sind Samen, Stecklinge oder sonstige Teile der Pflanze, aus denen die Kulturpflanze gezogen werden kann. Das mit einer wirksamen Menge einer Verbindung der Formel I behandelte Vermehrungsgut ist ebenfalls Gegenstand der Erfindung.

Die Erfindung betrifft auch herbizide Mittel, welche einen neuen Wirkstoff der Formel I enthalten, sowie Verfahren zur pre- und postemergenten Unkrautbekämpfung.

Die Verbindungen der Formel I werden in unveränderter Form oder vorzugsweise als Mittel zusammen mit den in der Formulierungstechnik üblichen Hilfsmitteln eingesetzt und werden daher z.B. zu Emulsionskonzentraten, direkt versprühbaren oder verdünnbaren Lösungen, verdünnten Emulsionen, Spritzpulvern, löslichen Pulvern, Stäubemitteln, Granulaten, auch Verkapselungen in z.B. polymeren Stoffen in bekannter Weise verarbeitet. Die Anwendungsverfahren wie Versprühen, Vernebeln, Verstäuben, Verstreuen oder Giessen werden gleich wie die Art der Mittel den angestrebten Zielen und den gegebenen Verhältnissen entsprechend gewählt.

Die Formulierungen, d.h. die den Wirkstoff der Formel I und gegebenenfalls einen oder mehrere feste oder flüssige Streckmittel oder Zusatzstoff enthaltende Mittel, Zubereitungen oder Zusammensetzungen werden in bekannter Weise hergestellt, z.B. durch inniges Vermischen und/oder Vermahlen der Wirkstoffe mit Streckmitteln, wie z.B. mit Lösungsmitteln, flüssigen festen Trägerstoffen, und gegebenenfalls oberflächenaktiven Verbindungen (Tensiden).

Als Lösungsmittel wie auch als Streckmittel können in Frage kommen: Aromatische Kohlenwasserstoffe, bevorzugt die Fraktionen $C_8$ bis $C_{12}$, wie z.B. Xylolgemische oder substituierte Naphthaline, Phthalsäureester wie Dibutyl- oder Dioctylphthalat, aliphatische Kohlenwasserstoffe wie Cyclohexan oder Paraffine, Alkohole und Glykole sowie deren Ether und Ester, wie Ethanol, Ethylenglykol, Ethylenglykolmonomethyl- oder -ethylether Ketone wie Cyclohexanon, stark polare Lösungsmittel wie N-Methyl-2-pyrrolidon, Dimethylsulfoxid oder Dimethylformamid, sowie gegebenenfalls epoxidierte Pflanzenöle, wie epoxidiertes Kokosnussöl oder Sojaöl oder Wasser.

Als feste Trägerstoffe, z.B. für Stäubemittel und dispergierbare Pulver, werden in der Regel natürliche Gesteinsmehle verwendet, wie Calcit, Talkum, Kaolin, Montmorillonit oder Attapulgit. Zur Verbesserung der physikalischen Eigenschaften können auch hochdisperse Kieselsäure oder hochdisperse saugfähige Polymerisate zugesetzt werden. Als gekörnte, adsorptive Granulatträger kommen poröse Typen wie z.B. Bimsstein, Ziegelbruch, Sepiolit oder Bentonit, als nicht sorptive Trägermaterialien z.B. Calcit oder Sand in Frage. Darüberhinaus kann eine Vielzahl von vorgranulierten Materialien anorganischer oder organischer Natur wie insbesondere Dolomit oder zerkleinerte Pflanzenrückstände verwendet werden.

Als oberflächenaktive Verbindungen kommen je nach der Art des zu formulierenden Wirkstoffes der Formel I nichtionogene, kation-und/oder anionaktive Tenside mit guten Emulgier-, Dispergier- und Netzeigenschaften in Betracht. Unter Tensiden sind auch Tensidgemische zu verstehen.

Geeignete anionische Tenside können sowohl sog. wasserlösliche Seifen als auch wasserlösliche synthetische oberflächenaktive Verbindungen sein.

Als Seifen seien die Alkali-, Erdalkali- oder gegebenenfalls substituierte Ammoniumsalze von höheren Fettsäuren ($C_{10}$-$C_{22}$), wie z.B. die Na- oder K-Salze der Oel- oder Stearinsäure, oder von natürlichen Fettsäuregemischen, die z.B. aus Kokosnuss- oder Talgöl gewonnen werden können, genannt. Ferner sind auch die Fettsäuremethyl-taurinsalze zu erwähnen.

Häufiger werden jedoch sogenannte synthetische Tenside verwendet, insbesondere Fettsulfonate, Fettsulfate, sulfonierte Benzimidazolderivate oder Alkylarylsulfonate.

Die Fettsulfonate oder -sulfate liegen in der Regel als Alkali-, Erdalkali- oder gegebenenfalls substituierte Ammoniumsalze vor und weisen einen Alkylrest mit 8 bis 22 C-Atomen auf, wobei Alkyl auch den Alkylteil von

Acylresten einschliesst, z.B. das Na- oder Ca-Salz der Ligninsulfonsäure, des Dodecylschwefelsäureesters oder eines aus natürlichen Fettsäuren hergestellten Fettalkoholsulfatgemisches.

Hierher gehören auch die Salze der Schwefelsäureester und Sulfonsäuren von Fettalkohol-Ethylenoxid-Addukten. Die sulfonierten Benzimidazolderivate enthalten vorzugsweise 2-Sulfonsäuregruppen und einen Fettsäurerest mit 8 bis 22 C-Atomen. Alkylarylsulfonate sind z.B. die Na-, Ca- oder Triethanolaminsalze der Dodecylbenzolsulfonsäure, der Dibutylnaphthalin sulfonsäure oder eines Naphthalinsulfonsäure-Formaldehydkondensationsproduktes.

Ferner kommen auch entsprechende Phosphate wie z.B. Salze des Phosphorsäureesters eines p-Nonylphenol-(4-14)-Ethylenoxid-Adduktes oder Phospholipide in Frage.

Als nicht ionische Tenside kommen in erster Linie Polyglykoletherderivate von aliphatischen oder cycloaliphatischen Alkoholen, gesättigten oder ungesättigten Fettsäuren und Alkylphenolen in Frage, die 3 bis 10 Glykolethergruppen und 8 bis 20 Kohlenstoffatome im (aliphatischen) Kohlenwasserstoffrest und 6 bis 18 Kohlenstoffatome im Alkylrest der Alkylphenole enthalten können.

Weitere geeignete nichtionische Tenside sind die wasserlöslichen, 20 bis 250 Ethylenglykolethergruppen und 10 bis 100 Propylenglykolethergruppen enthaltenden Polyethylenoxidaddukte an Polypropylenglykol, Ethylendiaminopolypropylenglykol und Alkylpolypropylenglykol mit 1 bis 10 Kohlenstoffatomen in der Alkylkette, die genannten Verbindungen enthalten üblicherweise pro Propylenglykol-Einheit 1 bis 5 Ethylenglykoleinheiten.

Als Beispiele nichtionischer Tenside seien Nonylphenolpolyethoxyethanole, Ricinusölpolyglykolether, Polypropylen-Polyethylenoxidaddukte, Tributylphenoxypolyethoxyethanol, Polyethylenglykol und Octylphenoxypolyethoxyethanol erwähnt.

Ferner kommen auch Fettsäureester von Polyoxyethylensorbitan wie das Polyoxyethylensorbitan-trioleat in Betracht.

Bei den kationischen Tenside handelt es sich vor allem um quaternäre Ammoniumsalze, welche als N-Substituenten mindestens einen Alkylrest mit 8 bis 22 C-Atomen enthalten und als weitere Substituenten niedrige, gegebenenfalls halogenierte Alkyl-, Benzyl- oder niedrige Hydroxyalkylreste aufweisen. Die Salze liegen vorzugsweise als Halogenide, Methylsulfate oder Ethylsulfate vor, z.B. das Stearyltrimethylammoniumchlorid oder das Benzyldi(2-chlorethyl)-ethylammoniumbromid.

Die in der Formulierungstechnik gebräuchlichen Tenside sind u.a. in folgenden Publikationen beschrieben: "1986 International Mc Cutcheon's Emulsifiers & Detergents" Glen Rock, N.J., USA, 1986; H. Stache, "Tensid-Taschenbuch", 2. Auflage., C. Hanser Verlag, München, Wien, 1981;

M. and J. Ash. "Encyclopedia of Surfactants", Vol. I-III, Chemical Publishing Co., New York, 1980-1981.

Die Wirkstoffzubereitungen enthalten in der Regel 0,1 bis 95 %, insbesondere 0,1 bis 80 % Wirkstoff der Formel I, 1 bis 99,9 % eines oder mehrerer fester oder flüssiger Zusatzstoffe und 0 bis 25 % eines Tensides.

Insbesondere setzen sich bevorzugte Formulierungen folgendermassen zusammen: (% = Gewichtsprozent).

Emulgierbare Konzentrate:

| | |
|---|---|
| Aktiver Wirkstoff: | 1 bis 20 %, bevorzugt 5 bis 10 % |
| oberflächenaktive Mittel: | 5 bis 30 %, vorzugsweise 10 bis 20 % |
| flüssige Trägermittel: | 50 bis 94 %, vorzugsweise 70 bis 85 % |
| Stäube: | |
| Aktiver Wirkstoff: | 0,1 bis 10 %, vorzugsweise 0,1 bis 1 % |
| festes Trägermittel: | 99,9 bis 90 %, vorzugsweise 99,9 bis 99 % |
| Suspensions-Konzentrate: | |
| Aktiver Wirkstoff: | 5 bis 75 %, vorzugsweise 10 bis 50 % |
| Wasser: | 94 bis 25 %, vorzugsweise 88 bis 30 % |
| oberflächenaktives Mittel: | 1 bis 40 %, vorzugsweise 2 bis 30 % |
| Benetzbares Pulver: | |
| Aktiver Wirkstoff: | 0,5 bis 90 %, vorzugsweise 1 bis 80 % |
| oberflächenaktives Mittel: | 0,5 bis 20 %, vorzugsweise 1 bis 15 % |
| festes Trägermittel: | 5 bis 95 %, vorzugsweise 15 bis 90 % |
| Granulate: | |
| Aktiver Wirkstoff: | 0,5 bis 30 %, vorzugsweise 3 bis 15 % |
| festes Trägermittel: | 99,5 bis 70 %, vorzugsweise 97 bis 85 % |

Während als Handelsware eher konzentrierte Mittel bevorzugt werden, verwendet der Endverbraucher in der Regel verdünnte Mittel. Die Anwendungsformen können bis hinab zu 0,001 % an Wirkstoff verdünnt werden.

Die Mittel können auch weitere Zusätze wie Stabilisatoren, Entschäumer, Viskositätsregulatoren, Bindemittel, Haftmittel sowie Dünger oder andere Wirkstoffe zur Erzielung spezieller Effekte enthalten.

Die nachfolgenden Beispiele erläutern die Erfindung.

H. Herstellungsbeispiele

H.1. Verbindungen der Formel I

H.1.1. Umsetzungen mit Cyclohexandionen der Formel II

H.1.1.1. 2-(3-Chlor-5-trifluormethyl-pyridin-2-yl-carbonyl)-cyclohex-1-en-1-ol-3-on

Zu einer Lösung von 2,2 g (20 mMol) 1,3-Cyclohexandion und 7 ml (50 mMol) Triethylamin in 25 ml Dichlormethan werden 4,9 g (20 mMol) 3-Chlor-5-trifluormethylpyridin-2-carbonsäurechlorid getropft, wobei die Temperatur auf 35°C ansteigt. Anschliessend wird 15 Stunden bei Raumtemperatur ausgerührt. Die schwarze Suspension wird mit 250 ml Dichlormethan verdünnt, bei 0-5° mit 1N HCl auf pH 1 gestellt und 2x mit $H_2O$ gewaschen. Das Produkt wird danach mit $NaHCO_3$-Lösung 5 % extrahiert, kalt mit 37 %-iger HCl ausgefällt, genutscht und getrocknet. Man isoliert 4,0 g (63 %) der Titelverbindung der Formel

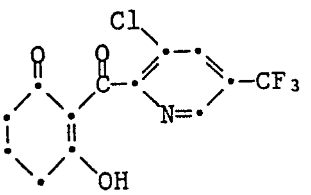

als Kristalle vom Smp. 102-105°C (Verb. Nr. 1.005).

H.1.1.2. 2-(5-Trifluormethylpyrid-2-yl-carbonyl)-cyclohex-1-en-1-ol-3-on

Zu einer Lösung von 9,5 g (85 mMol) 1,3-Cyclohexandion und 24 ml (170 mMol) Triethylamin in 85 ml Dichlormethan werden bei 20-25°C 20,4 g (85 mMol) 5-Trifluormethylpyridin-2-carbonsäurechlorid zugetropft. Nach 4 Stunden Rühren bei Raumtemperatur werden 0,8 ml Acetoncyanhydrin zugegeben und weitere 15 Stunden gerührt. Die Reaktionslösung wird mit 200 ml Dichlormethan verdünnt, bei 0-5°C mit HCl 1N auf pH 1 gestellt, 2x mit Wasser gewaschen und mit $NaHCO_3$-Lösung 5 % extrahiert. Diese wird mit Dichlormethan gewaschen, mit HCl 37 % auf pH 1 gestellt, das ausgefallene Produkt genutscht und getrocknet.

Man isoliert 17,2 g (71 %) der Titelverbindung der Formel

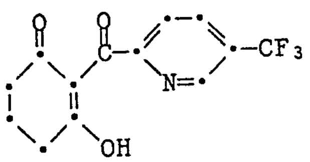

als Kristalle von Smp. 95-97°C (Verb. Nr. 1.010).

Analog zu dem vorstehenden Herstellungsverfahren können die Verbindungen der Tabelle 1 synthetisiert werden:

H.1.1.3. 2-(3-Chlor-5-methylthio-pyridin-2-yl-carbonyl)-cyclohex-1-en-1-ol-3-on

4,4 g (0,048 Mol) 1,3-Cyclohexandion und 10,6 g (0,048 Mol) 3-Chlor-5-methylthiopyridin-2-carbonsäurechlorid werden analog H.1.1.1. umgesetzt und gereinigt.

Man isoliert 7,2 g (50,4 %) der Titelverbindung der Formel

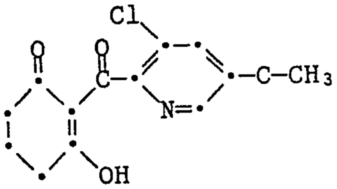

als Kristalle vom Smp. 113°C (Zers.) (Verb. Nr. 1.018).

H.1.1.4. (3-Chlor-5-methylsulfonyl-pyridin-2-yl-carbonyl)-cyclohex-1-en-1-ol-3-on

Zu einer Suspension von 10,2 g (40 mMol) 3-Chlor-5-methylsulfonyl-pyridin-2-carbonsäurechlorid in 80 ml Dichlormethan wird bei 0-5°C eine Lösung von 5,6 g (50 mMol) 1,3-Cyclohexandion und 14 ml (100 mMol)

Triethylamin in 50 ml Dichlormethan getropft. Nach 3 Stunden Rühren bei Raumtemperatur werden 0,5 ml Acetoncyanhydrin zur so erhaltenen Suspension gegeben. Anschliessend wird 3 Stunden bei Raumtemperatur ausgerührt. Die dunkelbraune Suspension wird mit 200 ml Dichlormethan verdünnt, bei 0-5°C mit 1N HCl auf pH 1 gestellt und 2x mit $H_2O$ gewaschen. Das Produkt wird danach mit $NaHCO_3$-Lösung 5 % extrahiert, kalt mit HCl 37 % ausgefällt, genutscht und getrocknet.

Man isoliert 10,4 g (78.9 %) der Titelverbindung der Formel

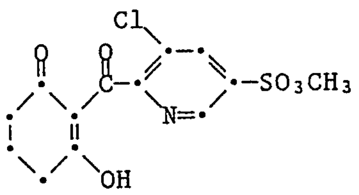

als weisse Kristalle vom Smp. >200°C (Zers.) (Verb. Nr. 1.020).

H.1.2.1. Herstellung von 2-(3-Chlor-5-methylsulfonyl-pyridin-2-yl-carbonyl)-cyclohex-1-en-1-ol-3-on

Zu einer Lösung von 3 g (0,01 Mol) 2-(3-Chlor-5-methylthiopyridin-2-yl-carbonyl)-cyclohex-1-en-1-ol-3-on in 25 ml Dichlormethan wird unter Kühlung bei 20-30°C eine Lösung von 4,1 g (0,02 Mol) 3-Chlorperbenzoesäure 85 % in 50 ml Dichlormethan getropft. Anschliessend wird 4 Stunden bei Raumtemperatur gerührt. Die gelbe Suspension wird von der Chlorbenzoesäure abfiltriert und am Rotavapor eingedampft. Die resultierende Masse wird mit 50 ml Ether verrieben, genutscht und getrocknet.

Man isoliert 1,8 g (51,6 %) der Titelverbindung der Formel

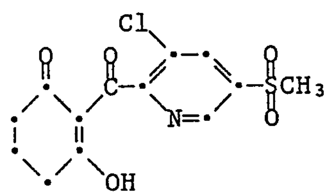

als Kristalle vom Smp. 150°C (Zers.) (Verb. Nr. 1.020).

Tabelle 1

Verbindungen der Formel

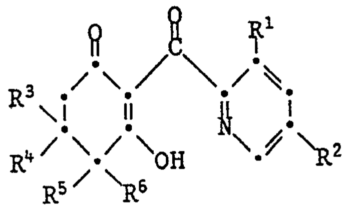

| Verb. Nr. | $R^1$ | $R^2$ | $R^3$ | $R^4$ | $R^5$ | $R^6$ | phys. Daten |
|---|---|---|---|---|---|---|---|
| 1.001 | Cl | Cl | H | H | H | H | Fp. 144-146°C |
| 1.002 | Cl | H | H | H | H | H | |
| 1.003 | H | Cl | H | H | H | H | Fp. 106-107°C |
| 1.004 | H | H | H | H | H | H | Harz |
| 1.005 | Cl | $CF_3$ | H | H | H | H | Fp. 102-105°C |
| 1.006 | $NO_2$ | H | H | H | H | H | |
| 1.007 | H | $NO_2$ | H | H | H | H | |
| 1.008 | $NO_2$ | Cl | H | H | H | H | |
| 1.009 | $CF_3$ | H | H | H | H | H | |
| 1.010 | H | $CF_3$ | H | H | H | H | Fp. 95-97°C |
| 1.011 | $OCH_3$ | H | H | H | H | H | |
| 1.012 | CN | H | H | H | H | H | |
| 1.013 | $OCH_3$ | Cl | H | H | H | H | |
| 1.014 | CN | Cl | H | H | H | H | |
| 1.015 | Br | Cl | H | H | H | H | |
| 1.016 | $SCH_3$ | Cl | H | H | H | H | |
| 1.017 | $SO_2CH_3$ | Cl | H | H | H | H | |
| 1.018 | Cl | $SCH_3$ | H | H | H | H | Fp. >113°C(Zers) |
| 1.019 | Cl | $SOCH_3$ | H | H | H | H | Fp. 134-136°C |
| 1.020 | Cl | $SO_2CH_3$ | H | H | H | H | Fp. >150°C(Zers) |
| 1.021 | $SOCH_3$ | Cl | H | H | H | H | |
| 1.022 | $SO_2CH_3$ | H | H | H | H | H | |
| 1.023 | H | $SO_2CH_3$ | H | H | H | H | |
| 1.024 | H | $CH_3$ | H | H | H | H | |
| 1.025 | Cl | F | H | H | H | H | Fp. 113-115°C |
| 1.026 | H | $CF_3$ | H | H | H | H | |
| 1.027 | F | F | H | H | H | H | |
| 1.028 | F | $CF_3$ | H | H | H | H | |
| 1.029 | $CF_3$ | F | H | H | H | H | |
| 1.030 | H | $CCl_3$ | H | H | H | H | |
| 1.031 | $CCl_3$ | H | H | H | H | H | |
| 1.032 | Cl | $CCl_3$ | H | H | H | H | |
| 1.033 | $CCl_3$ | Cl | H | H | H | H | |
| 1.034 | $CH_3$ | H | H | H | H | H | |
| 1.035 | Cl | Cl | $CH_3$ | H | H | H | Fp. 117-124°C |
| 1.036 | Cl | H | $CH_3$ | H | H | H | |
| 1.037 | H | Cl | $CH_3$ | H | H | H | |
| 1.038 | H | H | $CH_3$ | H | H | H | |
| 1.039 | Cl | $CF_3$ | $CH_3$ | H | H | H | Fp. 93-103°C |
| 1.040 | $NO_2$ | H | $CH_3$ | H | H | H | |
| 1.041 | H | $NO_2$ | $CH_3$ | H | H | H | |

| Verb. Nr. | $R^1$ | $R^2$ | $R^3$ | $R^4$ | $R^5$ | $R^6$ | phys. Daten |
|---|---|---|---|---|---|---|---|
| 1.042 | $NO_2$ | Cl | $CH_3$ | H | H | H | |
| 1.043 | $CF_3$ | H | $CH_3$ | H | H | H | |
| 1.044 | H | $CF_3$ | $CH_3$ | H | H | H | Fp. 99-101°C |
| 1.045 | $OCH_3$ | H | $CH_3$ | H | H | H | |
| 1.046 | CN | H | $CH_3$ | H | H | H | |
| 1.047 | $OCH_3$ | Cl | $CH_3$ | H | H | H | |
| 1.048 | CN | Cl | $CH_3$ | H | H | H | |
| 1.049 | Br | Cl | $CH_3$ | H | H | H | |
| 1.050 | $SCH_3$ | Cl | $CH_3$ | H | H | H | |
| 1.051 | $SO_2CH_3$ | Cl | $CH_3$ | H | H | H | |
| 1.052 | Cl | $SCH_3$ | $CH_3$ | H | H | H | |
| 1.053 | Cl | $SOCH_3$ | $CH_3$ | H | H | H | |
| 1.054 | Cl | $SO_2CH_3$ | $CH_3$ | H | H | H | |
| 1.055 | $SOCH_3$ | Cl | $CH_3$ | H | H | H | |
| 1.056 | $SO_2CH_3$ | H | $CH_3$ | H | H | H | |
| 1.057 | H | $SO_2CH_3$ | $CH_3$ | H | H | H | |
| 1.058 | H | $CH_3$ | $CH_3$ | H | H | H | |
| 1.059 | Cl | F | $CH_3$ | H | H | H | |
| 1.060 | H | $CF_3$ | $CH_3$ | H | H | H | |
| 1.061 | F | F | $CH_3$ | H | H | H | |
| 1.062 | F | $CF_3$ | $CH_3$ | H | H | H | |
| 1.063 | $CF_3$ | F | $CH_3$ | H | H | H | |
| 1.064 | H | $CCl_3$ | $CH_3$ | H | H | H | |
| 1.065 | $CCl_3$ | H | $CH_3$ | H | H | H | |
| 1.066 | Cl | $CCl_3$ | $CH_3$ | H | H | H | |
| 1.067 | $CCl_3$ | Cl | $CH_3$ | H | H | H | |
| 1.068 | $CH_3$ | H | $CH_3$ | H | H | H | |
| 1.069 | Cl | Cl | $C_2H_5$ | H | H | H | |
| 1.070 | Cl | H | $C_2H_5$ | H | H | H | |
| 1.071 | H | Cl | $C_2H_5$ | H | H | H | |
| 1.072 | H | H | $C_2H_5$ | H | H | H | |
| 1.073 | Cl | $CF_3$ | $C_2H_5$ | H | H | H | |
| 1.074 | $NO_2$ | H | $C_2H_5$ | H | H | H | |
| 1.075 | H | $NO_2$ | $C_2H_5$ | H | H | H | |
| 1.076 | $NO_2$ | Cl | $C_2H_5$ | H | H | H | |
| 1.077 | $CF_3$ | H | $C_2H_5$ | H | H | H | |
| 1.078 | H | $CF_3$ | $C_2H_5$ | H | H | H | |
| 1.079 | $OCH_3$ | H | $C_2H_5$ | H | H | H | |
| 1.080 | CN | H | $C_2H_5$ | H | H | H | |
| 1.081 | $OCH_3$ | Cl | $C_2H_5$ | H | H | H | |
| 1.082 | CN | Cl | $C_2H_5$ | H | H | H | |
| 1.083 | Br | Cl | $C_2H_5$ | H | H | H | |
| 1.084 | $SCH_3$ | Cl | $C_2H_5$ | H | H | H | |
| 1.085 | $SO_2CH_3$ | Cl | $C_2H_5$ | H | H | H | |
| 1.086 | Cl | $SCH_3$ | $C_2H_5$ | H | H | H | |
| 1.087 | Cl | $SOCH_3$ | $C_2H_5$ | H | H | H | |
| 1.088 | Cl | $SO_2CH_3$ | $C_2H_5$ | H | H | H | |
| 1.089 | $SOCH_3$ | Cl | $C_2H_5$ | H | H | H | |
| 1.090 | $SO_2CH_3$ | H | $C_2H_5$ | H | H | H | |
| 1.091 | H | $SO_2CH_3$ | $C_2H_5$ | H | H | H | |
| 1.092 | H | $CH_3$ | $C_2H_5$ | H | H | H | |
| 1.093 | Cl | F | $C_2H_5$ | H | H | H | |

| Verb. Nr. | $R^1$ | $R^2$ | $R^3$ | $R^4$ | $R^5$ | $R^6$ | phys. Daten |
|---|---|---|---|---|---|---|---|
| 1.094 | H | $CF_3$ | $C_2H_5$ | H | H | H | |
| 1.095 | F | F | $C_2H_5$ | H | H | H | |
| 1.096 | F | $CF_3$ | $C_2H_5$ | H | H | H | |
| 1.097 | $CF_3$ | F | $C_2H_5$ | H | H | H | |
| 1.098 | H | $CCl_3$ | $C_2H_5$ | H | H | H | |
| 1.099 | $CCl_3$ | H | $C_2H_5$ | H | H | H | |
| 1.100 | Cl | $CCl_3$ | $C_2H_5$ | H | H | H | |
| 1.101 | $CCl_3$ | Cl | $C_2H_5$ | H | H | H | |
| 1.102 | $CH_3$ | H | $C_2H_5$ | H | H | H | |
| 1.103 | Cl | Cl | $n-C_3H_7$ | H | H | H | |
| 1.104 | Cl | H | $n-C_3H_7$ | H | H | H | |
| 1.105 | H | Cl | $n-C_3H_7$ | H | H | H | |
| 1.106 | H | H | $n-C_3H_7$ | H | H | H | |
| 1.107 | Cl | $CF_3$ | $n-C_3H_7$ | H | H | H | |
| 1.108 | $NO_2$ | H | $n-C_3H_7$ | H | H | H | |
| 1.109 | H | $NO_2$ | $n-C_3H_7$ | H | H | H | |
| 1.110 | $NO_2$ | Cl | $n-C_3H_7$ | H | H | H | |
| 1.111 | $CF_3$ | H | $n-C_3H_7$ | H | H | H | |
| 1.112 | H | $CF_3$ | $n-C_3H_7$ | H | H | H | |
| 1.113 | $OCH_3$ | H | $n-C_3H_7$ | H | H | H | |
| 1.114 | CN | H | $n-C_3H_7$ | H | H | H | |
| 1.115 | $OCH_3$ | Cl | $n-C_3H_7$ | H | H | H | |
| 1.116 | CN | Cl | $n-C_3H_7$ | H | H | H | |
| 1.117 | Br | Cl | $n-C_3H_7$ | H | H | H | |
| 1.118 | $SCH_3$ | Cl | $n-C_3H_7$ | H | H | H | |
| 1.119 | $SO_2CH_3$ | Cl | $n-C_3H_7$ | H | H | H | |
| 1.120 | Cl | $SCH_3$ | $n-C_3H_7$ | H | H | H | |
| 1.121 | Cl | $SOCH_3$ | $n-C_3H_7$ | H | H | H | |
| 1.122 | Cl | $SO_2CH_3$ | $n-C_3H_7$ | H | H | H | |
| 1.123 | $SOCH_3$ | Cl | $n-C_3H_7$ | H | H | H | |
| 1.124 | $SO_2CH_3$ | H | $n-C_3H_7$ | H | H | H | |
| 1.125 | H | $SO_2CH_3$ | $n-C_3H_7$ | H | H | H | |
| 1.126 | H | $CH_3$ | $n-C_3H_7$ | H | H | H | |
| 1.127 | Cl | F | $n-C_3H_7$ | H | H | H | |
| 1.128 | H | $CF_3$ | $n-C_3H_7$ | H | H | H | |
| 1.129 | F | F | $n-C_3H_7$ | H | H | H | |
| 1.130 | F | $CF_3$ | $n-C_3H_7$ | H | H | H | |
| 1.131 | $CF_3$ | F | $n-C_3H_7$ | H | H | H | |
| 1.132 | H | $CCl_3$ | $n-C_3H_7$ | H | H | H | |
| 1.133 | $CCl_3$ | H | $n-C_3H_7$ | H | H | H | |
| 1.134 | Cl | $CCl_3$ | $n-C_3H_7$ | H | H | H | |
| 1.135 | $CCl_3$ | Cl | $n-C_3H_7$ | H | H | H | |
| 1.136 | $CH_3$ | H | $n-C_3H_7$ | H | H | H | |
| 1.137 | Cl | Cl | $i-C_3H_7$ | H | H | H | |
| 1.138 | Cl | H | $i-C_3H_7$ | H | H | H | |
| 1.139 | H | Cl | $i-C_3H_7$ | H | H | H | |
| 1.140 | H | H | $i-C_3H_7$ | H | H | H | |
| 1.141 | Cl | $CF_3$ | $i-C_3H_7$ | H | H | H | Fp. 72-75°C |
| 1.142 | $NO_2$ | H | $i-C_3H_7$ | H | H | H | |
| 1.143 | H | $NO_2$ | $i-C_3H_7$ | H | H | H | |
| 1.144 | $NO_2$ | Cl | $i-C_3H_7$ | H | H | H | |
| 1.145 | $CF_3$ | H | $i-C_3H_7$ | H | H | H | |

| Verb. Nr. | $R^1$ | $R^2$ | $R^3$ | $R^4$ | $R^5$ | $R^6$ | phys. Daten |
|---|---|---|---|---|---|---|---|
| 1.146 | H | $CF_3$ | i-$C_3H_7$ | H | H | H | Fp. 103-106°C |
| 1.147 | $OCH_3$ | H | i-$C_3H_7$ | H | H | H | |
| 1.148 | CN | H | i-$C_3H_7$ | H | H | H | |
| 1.149 | $OCH_3$ | Cl | i-$C_3H_7$ | H | H | H | |
| 1.150 | CN | Cl | i-$C_3H_7$ | H | H | H | |
| 1.151 | Br | Cl | i-$C_3H_7$ | H | H | H | |
| 1.152 | $SCH_3$ | Cl | i-$C_3H_7$ | H | H | H | |
| 1.153 | $SO_2CH_3$ | Cl | i-$C_3H_7$ | H | H | H | |
| 1.154 | Cl | $SCH_3$ | i-$C_3H_7$ | H | H | H | |
| 1.155 | Cl | $SOCH_3$ | i-$C_3H_7$ | H | H | H | |
| 1.156 | Cl | $SO_2CH_3$ | i-$C_3H_7$ | H | H | H | |
| 1.157 | $SOCH_3$ | Cl | i-$C_3H_7$ | H | H | H | |
| 1.158 | $SO_2CH_3$ | H | i-$C_3H_7$ | H | H | H | |
| 1.159 | H | $SO_2CH_3$ | i-$C_3H_7$ | H | H | H | |
| 1.160 | H | $CH_3$ | i-$C_3H_7$ | H | H | H | |
| 1.161 | Cl | F | i-$C_3H_7$ | H | H | H | |
| 1.162 | H | $CF_3$ | i-$C_3H_7$ | H | H | H | |
| 1.163 | F | F | i-$C_3H_7$ | H | H | H | |
| 1.164 | F | $CF_3$ | i-$C_3H_7$ | H | H | H | |
| 1.165 | $CF_3$ | F | i-$C_3H_7$ | H | H | H | |
| 1.166 | H | $CCl_3$ | i-$C_3H_7$ | H | H | H | |
| 1.167 | $CCl_3$ | H | i-$C_3H_7$ | H | H | H | |
| 1.168 | Cl | $CCl_3$ | i-$C_3H_7$ | H | H | H | |
| 1.169 | $CCl_3$ | Cl | i-$C_3H_7$ | H | H | H | |
| 1.170 | $CH_3$ | H | i-$C_3H_7$ | H | H | H | |
| 1.171 | Cl | Cl | n-$C_3H_7$ | H | H | $COOC_2H_5$ | |
| 1.172 | Cl | H | n-$C_3H_7$ | H | H | $COOC_2H_5$ | |
| 1.173 | H | Cl | n-$C_3H_7$ | H | H | $COOC_2H_5$ | |
| 1.174 | H | H | n-$C_3H_7$ | H | H | $COOC_2H_5$ | |
| 1.175 | Cl | $CF_3$ | n-$C_3H_7$ | H | H | $COOC_2H_5$ | |
| 1.176 | $NO_2$ | H | n-$C_3H_7$ | H | H | $COOC_2H_5$ | |
| 1.177 | H | $NO_2$ | n-$C_3H_7$ | H | H | $COOC_2H_5$ | |
| 1.178 | $NO_2$ | Cl | n-$C_3H_7$ | H | H | $COOC_2H_5$ | |
| 1.179 | $CF_3$ | H | n-$C_3H_7$ | H | H | $COOC_2H_5$ | |
| 1.180 | H | $CF_3$ | n-$C_3H_7$ | H | H | $COOC_2H_5$ | |
| 1.181 | $OCH_3$ | H | n-$C_3H_7$ | H | H | $COOC_2H_5$ | |
| 1.182 | CN | H | n-$C_3H_7$ | H | H | $COOC_2H_5$ | |
| 1.183 | $OCH_3$ | Cl | n-$C_3H_7$ | H | H | $COOC_2H_5$ | |
| 1.184 | CN | Cl | n-$C_3H_7$ | H | H | $COOC_2H_5$ | |
| 1.185 | Br | Cl | n-$C_3H_7$ | H | H | $COOC_2H_5$ | |
| 1.186 | $SCH_3$ | Cl | n-$C_3H_7$ | H | H | $COOC_2H_5$ | |
| 1.187 | $SO_2CH_3$ | Cl | n-$C_3H_7$ | H | H | $COOC_2H_5$ | |
| 1.188 | Cl | $SCH_3$ | n-$C_3H_7$ | H | H | $COOC_2H_5$ | |
| 1.189 | Cl | $SOCH_3$ | n-$C_3H_7$ | H | H | $COOC_2H_5$ | |
| 1.190 | Cl | $SO_2CH_3$ | n-$C_3H_7$ | H | H | $COOC_2H_5$ | |
| 1.191 | $SOCH_3$ | Cl | n-$C_3H_7$ | H | H | $COOC_2H_5$ | |
| 1.192 | $SO_2CH_3$ | H | n-$C_3H_7$ | H | H | $COOC_2H_5$ | |
| 1.193 | H | $SO_2CH_3$ | n-$C_3H_7$ | H | H | $COOC_2H_5$ | |
| 1.194 | H | $CH_3$ | n-$C_3H_7$ | H | H | $COOC_2H_5$ | |
| 1.195 | Cl | F | n-$C_3H_7$ | H | H | $COOC_2H_5$ | |
| 1.196 | H | $CF_3$ | n-$C_3H_7$ | H | H | $COOC_2H_5$ | |
| 1.197 | F | F | n-$C_3H_7$ | H | H | $COOC_2H_5$ | |

| Verb. Nr. | $R^1$ | $R^2$ | $R^3$ | $R^4$ | $R^5$ | $R^6$ | phys. Daten |
|---|---|---|---|---|---|---|---|
| 1.198 | F | $CF_3$ | n-$C_3H_7$ | H | H | $COOC_2H_5$ | |
| 1.199 | $CF_3$ | F | n-$C_3H_7$ | H | H | $COOC_2H_5$ | |
| 1.200 | H | $CCl_3$ | n-$C_3H_7$ | H | H | $COOC_2H_5$ | |
| 1.201 | $CCl_3$ | H | n-$C_3H_7$ | H | H | $COOC_2H_5$ | |
| 1.202 | Cl | $CCl_3$ | n-$C_3H_7$ | H | H | $COOC_2H_5$ | |
| 1.203 | $CCl_3$ | Cl | n-$C_3H_7$ | H | H | $COOC_2H_5$ | |
| 1.204 | $CH_3$ | H | n-$C_3H_7$ | H | H | $COOC_2H_5$ | |
| 1.205 | Cl | Cl | i-$C_3H_7$ | H | H | $COOCH_3$ | |
| 1.206 | Cl | H | i-$C_3H_7$ | H | H | $COOCH_3$ | |
| 1.207 | H | Cl | i-$C_3H_7$ | H | H | $COOCH_3$ | |
| 1.208 | H | H | i-$C_3H_7$ | H | H | $COOCH_3$ | |
| 1.209 | Cl | $CF_3$ | i-$C_3H_7$ | H | H | $COOCH_3$ | Fp. 91-92°C |
| 1.210 | $NO_2$ | H | i-$C_3H_7$ | H | H | $COOCH_3$ | |
| 1.211 | H | $NO_2$ | i-$C_3H_7$ | H | H | $COOCH_3$ | |
| 1.212 | $NO_2$ | Cl | i-$C_3H_7$ | H | H | $COOCH_3$ | |
| 1.213 | $CF_3$ | H | i-$C_3H_7$ | H | H | $COOCH_3$ | |
| 1.214 | H | $CF_3$ | i-$C_3H_7$ | H | H | $COOCH_3$ | |
| 1.215 | $OCH_3$ | H | i-$C_3H_7$ | H | H | $COOCH_3$ | |
| 1.216 | CN | H | i-$C_3H_7$ | H | H | $COOCH_3$ | |
| 1.217 | $OCH_3$ | Cl | i-$C_3H_7$ | H | H | $COOCH_3$ | |
| 1.218 | CN | Cl | i-$C_3H_7$ | H | H | $COOCH_3$ | |
| 1.219 | Br | Cl | i-$C_3H_7$ | H | H | $COOCH_3$ | |
| 1.220 | $SCH_3$ | Cl | i-$C_3H_7$ | H | H | $COOCH_3$ | |
| 1.221 | $SO_2CH_3$ | Cl | i-$C_3H_7$ | H | H | $COOCH_3$ | |
| 1.222 | Cl | $SCH_3$ | i-$C_3H_7$ | H | H | $COOCH_3$ | |
| 1.223 | Cl | $SOCH_3$ | i-$C_3H_7$ | H | H | $COOCH_3$ | |
| 1.224 | Cl | $SO_2CH_3$ | i-$C_3H_7$ | H | H | $COOCH_3$ | |
| 1.225 | $SOCH_3$ | Cl | i-$C_3H_7$ | H | H | $COOCH_3$ | |
| 1.226 | $SO_2CH_3$ | H | i-$C_3H_7$ | H | H | $COOCH_3$ | |
| 1.227 | H | $SO_2CH_3$ | i-$C_3H_7$ | H | H | $COOCH_3$ | |
| 1.228 | H | $CH_3$ | i-$C_3H_7$ | H | H | $COOCH_3$ | |
| 1.229 | Cl | F | i-$C_3H_7$ | H | H | $COOCH_3$ | |
| 1.230 | H | $CF_3$ | i-$C_3H_7$ | H | H | $COOCH_3$ | |
| 1.231 | F | F | i-$C_3H_7$ | H | H | $COOCH_3$ | |
| 1.232 | F | $CF_3$ | i-$C_3H_7$ | H | H | $COOCH_3$ | |
| 1.233 | $CF_3$ | F | i-$C_3H_7$ | H | H | $COOCH_3$ | |
| 1.234 | H | $CCl_3$ | i-$C_3H_7$ | H | H | $COOCH_3$ | |
| 1.235 | $CCl_3$ | H | i-$C_3H_7$ | H | H | $COOCH_3$ | |
| 1.236 | Cl | $CCl_3$ | i-$C_3H_7$ | H | H | $COOCH_3$ | |
| 1.237 | $CCl_3$ | Cl | i-$C_3H_7$ | H | H | $COOCH_3$ | |
| 1.238 | $CH_3$ | H | i-$C_3H_7$ | H | H | $COOCH_3$ | |
| 1.239 | Cl | Cl | $CH_3$ | H | H | $COOCH_3$ | |
| 1.240 | Cl | H | $CH_3$ | H | H | $COOCH_3$ | |
| 1.241 | H | Cl | $CH_3$ | H | H | $COOCH_3$ | |
| 1.242 | H | H | $CH_3$ | H | H | $COOCH_3$ | |
| 1.243 | Cl | $CF_3$ | $CH_3$ | H | H | $COOCH_3$ | |
| 1.244 | $NO_2$ | H | $CH_3$ | H | H | $COOCH_3$ | |
| 1.245 | H | $NO_2$ | $CH_3$ | H | H | $COOCH_3$ | |
| 1.246 | $NO_2$ | Cl | $CH_3$ | H | H | $COOCH_3$ | |
| 1.247 | $CF_3$ | H | $CH_3$ | H | H | $COOCH_3$ | |
| 1.248 | H | $CF_3$ | $CH_3$ | H | H | $COOCH_3$ | |
| 1.249 | $OCH_3$ | H | $CH_3$ | H | H | $COOCH_3$ | |

| Verb. Nr. | $R^1$ | $R^2$ | $R^3$ | $R^4$ | $R^5$ | $R^6$ | phys. Daten |
|---|---|---|---|---|---|---|---|
| 1.250 | CN | H | $CH_3$ | H | H | $COOCH_3$ | |
| 1.251 | $OCH_3$ | Cl | $CH_3$ | H | H | $COOCH_3$ | |
| 1.252 | CN | Cl | $CH_3$ | H | H | $COOCH_3$ | |
| 1.253 | Br | Cl | $CH_3$ | H | H | $COOCH_3$ | |
| 1.254 | $SCH_3$ | Cl | $CH_3$ | H | H | $COOCH_3$ | |
| 1.255 | $SO_2CH_3$ | Cl | $CH_3$ | H | H | $COOCH_3$ | |
| 1.256 | Cl | $SCH_3$ | $CH_3$ | H | H | $COOCH_3$ | |
| 1.257 | Cl | $SOCH_3$ | $CH_3$ | H | H | $COOCH_3$ | |
| 1.258 | Cl | $SO_2CH_3$ | $CH_3$ | H | H | $COOCH_3$ | |
| 1.259 | $SOCH_3$ | Cl | $CH_3$ | H | H | $COOCH_3$ | |
| 1.260 | $SO_2CH_3$ | H | $CH_3$ | H | H | $COOCH_3$ | |
| 1.261 | H | $SO_2CH_3$ | $CH_3$ | H | H | $COOCH_3$ | |
| 1.262 | H | $CH_3$ | $CH_3$ | H | H | $COOCH_3$ | |
| 1.263 | Cl | F | $CH_3$ | H | H | $COOCH_3$ | |
| 1.264 | H | $CF_3$ | $CH_3$ | H | H | $COOCH_3$ | |
| 1.265 | F | F | $CH_3$ | H | H | $COOCH_3$ | |
| 1.266 | F | $CF_3$ | $CH_3$ | H | H | $COOCH_3$ | |
| 1.267 | $CF_3$ | F | $CH_3$ | H | H | $COOCH_3$ | |
| 1.268 | H | $CCl_3$ | $CH_3$ | H | H | $COOCH_3$ | |
| 1.269 | $CCl_3$ | H | $CH_3$ | H | H | $COOCH_3$ | |
| 1.270 | Cl | $CCl_3$ | $CH_3$ | H | H | $COOCH_3$ | |
| 1.271 | $CCl_3$ | Cl | $CH_3$ | H | H | $COOCH_3$ | |
| 1.272 | $CH_3$ | H | $CH_3$ | H | H | $COOCH_3$ | |
| 1.273 | Cl | Cl | $CH_3$ | $CH_3$ | H | H | Fp. 131-133°C |
| 1.274 | Cl | H | $CH_3$ | $CH_3$ | H | H | |
| 1.275 | H | Cl | $CH_3$ | $CH_3$ | H | H | |
| 1.276 | H | H | $CH_3$ | $CH_3$ | H | H | |
| 1.277 | Cl | $CF_3$ | $CH_3$ | $CH_3$ | H | H | Fp. 95-98°C |
| 1.278 | $NO_2$ | H | $CH_3$ | $CH_3$ | H | H | |
| 1.279 | H | $NO_2$ | $CH_3$ | $CH_3$ | H | H | |
| 1.280 | $NO_2$ | Cl | $CH_3$ | $CH_3$ | H | H | |
| 1.281 | $CF_3$ | H | $CH_3$ | $CH_3$ | H | H | |
| 1.282 | H | $CF_3$ | $CH_3$ | $CH_3$ | H | H | |
| 1.283 | $OCH_3$ | H | $CH_3$ | $CH_3$ | H | H | |
| 1.284 | CN | H | $CH_3$ | $CH_3$ | H | H | |
| 1.285 | $OCH_3$ | Cl | $CH_3$ | $CH_3$ | H | H | |
| 1.286 | CN | Cl | $CH_3$ | $CH_3$ | H | H | |
| 1.287 | Br | Cl | $CH_3$ | $CH_3$ | H | H | |
| 1.288 | $SCH_3$ | Cl | $CH_3$ | $CH_3$ | H | H | |
| 1.289 | $SO_2CH_3$ | Cl | $CH_3$ | $CH_3$ | H | H | |
| 1.290 | Cl | $SCH_3$ | $CH_3$ | $CH_3$ | H | H | |
| 1.291 | Cl | $SOCH_3$ | $CH_3$ | $CH_3$ | H | H | |
| 1.292 | Cl | $SO_2CH_3$ | $CH_3$ | $CH_3$ | H | H | |
| 1.293 | $SOCH_3$ | Cl | $CH_3$ | $CH_3$ | H | H | |
| 1.294 | $SO_2CH_3$ | H | $CH_3$ | $CH_3$ | H | H | |
| 1.295 | H | $SO_2CH_3$ | $CH_3$ | $CH_3$ | H | H | |
| 1.296 | H | $CH_3$ | $CH_3$ | $CH_3$ | H | H | |
| 1.297 | Cl | F | $CH_3$ | $CH_3$ | H | H | |
| 1.298 | H | $CF_3$ | $CH_3$ | $CH_3$ | H | H | |
| 1.299 | F | F | $CH_3$ | $CH_3$ | H | H | |
| 1.300 | F | $CF_3$ | $CH_3$ | $CH_3$ | H | H | |
| 1.301 | $CF_3$ | F | $CH_3$ | $CH_3$ | H | H | |

| Verb. Nr. | $R^1$ | $R^2$ | $R^3$ | $R^4$ | $R^5$ | $R^6$ | phys. Daten |
|---|---|---|---|---|---|---|---|
| 1.302 | H | $CCl_3$ | $CH_3$ | $CH_3$ | H | H | |
| 1.303 | $CCl_3$ | H | $CH_3$ | $CH_3$ | H | H | |
| 1.304 | Cl | $CCl_3$ | $CH_3$ | $CH_3$ | H | H | |
| 1.305 | $CCl_3$ | Cl | $CH_3$ | $CH_3$ | H | H | |
| 1.306 | $CH_3$ | H | $CH_3$ | $CH_3$ | H | H | |
| 1.307 | Cl | Cl | $CH_3$ | $CH_3$ | H | $COOC_2H_5$ | |
| 1.308 | Cl | H | $CH_3$ | $CH_3$ | H | $COOC_2H_5$ | |
| 1.309 | H | Cl | $CH_3$ | $CH_3$ | H | $COOC_2H_5$ | |
| 1.310 | H | H | $CH_3$ | $CH_3$ | H | $COOC_2H_5$ | |
| 1.311 | Cl | $CF_3$ | $CH_3$ | $CH_3$ | H | $COOC_2H_5$ | |
| 1.312 | $NO_2$ | H | $CH_3$ | $CH_3$ | H | $COOC_2H_5$ | |
| 1.313 | H | $NO_2$ | $CH_3$ | $CH_3$ | H | $COOC_2H_5$ | |
| 1.314 | $NO_2$ | Cl | $CH_3$ | $CH_3$ | H | $COOC_2H_5$ | |
| 1.315 | $CF_3$ | H | $CH_3$ | $CH_3$ | H | $COOC_2H_5$ | |
| 1.316 | H | $CF_3$ | $CH_3$ | $CH_3$ | H | $COOC_2H_5$ | |
| 1.317 | $OCH_3$ | H | $CH_3$ | $CH_3$ | H | $COOC_2H_5$ | |
| 1.318 | CN | H | $CH_3$ | $CH_3$ | H | $COOC_2H_5$ | |
| 1.319 | $OCH_3$ | Cl | $CH_3$ | $CH_3$ | H | $COOC_2H_5$ | |
| 1.320 | CN | Cl | $CH_3$ | $CH_3$ | H | $COOC_2H_5$ | |
| 1.321 | Br | Cl | $CH_3$ | $CH_3$ | H | $COOC_2H_5$ | |
| 1.322 | $SCH_3$ | Cl | $CH_3$ | $CH_3$ | H | $COOC_2H_5$ | |
| 1.323 | $SO_2CH_3$ | Cl | $CH_3$ | $CH_3$ | H | $COOC_2H_5$ | |
| 1.324 | Cl | $SCH_3$ | $CH_3$ | $CH_3$ | H | $COOC_2H_5$ | |
| 1.325 | Cl | $SOCH_3$ | $CH_3$ | $CH_3$ | H | $COOC_2H_5$ | |
| 1.326 | Cl | $SO_2CH_3$ | $CH_3$ | $CH_3$ | H | $COOC_2H_5$ | |
| 1.327 | $SOCH_3$ | Cl | $CH_3$ | $CH_3$ | H | $COOC_2H_5$ | |
| 1.328 | $SO_2CH_3$ | H | $CH_3$ | $CH_3$ | H | $COOC_2H_5$ | |
| 1.329 | H | $SO_2CH_3$ | $CH_3$ | $CH_3$ | H | $COOC_2H_5$ | |
| 1.330 | H | $CH_3$ | $CH_3$ | $CH_3$ | H | $COOC_2H_5$ | |
| 1.331 | Cl | F | $CH_3$ | $CH_3$ | H | $COOC_2H_5$ | |
| 1.332 | H | $CF_3$ | $CH_3$ | $CH_3$ | H | $COOC_2H_5$ | |
| 1.333 | F | F | $CH_3$ | $CH_3$ | H | $COOC_2H_5$ | |
| 1.334 | F | $CF_3$ | $CH_3$ | $CH_3$ | H | $COOC_2H_5$ | |
| 1.335 | $CF_3$ | F | $CH_3$ | $CH_3$ | H | $COOC_2H_5$ | |
| 1.336 | H | $CCl_3$ | $CH_3$ | $CH_3$ | H | $COOC_2H_5$ | |
| 1.337 | $CCl_3$ | H | $CH_3$ | $CH_3$ | H | $COOC_2H_5$ | |
| 1.338 | Cl | $CCl_3$ | $CH_3$ | $CH_3$ | H | $COOC_2H_5$ | |
| 1.339 | $CCl_3$ | Cl | $CH_3$ | $CH_3$ | H | $COOC_2H_5$ | |
| 1.340 | $CH_3$ | H | $CH_3$ | $CH_3$ | H | $COOC_2H_5$ | |
| 1.341 | Cl | Cl | $C_6H_5$ | H | H | H | |
| 1.342 | Cl | H | $C_6H_5$ | H | H | H | |
| 1.343 | H | Cl | $C_6H_5$ | H | H | H | |
| 1.344 | H | H | $C_6H_5$ | H | H | H | |
| 1.345 | Cl | $CF_3$ | $C_6H_5$ | H | H | H | Fp.>150°C(Zers) |
| 1.346 | $NO_2$ | H | $C_6H_5$ | H | H | H | |
| 1.347 | H | $NO_2$ | $C_6H_5$ | H | H | H | |
| 1.348 | $NO_2$ | Cl | $C_6H_5$ | H | H | H | |
| 1.349 | $CF_3$ | H | $C_6H_5$ | H | H | H | |
| 1.350 | H | $CF_3$ | $C_6H_5$ | H | H | H | |
| 1.351 | $OCH_3$ | H | $C_6H_5$ | H | H | H | |
| 1.352 | CN | H | $C_6H_5$ | H | H | H | |
| 1.353 | $OCH_3$ | Cl | $C_6H_5$ | H | H | H | |

| Verb. Nr. | $R^1$ | $R^2$ | $R^3$ | $R^4$ | $R^5$ | $R^6$ | phys. Daten |
|---|---|---|---|---|---|---|---|
| 1.354 | CN | Cl | $C_6H_5$ | H | H | H | |
| 1.355 | Br | Cl | $C_6H_5$ | H | H | H | |
| 1.356 | $SCH_3$ | Cl | $C_6H_5$ | H | H | H | |
| 1.357 | $SO_2CH_3$ | Cl | $C_6H_5$ | H | H | H | |
| 1.358 | Cl | $SCH_3$ | $C_6H_5$ | H | H | H | |
| 1.359 | Cl | $SOCH_3$ | $C_6H_5$ | H | H | H | |
| 1.360 | Cl | $SO_2CH_3$ | $C_6H_5$ | H | H | H | |
| 1.361 | $SOCH_3$ | Cl | $C_6H_5$ | H | H | H | |
| 1.362 | $SO_2CH_3$ | H | $C_6H_5$ | H | H | H | |
| 1.363 | H | $SO_2CH_3$ | $C_6H_5$ | H | H | H | |
| 1.364 | H | $CH_3$ | $C_6H_5$ | H | H | H | |
| 1.365 | Cl | F | $C_6H_5$ | H | H | H | |
| 1.366 | H | $CF_3$ | $C_6H_5$ | H | H | H | |
| 1.367 | F | F | $C_6H_5$ | H | H | H | |
| 1.368 | F | $CF_3$ | $C_6H_5$ | H | H | H | |
| 1.369 | $CF_3$ | F | $C_6H_5$ | H | H | H | |
| 1.370 | H | $CCl_3$ | $C_6H_5$ | H | H | H | |
| 1.371 | $CCl_3$ | H | $C_6H_5$ | H | H | H | |
| 1.372 | Cl | $CCl_3$ | $C_6H_5$ | H | H | H | |
| 1.373 | $CCl_3$ | Cl | $C_6H_5$ | H | H | H | |
| 1.374 | $CH_3$ | H | $C_6H_5$ | H | H | H | |
| 1.375 | Cl | Cl | H | H | $CH_3$ | $CH_3$ | |
| 1.376 | Cl | H | H | H | $CH_3$ | $CH_3$ | |
| 1.377 | H | Cl | H | H | $CH_3$ | $CH_3$ | |
| 1.378 | H | H | H | H | $CH_3$ | $CH_3$ | |
| 1.379 | Cl | $CF_3$ | H | H | $CH_3$ | $CH_3$ | |
| 1.380 | $NO_2$ | H | H | H | $CH_3$ | $CH_3$ | |
| 1.381 | H | $NO_2$ | H | H | $CH_3$ | $CH_3$ | |
| 1.382 | $NO_2$ | Cl | H | H | $CH_3$ | $CH_3$ | |
| 1.383 | $CF_3$ | H | H | H | $CH_3$ | $CH_3$ | |
| 1.384 | H | $CF_3$ | H | H | $CH_3$ | $CH_3$ | |
| 1.385 | $OCH_3$ | H | H | H | $CH_3$ | $CH_3$ | |
| 1.386 | CN | H | H | H | $CH_3$ | $CH_3$ | |
| 1.387 | $OCH_3$ | Cl | H | H | $CH_3$ | $CH_3$ | |
| 1.388 | CN | Cl | H | H | $CH_3$ | $CH_3$ | |
| 1.389 | Br | Cl | H | H | $CH_3$ | $CH_3$ | |
| 1.390 | $SCH_3$ | Cl | H | H | $CH_3$ | $CH_3$ | |
| 1.391 | $SO_2CH_3$ | Cl | H | H | $CH_3$ | $CH_3$ | |
| 1.392 | Cl | $SCH_3$ | H | H | $CH_3$ | $CH_3$ | |
| 1.393 | Cl | $SOCH_3$ | H | H | $CH_3$ | $CH_3$ | |
| 1.394 | Cl | $SO_2CH_3$ | H | H | $CH_3$ | $CH_3$ | |
| 1.395 | $SOCH_3$ | Cl | H | H | $CH_3$ | $CH_3$ | |
| 1.396 | $SO_2CH_3$ | H | H | H | $CH_3$ | $CH_3$ | |
| 1.397 | H | $SO_2CH_3$ | H | H | $CH_3$ | $CH_3$ | |
| 1.398 | H | $CH_3$ | H | H | $CH_3$ | $CH_3$ | |
| 1.399 | Cl | F | H | H | $CH_3$ | $CH_3$ | |
| 1.400 | H | $CF_3$ | H | H | $CH_3$ | $CH_3$ | |
| 1.401 | F | F | H | H | $CH_3$ | $CH_3$ | |
| 1.402 | F | $CF_3$ | H | H | $CH_3$ | $CH_3$ | |
| 1.403 | $CF_3$ | F | H | H | $CH_3$ | $CH_3$ | |
| 1.404 | H | $CCl_3$ | H | H | $CH_3$ | $CH_3$ | |
| 1.405 | $CCl_3$ | H | H | H | $CH_3$ | $CH_3$ | |

| Verb. Nr. | $R^1$ | $R^2$ | $R^3$ | $R^4$ | $R^5$ | $R^6$ | phys. Daten |
|---|---|---|---|---|---|---|---|
| 1.406 | Cl | $CCl_3$ | H | H | $CH_3$ | $CH_3$ | |
| 1.407 | $CCl_3$ | Cl | H | H | $CH_3$ | $CH_3$ | |
| 1.408 | $CH_3$ | H | H | H | $CH_3$ | $CH_3$ | |
| 1.409 | Cl | Cl | $C_6H_5-CH_2-$ | H | H | H | |
| 1.410 | Cl | H | $C_6H_5-CH_2-$ | H | H | H | |
| 1.411 | H | Cl | $C_6H_5-CH_2-$ | H | H | H | |
| 1.412 | H | H | $C_6H_5-CH_2-$ | H | H | H | |
| 1.413 | Cl | $CF_3$ | $C_6H_5-CH_2-$ | H | H | H | |
| 1.414 | $NO_2$ | H | $C_6H_5-CH_2-$ | H | H | H | |
| 1.415 | H | $NO_2$ | $C_6H_5-CH_2-$ | H | H | H | |
| 1.416 | $NO_2$ | Cl | $C_6H_5-CH_2-$ | H | H | H | |
| 1.417 | $CF_3$ | H | $C_6H_5-CH_2-$ | H | H | H | |
| 1.418 | H | $CF_3$ | $C_6H_5-CH_2-$ | H | H | H | |
| 1.419 | $OCH_3$ | H | $C_6H_5-CH_2-$ | H | H | H | |
| 1.420 | CN | H | $C_6H_5-CH_2-$ | H | H | H | |
| 1.421 | $OCH_3$ | Cl | $C_6H_5-CH_2-$ | H | H | H | |
| 1.422 | CN | Cl | $C_6H_5-CH_2-$ | H | H | H | |
| 1.423 | Br | Cl | $C_6H_5-CH_2-$ | H | H | H | |
| 1.424 | $SCH_3$ | Cl | $C_6H_5-CH_2-$ | H | H | H | |
| 1.425 | $SO_2CH_3$ | Cl | $C_6H_5-CH_2-$ | H | H | H | |
| 1.426 | Cl | $SCH_3$ | $C_6H_5-CH_2-$ | H | H | H | |
| 1.427 | Cl | $SOCH_3$ | $C_6H_5-CH_2-$ | H | H | H | |
| 1.428 | Cl | $SO_2CH_3$ | $C_6H_5-CH_2-$ | H | H | H | |
| 1.429 | $SOCH_3$ | Cl | $C_6H_5-CH_2-$ | H | H | H | |
| 1.430 | $SO_2CH_3$ | H | $C_6H_5-CH_2-$ | H | H | H | |
| 1.431 | H | $SO_2CH_3$ | $C_6H_5-CH_2-$ | H | H | H | |
| 1.432 | H | $CH_3$ | $C_6H_5-CH_2-$ | H | H | H | |
| 1.433 | Cl | F | $C_6H_5-CH_2-$ | H | H | H | |
| 1.434 | H | $CF_3$ | $C_6H_5-CH_2-$ | H | H | H | |
| 1.435 | F | F | $C_6H_5-CH_2-$ | H | H | H | |
| 1.436 | F | $CF_3$ | $C_6H_5-CH_2-$ | H | H | H | |
| 1.437 | $CF_3$ | F | $C_6H_5-CH_2-$ | H | H | H | |
| 1.438 | H | $CCl_3$ | $C_6H_5-CH_2-$ | H | H | H | |
| 1.439 | $CCl_3$ | H | $C_6H_5-CH_2-$ | H | H | H | |
| 1.440 | Cl | $CCl_3$ | $C_6H_5-CH_2-$ | H | H | H | |
| 1.441 | $CCl_3$ | Cl | $C_6H_5-CH_2-$ | H | H | H | |
| 1.442 | $CH_3$ | H | $C_6H_5-CH_2-$ | H | H | H | |
| 1.443 | Cl | Cl | $4-Cl-C_6H_4$ | H | H | H | |
| 1.444 | Cl | H | $4-Cl-C_6H_4$ | H | H | H | |
| 1.445 | H | Cl | $4-Cl-C_6H_4$ | H | H | H | |
| 1.446 | H | H | $4-Cl-C_6H_4$ | H | H | H | |
| 1.447 | Cl | $CF_3$ | $4-Cl-C_6H_4$ | H | H | H | |
| 1.448 | $NO_2$ | H | $4-Cl-C_6H_4$ | H | H | H | |
| 1.449 | H | $NO_2$ | $4-Cl-C_6H_4$ | H | H | H | |
| 1.450 | $NO_2$ | Cl | $4-Cl-C_6H_4$ | H | H | H | |
| 1.451 | $CF_3$ | H | $4-Cl-C_6H_4$ | H | H | H | |
| 1.452 | H | $CF_3$ | $4-Cl-C_6H_4$ | H | H | H | |
| 1.453 | $OCH_3$ | H | $4-Cl-C_6H_4$ | H | H | H | |
| 1.454 | CN | H | $4-Cl-C_6H_4$ | H | H | H | |
| 1.455 | $OCH_3$ | Cl | $4-Cl-C_6H_4$ | H | H | H | |
| 1.456 | CN | Cl | $4-Cl-C_6H_4$ | H | H | H | |
| 1.457 | Br | Cl | $4-Cl-C_6H_4$ | H | H | H | |

| Verb. Nr. | $R^1$ | $R^2$ | $R^3$ | $R^4$ | $R^5$ | $R^6$ | phys. Daten |
|---|---|---|---|---|---|---|---|
| 1.458 | $SCH_3$ | Cl | 4-Cl-$C_6H_4$ | H | H | H | |
| 1.459 | $SO_2CH_3$ | Cl | 4-Cl-$C_6H_4$ | H | H | H | |
| 1.460 | Cl | $SCH_3$ | 4-Cl-$C_6H_4$ | H | H | H | |
| 1.461 | Cl | $SOCH_3$ | 4-Cl-$C_6H_4$ | H | H | H | |
| 1.462 | Cl | $SO_2CH_3$ | 4-Cl-$C_6H_4$ | H | H | H | |
| 1.463 | $SOCH_3$ | Cl | 4-Cl-$C_6H_4$ | H | H | H | |
| 1.464 | $SO_2CH_3$ | H | 4-Cl-$C_6H_4$ | H | H | H | |
| 1.465 | H | $SO_2CH_3$ | 4-Cl-$C_6H_4$ | H | H | H | |
| 1.466 | H | $CH_3$ | 4-Cl-$C_6H_4$ | H | H | H | |
| 1.467 | Cl | F | 4-Cl-$C_6H_4$ | H | H | H | |
| 1.468 | H | $CF_3$ | 4-Cl-$C_6H_4$ | H | H | H | |
| 1.469 | F | F | 4-Cl-$C_6H_4$ | H | H | H | |
| 1.470 | F | $CF_3$ | 4-Cl-$C_6H_4$ | H | H | H | |
| 1.471 | $CF_3$ | F | 4-Cl-$C_6H_4$ | H | H | H | |
| 1.472 | H | $CCl_3$ | 4-Cl-$C_6H_4$ | H | H | H | |
| 1.473 | $CCl_3$ | H | 4-Cl-$C_6H_4$ | H | H | H | |
| 1.474 | Cl | $CCl_3$ | 4-Cl-$C_6H_4$ | H | H | H | |
| 1.475 | $CCl_3$ | Cl | 4-Cl-$C_6H_4$ | H | H | H | |
| 1.476 | $CH_3$ | H | 4-Cl-$C_6H_4$ | H | H | H | |
| 1.477 | Cl | Cl | $CH_3$ | H | $CH_3$ | H | |
| 1.478 | Cl | H | $CH_3$ | H | $CH_3$ | H | |
| 1.479 | H | Cl | $CH_3$ | H | $CH_3$ | H | |
| 1.480 | H | H | $CH_3$ | H | $CH_3$ | H | |
| 1.481 | Cl | $CF_3$ | $CH_3$ | H | $CH_3$ | H | |
| 1.482 | $NO_2$ | H | $CH_3$ | H | $CH_3$ | H | |
| 1.483 | H | $NO_2$ | $CH_3$ | H | $CH_3$ | H | |
| 1.484 | $NO_2$ | Cl | $CH_3$ | H | $CH_3$ | H | |
| 1.485 | $CF_3$ | H | $CH_3$ | H | $CH_3$ | H | |
| 1.486 | H | $CF_3$ | $CH_3$ | H | $CH_3$ | H | |
| 1.487 | $OCH_3$ | H | $CH_3$ | H | $CH_3$ | H | |
| 1.488 | CN | H | $CH_3$ | H | $CH_3$ | H | |
| 1.489 | $OCH_3$ | Cl | $CH_3$ | H | $CH_3$ | H | |
| 1.490 | CN | Cl | $CH_3$ | H | $CH_3$ | H | |
| 1.491 | Br | Cl | $CH_3$ | H | $CH_3$ | H | |
| 1.492 | $SCH_3$ | Cl | $CH_3$ | H | $CH_3$ | H | |
| 1.493 | $SO_2CH_3$ | Cl | $CH_3$ | H | $CH_3$ | H | |
| 1.494 | Cl | $SCH_3$ | $CH_3$ | H | $CH_3$ | H | |
| 1.495 | Cl | $SOCH_3$ | $CH_3$ | H | $CH_3$ | H | |
| 1.496 | Cl | $SO_2CH_3$ | $CH_3$ | H | $CH_3$ | H | |
| 1.497 | $SOCH_3$ | Cl | $CH_3$ | H | $CH_3$ | H | |
| 1.498 | $SO_2CH_3$ | H | $CH_3$ | H | $CH_3$ | H | |
| 1.499 | H | $SO_2CH_3$ | $CH_3$ | H | $CH_3$ | H | |
| 1.500 | H | $CH_3$ | $CH_3$ | H | $CH_3$ | H | |
| 1.501 | Cl | F | $CH_3$ | H | $CH_3$ | H | |
| 1.502 | H | $CF_3$ | $CH_3$ | H | $CH_3$ | H | |
| 1.503 | F | F | $CH_3$ | H | $CH_3$ | H | |
| 1.504 | F | $CF_3$ | $CH_3$ | H | $CH_3$ | H | |
| 1.505 | $CF_3$ | F | $CH_3$ | H | $CH_3$ | H | |
| 1.506 | H | $CCl_3$ | $CH_3$ | H | $CH_3$ | H | |
| 1.507 | $CCl_3$ | H | $CH_3$ | H | $CH_3$ | H | |
| 1.508 | Cl | $CCl_3$ | $CH_3$ | H | $CH_3$ | H | |
| 1.509 | $CCl_3$ | Cl | $CH_3$ | H | $CH_3$ | H | |

| Verb. Nr. | $R^1$ | $R^2$ | $R^3$ | $R^4$ | $R^5$ | $R^6$ | phys. Daten |
|---|---|---|---|---|---|---|---|
| 1.510 | $CH_3$ | H | $CH_3$ | H | $CH_3$ | H | |
| 1.511 | Cl | Cl | H | H | H | CN | |
| 1.512 | Cl | H | H | H | H | CN | |
| 1.513 | H | Cl | H | H | H | CN | |
| 1.514 | H | H | H | H | H | CN | |
| 1.515 | Cl | $CF_3$ | H | H | H | CN | |
| 1.516 | $NO_2$ | H | H | H | H | CN | |
| 1.517 | H | $NO_2$ | H | H | H | CN | |
| 1.518 | $NO_2$ | Cl | H | H | H | CN | |
| 1.519 | $CF_3$ | H | H | H | H | CN | |
| 1.520 | H | $CF_3$ | H | H | H | CN | |
| 1.521 | $OCH_3$ | H | H | H | H | CN | |
| 1.522 | CN | H | H | H | H | CN | |
| 1.523 | $OCH_3$ | Cl | H | H | H | CN | |
| 1.524 | CN | Cl | H | H | H | CN | |
| 1.525 | Br | Cl | H | H | H | CN | |
| 1.526 | $SCH_3$ | Cl | H | H | H | CN | |
| 1.527 | $SO_2CH_3$ | Cl | H | H | H | CN | |
| 1.528 | Cl | $SCH_3$ | H | H | H | CN | |
| 1.529 | Cl | $SOCH_3$ | H | H | H | CN | |
| 1.530 | Cl | $SO_2CH_3$ | H | H | H | CN | |
| 1.531 | $SOCH_3$ | Cl | H | H | H | CN | |
| 1.532 | $SO_2CH_3$ | H | H | H | H | CN | |
| 1.533 | H | $SO_2CH_3$ | H | H | H | CN | |
| 1.534 | H | $CH_3$ | H | H | H | CN | |
| 1.535 | Cl | F | H | H | H | CN | |
| 1.536 | H | $CF_3$ | H | H | H | CN | |
| 1.537 | F | F | H | H | H | CN | |
| 1.538 | F | $CF_3$ | H | H | H | CN | |
| 1.539 | $CF_3$ | F | H | H | H | CN | |
| 1.540 | H | $CCl_3$ | H | H | H | CN | |
| 1.541 | $CCl_3$ | H | H | H | H | CN | |
| 1.542 | Cl | $CCl_3$ | H | H | H | CN | |
| 1.543 | $CCl_3$ | Cl | H | H | H | CN | |
| 1.544 | $CH_3$ | H | H | H | H | CN | |
| 1.545 | Cl | Cl | $CH_3$ | H | H | CN | |
| 1.546 | Cl | H | $CH_3$ | H | H | CN | |
| 1.547 | H | Cl | $CH_3$ | H | H | CN | |
| 1.548 | H | H | $CH_3$ | H | H | CN | |
| 1.549 | Cl | $CF_3$ | $CH_3$ | H | H | CN | |
| 1.550 | $NO_2$ | H | $CH_3$ | H | H | CN | |
| 1.551 | H | $NO_2$ | $CH_3$ | H | H | CN | |
| 1.552 | $NO_2$ | Cl | $CH_3$ | H | H | CN | |
| 1.553 | $CF_3$ | H | $CH_3$ | H | H | CN | |
| 1.554 | H | $CF_3$ | $CH_3$ | H | H | CN | |
| 1.555 | $OCH_3$ | H | $CH_3$ | H | H | CN | |
| 1.556 | CN | H | $CH_3$ | H | H | CN | |
| 1.557 | $OCH_3$ | Cl | $CH_3$ | H | H | CN | |
| 1.558 | CN | Cl | $CH_3$ | H | H | CN | |
| 1.559 | Br | Cl | $CH_3$ | H | H | CN | |
| 1.560 | $SCH_3$ | Cl | $CH_3$ | H | H | CN | |
| 1.561 | $SO_2CH_3$ | Cl | $CH_3$ | H | H | CN | |

| Verb. Nr. | $R^1$ | $R^2$ | $R^3$ | $R^4$ | $R^5$ | $R^6$ | phys. Daten |
|---|---|---|---|---|---|---|---|
| 1.562 | Cl | $SCH_3$ | $CH_3$ | H | H | CN | |
| 1.563 | Cl | $SOCH_3$ | $CH_3$ | H | H | CN | |
| 1.564 | Cl | $SO_2CH_3$ | $CH_3$ | H | H | CN | |
| 1.565 | $SOCH_3$ | Cl | $CH_3$ | H | H | CN | |
| 1.566 | $SO_2CH_3$ | H | $CH_3$ | H | H | CN | |
| 1.567 | H | $SO_2CH_3$ | $CH_3$ | H | H | CN | |
| 1.568 | H | $CH_3$ | $CH_3$ | H | H | CN | |
| 1.569 | Cl | F | $CH_3$ | H | H | CN | |
| 1.570 | H | $CF_3$ | $CH_3$ | H | H | CN | |
| 1.571 | F | F | $CH_3$ | H | H | CN | |
| 1.572 | F | $CF_3$ | $CH_3$ | H | H | CN | |
| 1.573 | $CF_3$ | F | $CH_3$ | H | H | CN | |
| 1.574 | H | $CCl_3$ | $CH_3$ | H | H | CN | |
| 1.575 | $CCl_3$ | H | $CH_3$ | H | H | CN | |
| 1.576 | Cl | $CCl_3$ | $CH_3$ | H | H | CN | |
| 1.577 | $CCl_3$ | Cl | $CH_3$ | H | H | CN | |
| 1.578 | $CH_3$ | H | $CH_3$ | H | H | CN | |
| 1.579 | Cl | Cl | $C_6H_5$ | H | H | $COOCH_3$ | |
| 1.580 | Cl | H | $C_6H_5$ | H | H | $COOCH_3$ | |
| 1.581 | H | Cl | $C_6H_5$ | H | H | $COOCH_3$ | |
| 1.582 | H | H | $C_6H_5$ | H | H | $COOCH_3$ | |
| 1.583 | Cl | $CF_3$ | $C_6H_5$ | H | H | $COOCH_3$ | Fp. >180° Zers. |
| 1.584 | $NO_2$ | H | $C_6H_5$ | H | H | $COOCH_3$ | |
| 1.585 | H | $NO_2$ | $C_6H_5$ | H | H | $COOCH_3$ | |
| 1.586 | $NO_2$ | Cl | $C_6H_5$ | H | H | $COOCH_3$ | |
| 1.587 | $CF_3$ | H | $C_6H_5$ | H | H | $COOCH_3$ | |
| 1.588 | H | $CF_3$ | $C_6H_5$ | H | H | $COOCH_3$ | |
| 1.589 | $OCH_3$ | H | $C_6H_5$ | H | H | $COOCH_3$ | |
| 1.590 | CN | H | $C_6H_5$ | H | H | $COOCH_3$ | |
| 1.591 | $OCH_3$ | Cl | $C_6H_5$ | H | H | $COOCH_3$ | |
| 1.592 | CN | Cl | $C_6H_5$ | H | H | $COOCH_3$ | |
| 1.593 | Br | Cl | $C_6H_5$ | H | H | $COOCH_3$ | |
| 1.594 | $SCH_3$ | Cl | $C_6H_5$ | H | H | $COOCH_3$ | |
| 1.595 | $SO_2CH_3$ | Cl | $C_6H_5$ | H | H | $COOCH_3$ | |
| 1.596 | Cl | $SCH_3$ | $C_6H_5$ | H | H | $COOCH_3$ | |
| 1.597 | Cl | $SOCH_3$ | $C_6H_5$ | H | H | $COOCH_3$ | |
| 1.598 | Cl | $SO_2CH_3$ | $C_6H_5$ | H | H | $COOCH_3$ | |
| 1.599 | $SOCH_3$ | Cl | $C_6H_5$ | H | H | $COOCH_3$ | |
| 1.600 | $SO_2CH_3$ | H | $C_6H_5$ | H | H | $COOCH_3$ | |
| 1.601 | H | $SO_2CH_3$ | $C_6H_5$ | H | H | $COOCH_3$ | |
| 1.602 | H | $CH_3$ | $C_6H_5$ | H | H | $COOCH_3$ | |
| 1.603 | Cl | F | $C_6H_5$ | H | H | $COOCH_3$ | |
| 1.604 | H | $CF_3$ | $C_6H_5$ | H | H | $COOCH_3$ | |
| 1.605 | F | F | $C_6H_5$ | H | H | $COOCH_3$ | |
| 1.606 | F | $CF_3$ | $C_6H_5$ | H | H | $COOCH_3$ | |
| 1.607 | $CF_3$ | F | $C_6H_5$ | H | H | $COOCH_3$ | |
| 1.608 | H | $CCl_3$ | $C_6H_5$ | H | H | $COOCH_3$ | |
| 1.609 | $CCl_3$ | H | $C_6H_5$ | H | H | $COOCH_3$ | |
| 1.610 | Cl | $CCl_3$ | $C_6H_5$ | H | H | $COOCH_3$ | |
| 1.611 | $CCl_3$ | Cl | $C_6H_5$ | H | H | $COOCH_3$ | |
| 1.612 | $CH_3$ | H | $C_6H_5$ | H | H | $COOCH_3$ | |

| Verb. Nr. | $R^1$ | $R^2$ | $R^3$ | $R^4$ | $R^5$ | $R^6$ | phys. Daten |
|---|---|---|---|---|---|---|---|
| 1.613 | H | $SCH_3$ | H | H | H | H | Fp. >140° Z |
| 1.614 | H | $SOCH_3$ | H | H | H | H | |
| 1.615 | H | $SO_2CH_3$ | H | H | H | H | |
| 1.616 | $CO_2H$ | H | H | H | H | H | Fp. >150° Z |
| 1.617 | $CO_2H$ | H | $CH_3$ | H | H | H | |
| 1.618 | $CO_2H$ | H | H | $C_3H_7(i)$ | H | H | |
| 1.619 | $CO_2H$ | H | H | Phenyl | H | H | |
| 1.620 | $CO_2CH_3$ | H | H | H | H | H | |
| 1.621 | $CO_2C_2H_5$ | H | H | H | H | H | |
| 1.622 | $CO_2NH_2$ | H | H | H | H | H | |
| 1.623 | CN | H | H | H | H | H | |
| 1.624 | $CO_2H$ | $CH_3$ | H | H | H | H | |
| 1.625 | $CO_2CH_3$ | $CH_3$ | H | H | H | H | |
| 1.626 | $CO_2H$ | $C_2H_5$ | H | H | H | H | |
| 1.627 | $CO_2CH_3$ | $C_2H_5$ | H | H | H | H | |
| 1.628 | $CO_2CH_3$ | Br | H | H | H | H | |
| 1.629 | $CO_2H$ | Br | H | H | H | H | |
| 1.630 | Cl | $CF_3$ | $CH_3$ | $CH_3$ | $CH_3$ | H | Fp. 65-70°C |
| 1.631 | H | $CF_3$ | $CH_3$ | $CH_3$ | $CH_3$ | H | |
| 1.632 | Cl | $OCH_3$ | H | H | H | H | |
| 1.633 | Cl | $SC_2H_5$ | H | H | H | H | |
| 1.634 | Cl | $SOC_2H_5$ | H | H | H | H | |
| 1.635 | Cl | $SO_2C_2H_5$ | H | H | H | H | |
| 1.636 | $SC_2H_5$ | Cl | H | H | H | H | |
| 1.637 | $SOC_2H_5$ | Cl | H | H | H | H | |
| 1.638 | $SO_2C_2H_5$ | Cl | H | H | H | H | |
| 1.639 | H | $SO_2C_2H_5$ | H | H | H | H | |
| 1.640 | Cl | $SO_2C_3H_7(i)$ | H | H | H | H | |
| 1.641 | Cl | $SOC_3H_7(i)$ | H | H | H | H | |
| 1.642 | Cl | $SC_3H_7(i)$ | H | H | H | H | |
| 1.643 | Cl | $OCH_3$ | H | H | H | H | |
| 1.644 | Cl | $OC_3H_7(i)$ | H | H | H | H | |
| 1.645 | Cl | Br | H | H | H | H | |
| 1.646 | H | $OCH_3$ | H | H | H | H | Fp. 79-85°C |

H.1.2. Herstellung von Salzen der Formel I

H.1.2.1. Natriumsalz von 2-(3-Chlor-5-trifluormethylpyridin-2-yl-carbonyl)-cyclohex-1-en-1-ol-3-on

3,2 g 2-(3-Chlor-5-trifluormethylpyridin-2-yl-carbonyl)-cyclohex-1-en-1-ol-3-on werden in 20 ml Methanol gelöst, mit 0,54 g Natriummethylat versetzt und 15 min bei Raumtemperatur gerührt. Danach wird am Rotationsverdampfer zur Trockne eingeengt. Der Rückstand wird mit Diethyläther verrieben, abfiltriert und getrocknet.

Man isoliert in quantitativer Ausbeute die Titelverbindung der Formel

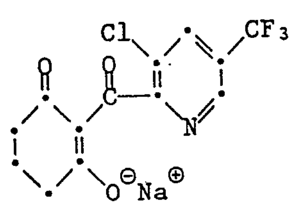

als farblosen Festkörper (Verb. No. 2.006).

Analog zu vorstehenden Herstellungsverfahren können die Salze der Tabelle 2 synthetisiert werden.

Tabelle 2

Verbindungen der Formel

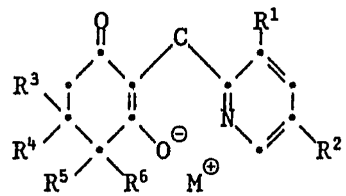

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.001 | Cl | Cl | H | H | H | H | $Na^{\oplus}$ | |
| 2.002 | Cl | Cl | H | H | H | H | $Li^{\oplus}$ | |
| 2.003 | Cl | Cl | H | H | H | H | 1/2 $Ca^{2\oplus}$ | |
| 2.004 | Cl | Cl | H | H | H | H | $NH(CH_3)_3^{\oplus}$ | |
| 2.005 | Cl | Cl | H | H | H | H | $NH(C_2H_5OH)_3^{\oplus}$ | |
| 2.006 | Cl | $CF_3$ | H | H | H | H | $Na^{\oplus}$ | farbloser Festkörper |
| 2.007 | Cl | $CF_3$ | H | H | H | H | $Li^{\oplus}$ | |
| 2.008 | Cl | $CF_3$ | H | H | H | H | 1/2 $Ca^{2\oplus}$ | |
| 2.009 | Cl | $CF_3$ | H | H | H | H | $NH(CH_3)_3^{\oplus}$ | |
| 2.010 | Cl | $CF_3$ | H | H | H | H | $NH(C_2H_5OH)_3^{\oplus}$ | |
| 2.011 | $NO_2$ | H | H | H | H | H | $Na^{\oplus}$ | |
| 2.012 | $NO_2$ | H | H | H | H | H | $Li^{\oplus}$ | |
| 2.013 | $NO_2$ | H | H | H | H | H | 1/2 $Ca^{2\oplus}$ | |
| 2.014 | $NO_2$ | H | H | H | H | H | $NH(CH_3)_3^{\oplus}$ | |
| 2.015 | $NO_2$ | H | H | H | H | H | $NH(C_2H_5OH)_3^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.016 | H | $NO_2$ | H | H | H | H | $Na^{\oplus}$ | |
| 2.017 | H | $NO_2$ | H | H | H | H | $Li^{\oplus}$ | |
| 2.018 | H | $NO_2$ | H | H | H | H | 1/2 $Ca^{2\oplus}$ | |
| 2.019 | H | $NO_2$ | H | H | H | H | $NH(CH_3)_3^{\oplus}$ | |
| 2.020 | H | $NO_2$ | H | H | H | H | $NH(C_2H_5OH)_3^{\oplus}$ | |
| 2.021 | H | H | H | H | H | H | $Na^{\oplus}$ | |
| 2.022 | H | H | H | H | H | H | $Li^{\oplus}$ | |
| 2.023 | H | H | H | H | H | H | 1/2 $Ca^{2\oplus}$ | |
| 2.024 | H | H | H | H | H | H | $NH(CH_3)_3^{\oplus}$ | |
| 2.025 | H | H | H | H | H | H | $NH(C_2H_5OH)_3^{\oplus}$ | |
| 2.026 | Cl | $CCl_3$ | H | H | H | H | $Na^{\oplus}$ | |
| 2.027 | Cl | $CCl_3$ | H | H | H | H | $Li^{\oplus}$ | |
| 2.028 | Cl | $CCl_3$ | H | H | H | H | 1/2 $Ca^{2\oplus}$ | |
| 2.029 | Cl | $CCl_3$ | H | H | H | H | $NH(CH_3)_3^{\oplus}$ | |
| 2.030 | Cl | $CCl_3$ | H | H | H | H | $NH(C_2H_5OH)_3^{\oplus}$ | |
| 2.031 | F | $CF_3$ | H | H | H | H | $Na^{\oplus}$ | |
| 2.032 | F | $CF_3$ | H | H | H | H | $Li^{\oplus}$ | |
| 2.033 | F | $CF_3$ | H | H | H | H | 1/2 $Ca^{2\oplus}$ | |
| 2.034 | F | $CF_3$ | H | H | H | H | $NH(CH_3)_3^{\oplus}$ | |
| 2.035 | F | $CF_3$ | H | H | H | H | $NH(C_2H_5OH)_3^{\oplus}$ | |
| 2.036 | Cl | Cl | $CH_3$ | H | H | H | $Na^{\oplus}$ | |
| 2.037 | Cl | Cl | $CH_3$ | H | H | H | $Li^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.038 | Cl | Cl | $CH_3$ | H | H | H | 1/2 $Ca^{2\oplus}$ | |
| 2.039 | Cl | Cl | $CH_3$ | H | H | H | $NH(CH_3)_3^{\oplus}$ | |
| 2.040 | Cl | Cl | $CH_3$ | H | H | H | $NH(C_2H_5OH)_3^{\oplus}$ | |
| 2.041 | Cl | $CF_3$ | $CH_3$ | H | H | H | $Na^{\oplus}$ | |
| 2.042 | Cl | $CF_3$ | $CH_3$ | H | H | H | $Li^{\oplus}$ | |
| 2.043 | Cl | $CF_3$ | $CH_3$ | H | H | H | 1/2 $Ca^{2\oplus}$ | |
| 2.044 | Cl | $CF_3$ | $CH_3$ | H | H | H | $NH(CH_3)_3^{\oplus}$ | |
| 2.045 | Cl | $CF_3$ | $CH_3$ | H | H | H | $NH(C_2H_5OH)_3^{\oplus}$ | |
| 2.046 | $NO_2$ | H | $CH_3$ | H | H | H | $Na^{\oplus}$ | |
| 2.047 | $NO_2$ | H | $CH_3$ | H | H | H | $Li^{\oplus}$ | |
| 2.048 | $NO_2$ | H | $CH_3$ | H | H | H | 1/2 $Ca^{2\oplus}$ | |
| 2.049 | $NO_2$ | H | $CH_3$ | H | H | H | $NH(CH_3)_3^{\oplus}$ | |
| 2.050 | $NO_2$ | H | $CH_3$ | H | H | H | $NH(C_2H_5OH)_3^{\oplus}$ | |
| 2.051 | H | $NO_2$ | $CH_3$ | H | H | H | $Na^{\oplus}$ | |
| 2.052 | H | $NO_2$ | $CH_3$ | H | H | H | $Li^{\oplus}$ | |
| 2.053 | H | $NO_2$ | $CH_3$ | H | H | H | 1/2 $Ca^{2\oplus}$ | |
| 2.054 | H | $NO_2$ | $CH_3$ | H | H | H | $NH(CH_3)_3^{\oplus}$ | |
| 2.055 | H | $NO_2$ | $CH_3$ | H | H | H | $NH(C_2H_5OH)_3^{\oplus}$ | |
| 2.056 | H | H | $CH_3$ | H | H | H | $Na^{\oplus}$ | |
| 2.057 | H | H | $CH_3$ | H | H | H | $Li^{\oplus}$ | |
| 2.058 | H | H | $CH_3$ | H | H | H | 1/2 $Ca^{2\oplus}$ | |
| 2.059 | H | H | $CH_3$ | H | H | H | $NH(CH_3)_3^{\oplus}$ | |

| Verb. Nr. | $R^1$ | $R^2$ | $R^3$ | $R^4$ | $R^5$ | $R^6$ | M | phys. Daten |
|---|---|---|---|---|---|---|---|---|
| 2.060 | H | H | $CH_3$ | H | H | H | $NH(C_2H_5OH)_3^{\oplus}$ | |
| 2.061 | Cl | $CCl_3$ | $CH_3$ | H | H | H | $Na^{\oplus}$ | |
| 2.062 | Cl | $CCl_3$ | $CH_3$ | H | H | H | $Li^{\oplus}$ | |
| 2.063 | Cl | $CCl_3$ | $CH_3$ | H | H | H | 1/2 $Ca^{2\oplus}$ | |
| 2.064 | Cl | $CCl_3$ | $CH_3$ | H | H | H | $NH(CH_3)_3^{\oplus}$ | |
| 2.065 | Cl | $CCl_3$ | $CH_3$ | H | H | H | $NH(C_2H_5OH)_3^{\oplus}$ | |
| 2.066 | F | $CF_3$ | $CH_3$ | H | H | H | $Na^{\oplus}$ | |
| 2.067 | F | $CF_3$ | $CH_3$ | H | H | H | $Li^{\oplus}$ | |
| 2.068 | F | $CF_3$ | $CH_3$ | H | H | H | 1/2 $Ca^{2\oplus}$ | |
| 2.069 | F | $CF_3$ | $CH_3$ | H | H | H | $NH(CH_3)_3^{\oplus}$ | |
| 2.070 | F | $CF_3$ | $CH_3$ | H | H | H | $NH(C_2H_5OH)_3^{\oplus}$ | |
| 2.071 | Cl | Cl | n-$C_3H_7$ | H | H | H | $Na^{\oplus}$ | |
| 2.072 | Cl | Cl | n-$C_3H_7$ | H | H | H | $Li^{\oplus}$ | |
| 2.073 | Cl | Cl | n-$C_3H_7$ | H | H | H | 1/2 $Ca^{2\oplus}$ | |
| 2.074 | Cl | Cl | n-$C_3H_7$ | H | H | H | $NH(CH_3)_3^{\oplus}$ | |
| 2.075 | Cl | Cl | n-$C_3H_7$ | H | H | H | $NH(C_2H_5OH)_3^{\oplus}$ | |
| 2.076 | Cl | $CF_3$ | n-$C_3H_7$ | H | H | H | $Na^{\oplus}$ | |
| 2.077 | Cl | $CF_3$ | n-$C_3H_7$ | H | H | H | $Li^{\oplus}$ | |
| 2.078 | Cl | $CF_3$ | n-$C_3H_7$ | H | H | H | 1/2 $Ca^{2\oplus}$ | |
| 2.079 | Cl | $CF_3$ | n-$C_3H_7$ | H | H | H | $NH(CH_3)_3^{\oplus}$ | |
| 2.080 | Cl | $CF_3$ | n-$C_3H_7$ | H | H | H | $NH(C_2H_5OH)_3^{\oplus}$ | |
| 2.081 | $NO_2$ | H | n-$C_3H_7$ | H | H | H | $Na^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.082 | $NO_2$ | H | n-$C_3H_7$ | H | H | H | $Li^{\oplus}$ | |
| 2.083 | $NO_2$ | H | n-$C_3H_7$ | H | H | H | 1/2 $Ca^{2\oplus}$ | |
| 2.084 | $NO_2$ | H | n-$C_3H_7$ | H | H | H | $NH(CH_3)_3^{\oplus}$ | |
| 2.085 | $NO_2$ | H | n-$C_3H_7$ | H | H | H | $NH(C_2H_5OH)_3^{\oplus}$ | |
| 2.086 | H | $NO_2$ | n-$C_3H_7$ | H | H | H | $Na^{\oplus}$ | |
| 2.087 | H | $NO_2$ | n-$C_3H_7$ | H | H | H | $Li^{\oplus}$ | |
| 2.088 | H | $NO_2$ | n-$C_3H_7$ | H | H | H | 1/2 $Ca^{2\oplus}$ | |
| 2.089 | H | $NO_2$ | n-$C_3H_7$ | H | H | H | $NH(CH_3)_3^{\oplus}$ | |
| 2.090 | H | $NO_2$ | n-$C_3H_7$ | H | H | H | $NH(C_2H_5OH)_3^{\oplus}$ | |
| 2.091 | H | H | n-$C_3H_7$ | H | H | H | $Na^{\oplus}$ | |
| 2.092 | H | H | n-$C_3H_7$ | H | H | H | $Li^{\oplus}$ | |
| 2.093 | H | H | n-$C_3H_7$ | H | H | H | 1/2 $Ca^{2\oplus}$ | |
| 2.094 | H | H | n-$C_3H_7$ | H | H | H | $NH(CH_3)_3^{\oplus}$ | |
| 2.095 | H | H | n-$C_3H_7$ | H | H | H | $NH(C_2H_5OH)_3^{\oplus}$ | |
| 2.096 | Cl | $CCl_3$ | n-$C_3H_7$ | H | H | H | $Na^{\oplus}$ | |
| 2.097 | Cl | $CCl_3$ | n-$C_3H_7$ | H | H | H | $Li^{\oplus}$ | |
| 2.098 | Cl | $CCl_3$ | n-$C_3H_7$ | H | H | H | 1/2 $Ca^{2\oplus}$ | |
| 2.099 | Cl | $CCl_3$ | n-$C_3H_7$ | H | H | H | $NH(CH_3)_3^{\oplus}$ | |
| 2.100 | Cl | $CCl_3$ | n-$C_3H_7$ | H | H | H | $NH(C_2H_5OH)_3^{\oplus}$ | |
| 2.101 | F | $CF_3$ | n-$C_3H_7$ | H | H | H | $Na^{\oplus}$ | |
| 2.102 | F | $CF_3$ | n-$C_3H_7$ | H | H | H | $Li^{\oplus}$ | |
| 2.103 | F | $CF_3$ | n-$C_3H_7$ | H | H | H | 1/2 $Ca^{2\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.104 | F | $CF_3$ | n-$C_3H_7$ | H | H | H | $NH(CH_3)_3^{\oplus}$ | |
| 2.105 | F | $CF_3$ | n-$C_3H_7$ | H | H | H | $NH(C_2H_5OH)_3^{\oplus}$ | |
| 2.106 | Cl | Cl | i-$C_3H_7$ | H | H | H | $Na^{\oplus}$ | |
| 2.107 | Cl | Cl | i-$C_3H_7$ | H | H | H | $Li^{\oplus}$ | |
| 2.108 | Cl | Cl | i-$C_3H_7$ | H | H | H | 1/2 $Ca^{2\oplus}$ | |
| 2.109 | Cl | Cl | i-$C_3H_7$ | H | H | H | $NH(CH_3)_3^{\oplus}$ | |
| 2.110 | Cl | Cl | i-$C_3H_7$ | H | H | H | $NH(C_2H_5OH)_3^{\oplus}$ | |
| 2.111 | Cl | $CF_3$ | i-$C_3H_7$ | H | H | H | $Na^{\oplus}$ | |
| 2.112 | Cl | $CF_3$ | i-$C_3H_7$ | H | H | H | $Li^{\oplus}$ | |
| 2.113 | Cl | $CF_3$ | i-$C_3H_7$ | H | H | H | 1/2 $Ca^{2\oplus}$ | |
| 2.114 | Cl | $CF_3$ | i-$C_3H_7$ | H | H | H | $NH(CH_3)_3^{\oplus}$ | |
| 2.115 | Cl | $CF_3$ | i-$C_3H_7$ | H | H | H | $NH(C_2H_5OH)_3^{\oplus}$ | |
| 2.116 | $NO_2$ | H | i-$C_3H_7$ | H | H | H | $Na^{\oplus}$ | |
| 2.117 | $NO_2$ | H | i-$C_3H_7$ | H | H | H | $Li^{\oplus}$ | |
| 2.118 | $NO_2$ | H | i-$C_3H_7$ | H | H | H | 1/2 $Ca^{2\oplus}$ | |
| 2.119 | $NO_2$ | H | i-$C_3H_7$ | H | H | H | $NH(CH_3)_3^{\oplus}$ | |
| 2.120 | $NO_2$ | H | i-$C_3H_7$ | H | H | H | $NH(C_2H_5OH)_3^{\oplus}$ | |
| 2.121 | H | $NO_2$ | i-$C_3H_7$ | H | H | H | $Na^{\oplus}$ | |
| 2.122 | H | $NO_2$ | i-$C_3H_7$ | H | H | H | $Li^{\oplus}$ | |
| 2.123 | H | $NO_2$ | i-$C_3H_7$ | H | H | H | 1/2 $Ca^{2\oplus}$ | |
| 2.124 | H | $NO_2$ | i-$C_3H_7$ | H | H | H | $NH(CH_3)_3^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.125 | H | $NO_2$ | $i-C_3H_7$ | H | H | H | $NH(C_2H_5OH)_3^{\oplus}$ | |
| 2.126 | H | H | $i-C_3H_7$ | H | H | H | $Na^{\oplus}$ | |
| 2.127 | H | H | $i-C_3H_7$ | H | H | H | $Li^{\oplus}$ | |
| 2.128 | H | H | $i-C_3H_7$ | H | H | H | 1/2 $Ca^{2\oplus}$ | |
| 2.129 | H | H | $i-C_3H_7$ | H | H | H | $NH(CH_3)_3^{\oplus}$ | |
| 2.130 | H | H | $i-C_3H_7$ | H | H | H | $NH(C_2H_5OH)_3^{\oplus}$ | |
| 2.131 | Cl | $CCl_3$ | $i-C_3H_7$ | H | H | H | $Na^{\oplus}$ | |
| 2.132 | Cl | $CCl_3$ | $i-C_3H_7$ | H | H | H | $Li^{\oplus}$ | |
| 2.133 | Cl | $CCl_3$ | $i-C_3H_7$ | H | H | H | 1/2 $Ca^{2\oplus}$ | |
| 2.134 | Cl | $CCl_3$ | $i-C_3H_7$ | H | H | H | $NH(CH_3)_3^{\oplus}$ | |
| 2.135 | Cl | $CCl_3$ | $i-C_3H_7$ | H | H | H | $NH(C_2H_5OH)_3^{\oplus}$ | |
| 2.136 | F | $CF_3$ | $i-C_3H_7$ | H | H | H | $Na^{\oplus}$ | |
| 2.137 | F | $CF_3$ | $i-C_3H_7$ | H | H | H | $Li^{\oplus}$ | |
| 2.138 | F | $CF_3$ | $i-C_3H_7$ | H | H | H | 1/2 $Ca^{2\oplus}$ | |
| 2.139 | F | $CF_3$ | $i-C_3H_7$ | H | H | H | $NH(CH_3)_3^{\oplus}$ | |
| 2.140 | F | $CF_3$ | $i-C_3H_7$ | H | H | H | $NH(C_2H_5OH)_3^{\oplus}$ | |
| 2.141 | Cl | Cl | $C_6H_5$ | H | H | H | $Na^{\oplus}$ | |
| 2.142 | Cl | Cl | $C_6H_5$ | H | H | H | $Li^{\oplus}$ | |
| 2.143 | Cl | Cl | $C_6H_5$ | H | H | H | 1/2 $Ca^{2\oplus}$ | |
| 2.144 | Cl | Cl | $C_6H_5$ | H | H | H | $NH(CH_3)_3^{\oplus}$ | |
| 2.145 | Cl | Cl | $C_6H_5$ | H | H | H | $NH(C_2H_5OH)_3^{\oplus}$ | |
| 2.146 | Cl | $CF_3$ | $C_6H_5$ | H | H | H | $Na^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.147 | Cl | $CF_3$ | $C_6H_5$ | H | H | H | $Li^{\oplus}$ | |
| 2.148 | Cl | $CF_3$ | $C_6H_5$ | H | H | H | 1/2 $Ca^{2\oplus}$ | |
| 2.149 | Cl | $CF_3$ | $C_6H_5$ | H | H | H | $NH(CH_3)_3^{\oplus}$ | |
| 2.150 | Cl | $CF_3$ | $C_6H_5$ | H | H | H | $NH(C_2H_5OH)_3^{\oplus}$ | |
| 2.151 | $NO_2$ | H | $C_6H_5$ | H | H | H | $Na^{\oplus}$ | |
| 2.152 | $NO_2$ | H | $C_6H_5$ | H | H | H | $Li^{\oplus}$ | |
| 2.153 | $NO_2$ | H | $C_6H_5$ | H | H | H | 1/2 $Ca^{2\oplus}$ | |
| 2.154 | $NO_2$ | H | $C_6H_5$ | H | H | H | $NH(CH_3)_3^{\oplus}$ | |
| 2.155 | $NO_2$ | H | $C_6H_5$ | H | H | H | $NH(C_2H_5OH)_3^{\oplus}$ | |
| 2.156 | H | $NO_2$ | $C_6H_5$ | H | H | H | $Na^{\oplus}$ | |
| 2.157 | H | $NO_2$ | $C_6H_5$ | H | H | H | $Li^{\oplus}$ | |
| 2.158 | H | $NO_2$ | $C_6H_5$ | H | H | H | 1/2 $Ca^{2\oplus}$ | |
| 2.159 | H | $NO_2$ | $C_6H_5$ | H | H | H | $NH(CH_3)_3^{\oplus}$ | |
| 2.160 | H | $NO_2$ | $C_6H_5$ | H | H | H | $NH(C_2H_5OH)_3^{\oplus}$ | |
| 2.161 | H | H | $C_6H_5$ | H | H | H | $Na^{\oplus}$ | |
| 2.162 | H | H | $C_6H_5$ | H | H | H | $Li^{\oplus}$ | |
| 2.163 | H | H | $C_6H_5$ | H | H | H | 1/2 $Ca^{2\oplus}$ | |
| 2.164 | H | H | $C_6H_5$ | H | H | H | $NH(CH_3)_3^{\oplus}$ | |
| 2.165 | H | H | $C_6H_5$ | H | H | H | $NH(C_2H_5OH)_3^{\oplus}$ | |
| 2.166 | Cl | $CCl_3$ | $C_6H_5$ | H | H | H | $Na^{\oplus}$ | |
| 2.167 | Cl | $CCl_3$ | $C_6H_5$ | H | H | H | $Li^{\oplus}$ | |
| 2.168 | Cl | $CCl_3$ | $C_6H_5$ | H | H | H | 1/2 $Ca^{2\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.169 | Cl | $CCl_3$ | $C_6H_5$ | H | H | H | $NH(CH_3)_3^{\oplus}$ | |
| 2.170 | Cl | $CCl_3$ | $C_6H_5$ | H | H | H | $NH(C_2H_5OH)_3^{\oplus}$ | |
| 2.171 | F | $CF_3$ | $C_6H_5$ | H | H | H | $Na^{\oplus}$ | |
| 2.172 | F | $CF_3$ | $C_6H_5$ | H | H | H | $Li^{\oplus}$ | |
| 2.173 | F | $CF_3$ | $C_6H_5$ | H | H | H | 1/2 $Ca^{2\oplus}$ | |
| 2.174 | F | $CF_3$ | $C_6H_5$ | H | H | H | $NH(CH_3)_3^{\oplus}$ | |
| 2.175 | F | $CF_3$ | $C_6H_5$ | H | H | H | $NH(C_2H_5OH)_3^{\oplus}$ | |
| 2.176 | Cl | Cl | $CH_3$ | $CH_3$ | H | H | $Na^{\oplus}$ | |
| 2.177 | Cl | Cl | $CH_3$ | $CH_3$ | H | H | $Li^{\oplus}$ | |
| 2.178 | Cl | Cl | $CH_3$ | $CH_3$ | H | H | 1/2 $Ca^{2\oplus}$ | |
| 2.179 | Cl | Cl | $CH_3$ | $CH_3$ | H | H | $NH(CH_3)_3^{\oplus}$ | |
| 2.180 | Cl | Cl | $CH_3$ | $CH_3$ | H | H | $NH(C_2H_5OH)_3^{\oplus}$ | |
| 2.181 | Cl | $CF_3$ | $CH_3$ | $CH_3$ | H | H | $Na^{\oplus}$ | |
| 2.182 | Cl | $CF_3$ | $CH_3$ | $CH_3$ | H | H | $Li^{\oplus}$ | |
| 2.183 | Cl | $CF_3$ | $CH_3$ | $CH_3$ | H | H | 1/2 $Ca^{2\oplus}$ | |
| 2.184 | Cl | $CF_3$ | $CH_3$ | $CH_3$ | H | H | $NH(CH_3)_3^{\oplus}$ | |
| 2.185 | Cl | $CF_3$ | $CH_3$ | $CH_3$ | H | H | $NH(C_2H_5OH)_3^{\oplus}$ | |
| 2.186 | $NO_2$ | H | $CH_3$ | $CH_3$ | H | H | $Na^{\oplus}$ | |
| 2.187 | $NO_2$ | H | $CH_3$ | $CH_3$ | H | H | $Li^{\oplus}$ | |
| 2.188 | $NO_2$ | H | $CH_3$ | $CH_3$ | H | H | 1/2 $Ca^{2\oplus}$ | |
| 2.189 | $NO_2$ | H | $CH_3$ | $CH_3$ | H | H | $NH(CH_3)_3^{\oplus}$ | |
| 2.190 | $NO_2$ | H | $CH_3$ | $CH_3$ | H | H | $NH(C_2H_5OH)_3^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.191 | H | $NO_2$ | $CH_3$ | $CH_3$ | H | H | $Na^{\oplus}$ | |
| 2.192 | H | $NO_2$ | $CH_3$ | $CH_3$ | H | H | $Li^{\oplus}$ | |
| 2.193 | H | $NO_2$ | $CH_3$ | $CH_3$ | H | H | 1/2 $Ca^{2\oplus}$ | |
| 2.194 | H | $NO_2$ | $CH_3$ | $CH_3$ | H | H | $NH(CH_3)_3^{\oplus}$ | |
| 2.195 | H | $NO_2$ | $CH_3$ | $CH_3$ | H | H | $NH(C_2H_5OH)_3^{\oplus}$ | |
| 2.196 | H | H | $CH_3$ | $CH_3$ | H | H | $Na^{\oplus}$ | |
| 2.197 | H | H | $CH_3$ | $CH_3$ | H | H | $Li^{\oplus}$ | |
| 2.198 | H | H | $CH_3$ | $CH_3$ | H | H | 1/2 $Ca^{2\oplus}$ | |
| 2.199 | H | H | $CH_3$ | $CH_3$ | H | H | $NH(CH_3)_3^{\oplus}$ | |
| 2.200 | H | H | $CH_3$ | $CH_3$ | H | H | $NH(C_2H_5OH)_3^{\oplus}$ | |
| 2.201 | Cl | $CCl_3$ | $CH_3$ | $CH_3$ | H | H | $Na^{\oplus}$ | |
| 2.202 | Cl | $CCl_3$ | $CH_3$ | $CH_3$ | H | H | $Li^{\oplus}$ | |
| 2.203 | Cl | $CCl_3$ | $CH_3$ | $CH_3$ | H | H | 1/2 $Ca^{2\oplus}$ | |
| 2.204 | Cl | $CCl_3$ | $CH_3$ | $CH_3$ | H | H | $NH(CH_3)_3^{\oplus}$ | |
| 2.205 | Cl | $CCl_3$ | $CH_3$ | $CH_3$ | H | H | $NH(C_2H_5OH)_3^{\oplus}$ | |
| 2.206 | F | $CF_3$ | $CH_3$ | $CH_3$ | H | H | $Na^{\oplus}$ | |
| 2.207 | F | $CF_3$ | $CH_3$ | $CH_3$ | H | H | $Li^{\oplus}$ | |
| 2.208 | F | $CF_3$ | $CH_3$ | $CH_3$ | H | H | 1/2 $Ca^{2\oplus}$ | |
| 2.209 | F | $CF_3$ | $CH_3$ | $CH_3$ | H | H | $NH(CH_3)_3^{\oplus}$ | |
| 2.210 | F | $CF_3$ | $CH_3$ | $CH_3$ | H | H | $NH(C_2H_5OH)_3^{\oplus}$ | |
| 2.211 | Cl | Cl | $CH_3$ | H | $CH_3$ | H | $Na^{\oplus}$ | |
| 2.212 | Cl | Cl | $CH_3$ | H | $CH_3$ | H | $Li^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.213 | Cl | Cl | $CH_3$ | H | $CH_3$ | H | $1/2\ Ca^{2\oplus}$ | |
| 2.214 | Cl | Cl | $CH_3$ | H | $CH_3$ | H | $NH(CH_3)_3^{\oplus}$ | |
| 2.215 | Cl | Cl | $CH_3$ | H | $CH_3$ | H | $NH(C_2H_5OH)_3^{\oplus}$ | |
| 2.216 | Cl | $CF_3$ | $CH_3$ | H | $CH_3$ | H | $Na^{\oplus}$ | |
| 2.217 | Cl | $CF_3$ | $CH_3$ | H | $CH_3$ | H | $Li^{\oplus}$ | |
| 2.218 | Cl | $CF_3$ | $CH_3$ | H | $CH_3$ | H | $1/2\ Ca^{2\oplus}$ | |
| 2.219 | Cl | $CF_3$ | $CH_3$ | H | $CH_3$ | H | $NH(CH_3)_3^{\oplus}$ | |
| 2.220 | Cl | $CF_3$ | $CH_3$ | H | $CH_3$ | H | $NH(C_2H_5OH)_3^{\oplus}$ | |
| 2.221 | $NO_2$ | H | $CH_3$ | H | $CH_3$ | H | $Na^{\oplus}$ | |
| 2.222 | $NO_2$ | H | $CH_3$ | H | $CH_3$ | H | $Li^{\oplus}$ | |
| 2.223 | $NO_2$ | H | $CH_3$ | H | $CH_3$ | H | $1/2\ Ca^{2\oplus}$ | |
| 2.224 | $NO_2$ | H | $CH_3$ | H | $CH_3$ | H | $NH(CH_3)_3^{\oplus}$ | |
| 2.225 | $NO_2$ | H | $CH_3$ | H | $CH_3$ | H | $NH(C_2H_5OH)_3^{\oplus}$ | |
| 2.226 | H | $NO_2$ | $CH_3$ | H | $CH_3$ | H | $Na^{\oplus}$ | |
| 2.227 | H | $NO_2$ | $CH_3$ | H | $CH_3$ | H | $Li^{\oplus}$ | |
| 2.228 | H | $NO_2$ | $CH_3$ | H | $CH_3$ | H | $1/2\ Ca^{2\oplus}$ | |
| 2.229 | H | $NO_2$ | $CH_3$ | H | $CH_3$ | H | $NH(CH_3)_3^{\oplus}$ | |
| 2.230 | H | $NO_2$ | $CH_3$ | H | $CH_3$ | H | $NH(C_2H_5OH)_3^{\oplus}$ | |
| 2.231 | H | H | $CH_3$ | H | $CH_3$ | H | $Na^{\oplus}$ | |
| 2.232 | H | H | $CH_3$ | H | $CH_3$ | H | $Li^{\oplus}$ | |
| 2.233 | H | H | $CH_3$ | H | $CH_3$ | H | $1/2\ Ca^{2\oplus}$ | |
| 2.234 | H | H | $CH_3$ | H | $CH_3$ | H | $NH(CH_3)_3^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.235 | H | H | $CH_3$ | H | $CH_3$ | H | $NH(C_2H_5OH)_3^{\oplus}$ | |
| 2.236 | Cl | $CCl_3$ | $CH_3$ | H | $CH_3$ | H | $Na^{\oplus}$ | |
| 2.237 | Cl | $CCl_3$ | $CH_3$ | H | $CH_3$ | H | $Li^{\oplus}$ | |
| 2.238 | Cl | $CCl_3$ | $CH_3$ | H | $CH_3$ | H | 1/2 $Ca^{2\oplus}$ | |
| 2.239 | Cl | $CCl_3$ | $CH_3$ | H | $CH_3$ | H | $NH(CH_3)_3^{\oplus}$ | |
| 2.240 | Cl | $CCl_3$ | $CH_3$ | H | $CH_3$ | H | $NH(C_2H_5OH)_3^{\oplus}$ | |
| 2.241 | F | $CF_3$ | $CH_3$ | H | $CH_3$ | H | $Na^{\oplus}$ | |
| 2.242 | F | $CF_3$ | $CH_3$ | H | $CH_3$ | H | $Li^{\oplus}$ | |
| 2.243 | F | $CF_3$ | $CH_3$ | H | $CH_3$ | H | 1/2 $Ca^{2\oplus}$ | |
| 2.244 | F | $CF_3$ | $CH_3$ | H | $CH_3$ | H | $NH(CH_3)_3^{\oplus}$ | |
| 2.245 | F | $CF_3$ | $CH_3$ | H | $CH_3$ | H | $NH(C_2H_5OH)_3^{\oplus}$ | |
| 2.246 | Cl | Cl | H | H | H | CN | $Na^{\oplus}$ | |
| 2.247 | Cl | Cl | H | H | H | CN | $Li^{\oplus}$ | |
| 2.248 | Cl | Cl | H | H | H | CN | 1/2 $Ca^{2\oplus}$ | |
| 2.249 | Cl | Cl | H | H | H | CN | $NH(CH_3)_3^{\oplus}$ | |
| 2.250 | Cl | Cl | H | H | H | CN | $NH(C_2H_5OH)_3^{\oplus}$ | |
| 2.251 | Cl | $CF_3$ | H | H | H | CN | $Na^{\oplus}$ | |
| 2.252 | Cl | $CF_3$ | H | H | H | CN | $Li^{\oplus}$ | |
| 2.253 | Cl | $CF_3$ | H | H | H | CN | 1/2 $Ca^{2\oplus}$ | |
| 2.254 | Cl | $CF_3$ | H | H | H | CN | $NH(CH_3)_3^{\oplus}$ | |
| 2.255 | Cl | $CF_3$ | H | H | H | CN | $NH(C_2H_5OH)_3^{\oplus}$ | |
| 2.256 | $NO_2$ | H | H | H | H | CN | $Na^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.257 | $NO_2$ | H | H | H | H | CN | $Li^{\oplus}$ | |
| 2.258 | $NO_2$ | H | H | H | H | CN | 1/2 $Ca^{2\oplus}$ | |
| 2.259 | $NO_2$ | H | H | H | H | CN | $NH(CH_3)_3^{\oplus}$ | |
| 2.260 | $NO_2$ | H | H | H | H | CN | $NH(C_2H_5OH)_3^{\oplus}$ | |
| 2.261 | H | $NO_2$ | H | H | H | CN | $Na^{\oplus}$ | |
| 2.262 | H | $NO_2$ | H | H | H | CN | $Li^{\oplus}$ | |
| 2.263 | H | $NO_2$ | H | H | H | CN | 1/2 $Ca^{2\oplus}$ | |
| 2.264 | H | $NO_2$ | H | H | H | CN | $NH(CH_3)_3^{\oplus}$ | |
| 2.265 | H | $NO_2$ | H | H | H | CN | $NH(C_2H_5OH)_3^{\oplus}$ | |
| 2.266 | H | H | H | H | H | CN | $Na^{\oplus}$ | |
| 2.267 | H | H | H | H | H | CN | $Li^{\oplus}$ | |
| 2.268 | H | H | H | H | H | CN | 1/2 $Ca^{2\oplus}$ | |
| 2.269 | H | H | H | H | H | CN | $NH(CH_3)_3^{\oplus}$ | |
| 2.270 | H | H | H | H | H | CN | $NH(C_2H_5OH)_3^{\oplus}$ | |
| 2.271 | Cl | $CCl_3$ | H | H | H | CN | $Na^{\oplus}$ | |
| 2.272 | Cl | $CCl_3$ | H | H | H | CN | $Li^{\oplus}$ | |
| 2.273 | Cl | $CCl_3$ | H | H | H | CN | 1/2 $Ca^{2\oplus}$ | |
| 2.274 | Cl | $CCl_3$ | H | H | H | CN | $NH(CH_3)_3^{\oplus}$ | |
| 2.275 | Cl | $CCl_3$ | H | H | H | CN | $NH(C_2H_5OH)_3^{\oplus}$ | |
| 2.276 | F | $CF_3$ | H | H | H | CN | $Na^{\oplus}$ | |
| 2.277 | F | $CF_3$ | H | H | H | CN | $Li^{\oplus}$ | |
| 2.278 | F | $CF_3$ | H | H | H | CN | 1/2 $Ca^{2\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.279 | F | $CF_3$ | H | H | H | CN | $NH(CH_3)_3^{\oplus}$ | |
| 2.280 | F | $CF_3$ | H | H | H | CN | $NH(C_2H_5OH)_3^{\oplus}$ | |
| 2.281 | Cl | Cl | H | H | H | CN | $Na^{\oplus}$ | |
| 2.282 | Cl | Cl | H | H | H | CN | $Li^{\oplus}$ | |
| 2.283 | Cl | Cl | H | H | H | CN | 1/2 $Ca^{2\oplus}$ | |
| 2.284 | Cl | Cl | H | H | H | CN | $NH(CH_3)_3^{\oplus}$ | |
| 2.285 | Cl | Cl | H | H | H | CN | $NH(C_2H_5OH)_3^{\oplus}$ | |
| 2.286 | Cl | $CF_3$ | H | H | H | CN | $Na^{\oplus}$ | |
| 2.287 | Cl | $CF_3$ | H | H | H | CN | $Li^{\oplus}$ | |
| 2.288 | Cl | $CF_3$ | H | H | H | CN | 1/2 $Ca^{2\oplus}$ | |
| 2.289 | Cl | $CF_3$ | H | H | H | CN | $NH(CH_3)_3^{\oplus}$ | |
| 2.290 | Cl | $CF_3$ | H | H | H | CN | $NH(C_2H_5OH)_3^{\oplus}$ | |
| 2.291 | $NO_2$ | H | $CH_3$ | H | H | CN | $Na^{\oplus}$ | |
| 2.292 | $NO_2$ | H | $CH_3$ | H | H | CN | $Li^{\oplus}$ | |
| 2.293 | $NO_2$ | H | $CH_3$ | H | H | CN | 1/2 $Ca^{2\oplus}$ | |
| 2.294 | $NO_2$ | H | $CH_3$ | H | H | CN | $NH(CH_3)_3^{\oplus}$ | |
| 2.295 | $NO_2$ | H | $CH_3$ | H | H | CN | $NH(C_2H_5OH)_3^{\oplus}$ | |
| 2.296 | H | $NO_2$ | $CH_3$ | H | H | CN | $Na^{\oplus}$ | |
| 2.297 | H | $NO_2$ | $CH_3$ | H | H | CN | $Li^{\oplus}$ | |
| 2.298 | H | $NO_2$ | $CH_3$ | H | H | CN | 1/2 $Ca^{2\oplus}$ | |
| 2.299 | H | $NO_2$ | $CH_3$ | H | H | CN | $NH(CH_3)_3^{\oplus}$ | |
| 2.300 | H | $NO_2$ | $CH_3$ | H | H | CN | $NH(C_2H_5OH)_3^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.301 | H | H | $CH_3$ | H | H | CN | $Na^{\oplus}$ | |
| 2.302 | H | H | $CH_3$ | H | H | CN | $Li^{\oplus}$ | |
| 2.303 | H | H | $CH_3$ | H | H | CN | 1/2 $Ca^{2\oplus}$ | |
| 2.304 | H | H | $CH_3$ | H | H | CN | $NH(CH_3)_3^{\oplus}$ | |
| 2.305 | H | H | $CH_3$ | H | H | CN | $NH(C_2H_5OH)_3^{\oplus}$ | |
| 2.306 | Cl | $CCl_3$ | $CH_3$ | H | H | CN | $Na^{\oplus}$ | |
| 2.307 | Cl | $CCl_3$ | $CH_3$ | H | H | CN | $Li^{\oplus}$ | |
| 2.308 | Cl | $CCl_3$ | $CH_3$ | H | H | CN | 1/2 $Ca^{2\oplus}$ | |
| 2.309 | Cl | $CCl_3$ | $CH_3$ | H | H | CN | $NH(CH_3)_3^{\oplus}$ | |
| 2.310 | Cl | $CCl_3$ | $CH_3$ | H | H | CN | $NH(C_2H_5OH)_3^{\oplus}$ | |
| 2.311 | F | $CF_3$ | $CH_3$ | H | H | CN | $Na^{\oplus}$ | |
| 2.312 | F | $CF_3$ | $CH_3$ | H | H | CN | $Li^{\oplus}$ | |
| 2.313 | F | $CF_3$ | $CH_3$ | H | H | CN | 1/2 $Ca^{2\oplus}$ | |
| 2.314 | F | $CF_3$ | $CH_3$ | H | H | CN | $NH(CH_3)_3^{\oplus}$ | |
| 2.315 | F | $CF_3$ | $CH_3$ | H | H | CN | $NH(C_2H_5OH)_3^{\oplus}$ | |
| 2.316 | Cl | Cl | $C_6H_5-CH_2-$ | H | H | H | $Na^{\oplus}$ | |
| 2.317 | Cl | Cl | $C_6H_5-CH_2-$ | H | H | H | $Li^{\oplus}$ | |
| 2.318 | Cl | Cl | $C_6H_5-CH_2-$ | H | H | H | 1/2 $Ca^{2\oplus}$ | |
| 2.319 | Cl | Cl | $C_6H_5-CH_2-$ | H | H | H | $NH(CH_3)_3^{\oplus}$ | |
| 2.320 | Cl | Cl | $C_6H_5-CH_2-$ | H | H | H | $NH(C_2H_5OH)_3^{\oplus}$ | |
| 2.321 | Cl | $CF_3$ | $C_6H_5-CH_2-$ | H | H | H | $Na^{\oplus}$ | |
| 2.322 | Cl | $CF_3$ | $C_6H_5-CH_2-$ | H | H | H | $Li^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.323 | Cl | $CF_3$ | $C_6H_5-CH_2-$ | H | H | H | $1/2\ Ca^{2\oplus}$ | |
| 2.324 | Cl | $CF_3$ | $C_6H_5-CH_2-$ | H | H | H | $NH(CH_3)_3^{\oplus}$ | |
| 2.325 | Cl | $CF_3$ | $C_6H_5-CH_2-$ | H | H | H | $NH(C_2H_5OH)_3^{\oplus}$ | |
| 2.326 | $NO_2$ | H | $C_6H_5-CH_2-$ | H | H | H | $Na^{\oplus}$ | |
| 2.327 | $NO_2$ | H | $C_6H_5-CH_2-$ | H | H | H | $Li^{\oplus}$ | |
| 2.328 | $NO_2$ | H | $C_6H_5-CH_2-$ | H | H | H | $1/2\ Ca^{2\oplus}$ | |
| 2.329 | $NO_2$ | H | $C_6H_5-CH_2-$ | H | H | H | $NH(CH_3)_3^{\oplus}$ | |
| 2.330 | $NO_2$ | H | $C_6H_5-CH_2-$ | H | H | H | $NH(C_2H_5OH)_3^{\oplus}$ | |
| 2.331 | H | $NO_2$ | $C_6H_5-CH_2-$ | H | H | H | $Na^{\oplus}$ | |
| 2.332 | H | $NO_2$ | $C_6H_5-CH_2-$ | H | H | H | $Li^{\oplus}$ | |
| 2.333 | H | $NO_2$ | $C_6H_5-CH_2-$ | H | H | H | $1/2\ Ca^{2\oplus}$ | |
| 2.334 | H | $NO_2$ | $C_6H_5-CH_2-$ | H | H | H | $NH(CH_3)_3^{\oplus}$ | |
| 2.335 | H | $NO_2$ | $C_6H_5-CH_2-$ | H | H | H | $NH(C_2H_5OH)_3^{\oplus}$ | |
| 2.336 | H | H | $C_6H_5-CH_2-$ | H | H | H | $Na^{\oplus}$ | |
| 2.337 | H | H | $C_6H_5-CH_2-$ | H | H | H | $Li^{\oplus}$ | |
| 2.338 | H | H | $C_6H_5-CH_2-$ | H | H | H | $1/2\ Ca^{2\oplus}$ | |
| 2.339 | H | H | $C_6H_5-CH_2-$ | H | H | H | $NH(CH_3)_3^{\oplus}$ | |
| 2.340 | H | H | $C_6H_5-CH_2-$ | H | H | H | $NH(C_2H_5OH)_3^{\oplus}$ | |
| 2.341 | Cl | $CCl_3$ | $C_6H_5-CH_2-$ | H | H | H | $Na^{\oplus}$ | |
| 2.342 | Cl | $CCl_3$ | $C_6H_5-CH_2-$ | H | H | H | $Li^{\oplus}$ | |
| 2.343 | Cl | $CCl_3$ | $C_6H_5-CH_2-$ | H | H | H | $1/2\ Ca^{2\oplus}$ | |
| 2.344 | Cl | $CCl_3$ | $C_6H_5-CH_2-$ | H | H | H | $NH(CH_3)_3^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.345 | Cl | $CCl_3$ | $C_6H_5-CH_2-$ | H | H | H | $NH(C_2H_5OH)_3^{\oplus}$ | |
| 2.346 | F | $CF_3$ | $C_6H_5-CH_2-$ | H | H | H | $Na^{\oplus}$ | |
| 2.347 | F | $CF_3$ | $C_6H_5-CH_2-$ | H | H | H | $Li^{\oplus}$ | |
| 2.348 | F | $CF_3$ | $C_6H_5-CH_2-$ | H | H | H | 1/2 $Ca^{2\oplus}$ | |
| 2.349 | F | $CF_3$ | $C_6H_5-CH_2-$ | H | H | H | $NH(CH_3)_3^{\oplus}$ | |
| 2.350 | F | $CF_3$ | $C_6H_5-CH_2-$ | H | H | H | $NH(C_2H_5OH)_3^{\oplus}$ | |
| 2.351 | Cl | Cl | $C_6H_5$ | H | H | $COOCH_3$ | $Na^{\oplus}$ | |
| 2.352 | Cl | Cl | $C_6H_5$ | H | H | $COOCH_3$ | $Li^{\oplus}$ | |
| 2.353 | Cl | Cl | $C_6H_5$ | H | H | $COOCH_3$ | 1/2 $Ca^{2\oplus}$ | |
| 2.354 | Cl | Cl | $C_6H_5$ | H | H | $COOCH_3$ | $NH(CH_3)_3^{\oplus}$ | |
| 2.355 | Cl | Cl | $C_6H_5$ | H | H | $COOCH_3$ | $NH(C_2H_5OH)_3^{\oplus}$ | |
| 2.356 | Cl | $CF_3$ | $C_6H_5$ | H | H | $COOCH_3$ | $Na^{\oplus}$ | |
| 2.357 | Cl | $CF_3$ | $C_6H_5$ | H | H | $COOCH_3$ | $Li^{\oplus}$ | |
| 2.358 | Cl | $CF_3$ | $C_6H_5$ | H | H | $COOCH_3$ | 1/2 $Ca^{2\oplus}$ | |
| 2.359 | Cl | $CF_3$ | $C_6H_5$ | H | H | $COOCH_3$ | $NH(CH_3)_3^{\oplus}$ | |
| 2.360 | Cl | $CF_3$ | $C_6H_5$ | H | H | $COOCH_3$ | $NH(C_2H_5OH)_3^{\oplus}$ | |
| 2.361 | $NO_2$ | H | $C_6H_5$ | H | H | $COOCH_3$ | $Na^{\oplus}$ | |
| 2.362 | $NO_2$ | H | $C_6H_5$ | H | H | $COOCH_3$ | $Li^{\oplus}$ | |
| 2.363 | $NO_2$ | H | $C_6H_5$ | H | H | $COOCH_3$ | 1/2 $Ca^{2\oplus}$ | |
| 2.364 | $NO_2$ | H | $C_6H_5$ | H | H | $COOCH_3$ | $NH(CH_3)_3^{\oplus}$ | |
| 2.365 | $NO_2$ | H | $C_6H_5$ | H | H | $COOCH_3$ | $NH(C_2H_5OH)_3^{\oplus}$ | |

| Verb. Nr. | $R^1$ | $R^2$ | $R^3$ | $R^4$ | $R^5$ | $R^6$ | $M^{\oplus}$ | phys. Daten |
|---|---|---|---|---|---|---|---|---|
| 2.366 | H | $NO_2$ | $C_6H_5$ | H | H | $COOCH_3$ | $Na^{\oplus}$ | |
| 2.367 | H | $NO_2$ | $C_6H_5$ | H | H | $COOCH_3$ | $Li^{\oplus}$ | |
| 2.368 | H | $NO_2$ | $C_6H_5$ | H | H | $COOCH_3$ | 1/2 $Ca^{2\oplus}$ | |
| 2.369 | H | $NO_2$ | $C_6H_5$ | H | H | $COOCH_3$ | $NH(CH_3)_3^{\oplus}$ | |
| 2.370 | H | $NO_2$ | $C_6H_5$ | H | H | $COOCH_3$ | $NH(C_2H_5OH)_3^{\oplus}$ | |
| 2.371 | H | H | $C_6H_5$ | H | H | $COOCH_3$ | $Na^{\oplus}$ | |
| 2.372 | H | H | $C_6H_5$ | H | H | $COOCH_3$ | $Li^{\oplus}$ | |
| 2.373 | H | H | $C_6H_5$ | H | H | $COOCH_3$ | 1/2 $Ca^{2\oplus}$ | |
| 2.374 | H | H | $C_6H_5$ | H | H | $COOCH_3$ | $NH(CH_3)_3^{\oplus}$ | |
| 2.375 | H | H | $C_6H_5$ | H | H | $COOCH_3$ | $NH(C_2H_5OH)_3^{\oplus}$ | |
| 2.376 | Cl | $CCl_3$ | $C_6H_5$ | H | H | $COOCH_3$ | $Na^{\oplus}$ | |
| 2.377 | Cl | $CCl_3$ | $C_6H_5$ | H | H | $COOCH_3$ | $Li^{\oplus}$ | |
| 2.378 | Cl | $CCl_3$ | $C_6H_5$ | H | H | $COOCH_3$ | 1/2 $Ca^{2\oplus}$ | |
| 2.379 | Cl | $CCl_3$ | $C_6H_5$ | H | H | $COOCH_3$ | $NH(CH_3)_3^{\oplus}$ | |
| 2.380 | Cl | $CCl_3$ | $C_6H_5$ | H | H | $COOCH_3$ | $NH(C_2H_5OH)_3^{\oplus}$ | |
| 2.381 | F | $CF_3$ | $C_6H_5$ | H | H | $COOCH_3$ | $Na^{\oplus}$ | |
| 2.382 | F | $CF_3$ | $C_6H_5$ | H | H | $COOCH_3$ | $Li^{\oplus}$ | |
| 2.383 | F | $CF_3$ | $C_6H_5$ | H | H | $COOCH_3$ | 1/2 $Ca^{2\oplus}$ | |
| 2.384 | F | $CF_3$ | $C_6H_5$ | H | H | $COOCH_3$ | $NH(CH_3)_3^{\oplus}$ | |
| 2.385 | F | $CF_3$ | $C_6H_5$ | H | H | $COOCH_3$ | $NH(C_2H_5OH)_3^{\oplus}$ | |

H.2.1. Herstellung der Pyridincarbonsäurehalogenide der Formel II

H.2.1.1. Herstellung von 5-Trifluormethylpyridin-2-carbonsäurechlorid

Zu einer Suspension von 22,9 g (0,1 Mol) 5-Trifluormethylpyridin-2-carbonsäure-Kalium-Salz und 10 Tropfen DMF in 250 ml Toluol werden 9,8 ml (0,11 Mol) Oxalylchlorid getropft, wobei die Temperatur auf 40°C ansteigt und eine lebhafte Gasentwicklung einsetzt. Anschliessend wird 2 Stunden bei 40°C gerührt. Die hellbraune Suspension wird danach am Rotavapor eingedampft, mit 250 ml Aether abs. verrührt, filtriert und erneut eingedampft.

Man isoliert 17,8 g (85 %) der Titelverbindung der Formel

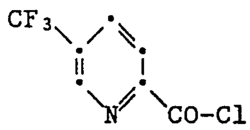

als braunes Oel (Verb. Nr. 3.078).

H.2.1.2. Herstellung von 3-Chlor-5-trifluormethyl-pyridin-2-carbonsäureethylester

216 g (1 Mol) 2,3-Dichlor-5-trifluormethylpyridin, 16,7 g [$PdCl_2$ $(PPh_3)_2$] und 420 ml (3 Mol) Triethylamin werden in 3,3 l Ethanol unter CO-Atmosphäre bei 50 bar und 100°C 14 Stunden gerührt. Anschliessend wird bei 40°C am Rotavapor eingedampft. Die so erhaltene Masse wird mit 2 l Aether verrührt, vom Triethylamin-Hydrochlorid abfiltriert und am Rotavapor eingedampft. Man erhält 245 g eines braunen Oels welches an Kieselgel mit Essigester/Hexan (1:9) gereinigt wird.

Man isoliert 206,8 g (81,6 %) der Titelverbindung der Formel

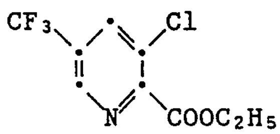

als gelbes Oel (Verb. Nr. 3.005).

H2.1.3. Herstellung von 3-Chlor-5-trifluormethylpyridin-2-carbonsäure

76 g (0,3 Mol) 3-Chlor-5-trifluormethylpyridin-2-carbonsäureethylester werden mit 165 ml (0,33 Mol) 2N NaOH 6 Stunden gerührt. Die entstandene Lösung wird 2 mal mit Methylenchlorid gewaschen. Die wässrige Lösung wird danach mit Salzsäure 37 % auf pH 1 gestellt und das Produkt abgenutscht und im Vakuum bei Raumtemperatur getrocknet.

Man isoliert 65 g (96 %) der Titelverbindung der Formel

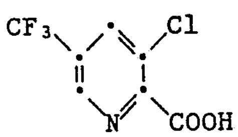

als weisse Kristalle vom Smp. 135°C (Zers.) (Verb. Nr. 3.039).

H.2.1.4. Herstellung von 3-Chlor-5-trifluormethylpyridin-2-carbonsäurechlorid

16,9 g (75 mMol) 3-Chlor-5-trifluormethylpyridin-2-carbonsäure werden in 75 ml Hexan suspendiert. Nach Zugabe von 2 Tropfen DMF werden 7 ml (80 mMol) Oxalylchlorid in 25 ml Hexan zugetropft. Anschliessend wird 4 Stunden bei 50°C gerührt, bis die Gasentwicklung beendet ist. Die Reaktionslösung wird filtriert und am Rotavapor eingedampft.

Man isoliert 18 g (98 %) der Titelverbindung der Formel

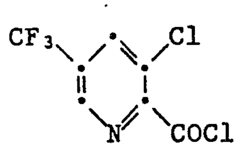

als gelbes Oel (Verb. Nr. 3.073).

Analog zu den vorstehenden Herstellungsverfahren können die Verbindungen der Tabelle 3 synthetisiert werden.

H.2.1.5. Herstellung von 3,5-Dichlor-pyridin-2-carbonsäure-ethylester

250 g (1,3 Mol) 2,3,5-Trichlorpyridin 95 % werden nach der Vorschrift H.2.1.2. umgesetzt und gereinigt.

Man isoliert 147 g (51,5 %) der Titelverbindung als gelbes Oel der Formel

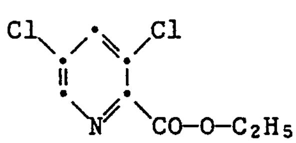

als gelbes Oel (Verb. Nr. 3.139).

H.2.1.6. Herstellung von 3-Chlor-5-methylthio-2-carbonsäure-ethylester

In eine Suspension von 17.9 g (0,16 Mol) Kalium-tert.-butylat und 4,8 g Polyäthylenglykol 1500 in 480 ml Toluol werden bei 25-30°C 8,6 g (0,18 Mol) Methylmercaptan eingeleitet. Anschliessend wird 15 Minuten bei 35°C gerührt. Die entstandene weisse Suspension wird auf -30°C abgekühlt und mit 35,2 g (0,16 Mol) 3,5-Dichlor-pyridin-2-carbonsäure-ethylester versetzt, auf 20-25°C aufwärmen lassen und bei dieser Temperatur 15 Stunden gerührt. Das Reaktionsbemisch wird vom Kaliumchlorid abfiltriert und am Rotavapor eingedampft. Man erhält 41 g eines gelben Oels, welches an Kieselgel mit Petrolether/Ether (3:1) gereinigt wird.

Man isoliert 16 g (43,3 %) der Titelverbindung der Formel

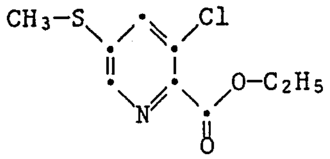

als gelbes Oel (Verb. Nr. 3.018).

H.2.1.7. Herstellung von 3-Chlor-5-methylthiopyridin-2-carbonsäure

22 g (0,095 Mol) 3-Chlor-5-methylthiopyridin-2-carbonsäureethylester werden analog H.2.1.3. umgesetzt und gereinigt.

Man isoliert 18,3 g (94,7 %) der Titelverbindung der Formel

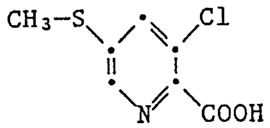

als weisse Kristalle (Verb. Nr. 3.052).

H.2.1.8. Herstellung von 3-Chlor-5-methylthiopyridin-2-carbonsäurechlorid

9,8 g (0,048 Mol) 3-Chlor-5-methylthiopyridin-2-carbonsäure werden in 65 ml Hexan und 25 ml Dichlorethan suspensiert. Nach Zugabe von 2 Tropfen DMF und Aufwärmen auf 55°C werden 4,9 ml (0,055 Mol) Oxalylchlorid zugetropft. Anschliessend wird 4 Stunden bei 55°C gerührt, bis die Gasentwicklung beendet ist. Die Reaktionslösung wird filtriert und am Rotavapor eingedampft.

Man isoliert 10,6 g (99,5 %) der Titelverbindung der Formel

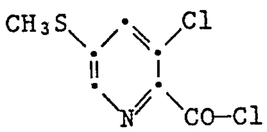

als gelbe, wachsartige Kristalle (Verb. Nr. 3.086).

H.2.2. 3-Chlor-5-methylsulfonyl-pyridin-2-carbonsäure-ethylester

Zu einer Lösung von 16,2 g (70 mMol) 3-Chlor-5-methylthiopyridin-2-carbonsäure-ethylester in 50 ml Dichlormethan werden 44 g (140 mMol) 3-Chlorperbenzoesäure 55 % in 150 ml Dichlormethan so zugetropft, dass die Temperatur 30°C nicht übersteigt. Nach 15 Stunden Rühren bei Raumtemperatur werden zur Vervollständigung der Reaktion nochmals 19 g (70 mMol) 3-Chlorperbenzoesäure 55 % in 75 ml Dichlormethan zur entstandenen Suspension getropft. Anschliessend wird 3 Stunden bei Raumtemperatur ausgerührt. Die weisse Suspension wird mit ca. 200 ml Dichlormethan verdünnt und von der ausgefallenen 3-Chlorbenzoesäure abfilt riert. Die entstandene Lösung wird mit $NaHCO_3$-Lösung 5 % und mit Wasser gewaschen. Die Lösung wird am Rotavapor eingedampft.

Man isoliert 15,3 g (83 %) der Titelverbindung der Formel:

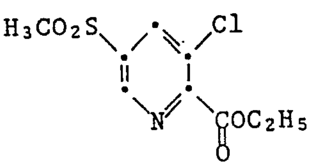

als Kristalle vom Smp. 90-93°C (Verb. Nr. 3.017).

H.2.2.2. 3-Chlor-5-methylsulfonyl-pyridin-2-carbonsäure

Zu einem Gemisch von 55 ml (55 mMol) NaOH 1N und 5,5 ml Ethanol werden 13,2 g (50 mMol) 3-Chlor-5-methylsulfonyl-pyridin-2-carbonsäure-ethylester gegeben, wobei die Temperatur auf 32° C ansteigt. Anschliessend wird 3 Stunden bei Raumtemperatur ausgerührt. Die Lösung wird mit 150 ml $H_2O$ verdünnt, 2x mit Dichlormethan gewaschen und dann bei 0-5° C mit HCl 37 % stark sauer gestellt. Das ausgefallene Produkt wird abfiltriert, mit wenig Eiswasser gespü lt und getrocknet.

Man isoliert 10,4 g (88,3 %) der Titelverbindung der Formel

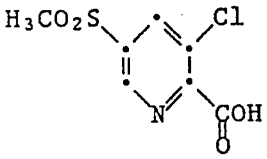

als Kristalle vom Smp. >150° C (Zers.) (Verb. Nr. 3.051).

H.2.2.3. 3-Chlor-5-methylsulfonyl-pyridin-2-carbonsäurechlorid

9,9 g (42 mMol) 3-Chlor-5-methylsulfonyl-pyridin-2-carbonsäure werden in 100 ml Toluol mit 4,4 ml (60 mMol) Thionylchlorid 2 Stunden am Rückfluss gekocht. Die braune Suspension wird am Rotavapor eingedampft.

Man isoliert 10,4 g (97,5 %) der Titelverbindung der Formel

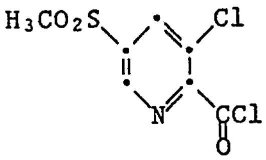

als braunes Wachs (Verb. Nr. 3.086).

Tabelle 3

Verbindungen der Formel

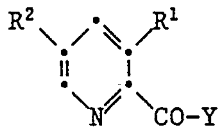

| Verb. Nr. | $R^1$ | $R^2$ | Y | phys. Daten |
|---|---|---|---|---|
| 3.001 | Cl | Cl | $OC_2H_5$ | Oel |
| 3.002 | Cl | H | $OC_2H_5$ | Oel |
| 3.003 | H | Cl | $OC_2H_5$ | Fp. 56-58°C |
| 3.004 | H | H | $OC_2H_5$ | |
| 3.005 | Cl | $CF_3$ | $OC_2H_5$ | Oel |
| 3.006 | $NO_2$ | H | $OC_2H_5$ | |
| 3.007 | H | $NO_2$ | $OC_2H_5$ | Fp. 89-91°C |
| 3.008 | $NO_2$ | Cl | $OC_2H_5$ | |
| 3.009 | $CF_3$ | H | $OC_2H_5$ | |
| 3.010 | H | $CF_3$ | $OC_2H_5$ | Fp. 43-45°C |
| 3.011 | $OCH_3$ | H | $OC_2H_5$ | |
| 3.012 | CN | H | $OC_2H_5$ | |
| 3.013 | $OCH_3$ | Cl | $OC_2H_5$ | |
| 3.014 | CN | Cl | $OC_2H_5$ | |
| 3.015 | Br | Cl | $OC_2H_5$ | |
| 3.016 | $SCH_3$ | Cl | $OC_2H_5$ | Fp. 77-80°C |
| 3.017 | $SO_2CH_3$ | Cl | $OC_2H_5$ | Fp. 90-93°C |
| 3.018 | Cl | $SCH_3$ | $OC_2H_5$ | Oel |
| 3.019 | Cl | $SOCH_3$ | $OC_2H_5$ | |
| 3.020 | Cl | $SO_2CH_3$ | $OC_2H_5$ | |
| 3.021 | $SOCH_3$ | Cl | $OC_2H_5$ | |
| 3.022 | $SO_2CH_3$ | H | $OC_2H_5$ | |
| 3.023 | H | $SO_2CH_3$ | $OC_2H_5$ | |
| 3.024 | H | $CH_3$ | $OC_2H_5$ | Oel |
| 3.025 | Cl | F | $OC_2H_5$ | Oel |
| 3.026 | H | $CF_3$ | $OC_2H_5$ | |
| 3.027 | F | F | $OC_2H_5$ | |
| 3.028 | F | $CF_3$ | $OC_2H_5$ | |
| 3.029 | $CF_3$ | F | $OC_2H_5$ | |
| 3.030 | H | $CCl_3$ | $OC_2H_5$ | |
| 3.031 | $CCl_3$ | H | $OC_2H_5$ | |
| 3.032 | Cl | $CCl_3$ | $OC_2H_5$ | |
| 3.033 | $CCl_3$ | Cl | $OC_2H_5$ | |
| 3.034 | $CH_3$ | H | $OC_2H_5$ | |
| 3.035 | Cl | Cl | OH | Fp. 157°C (Zers.) |
| 3.036 | Cl | H | OH | Fp. >125°C (Zers.) |
| 3.037 | H | Cl | OH | Fp. 135°C (Zers.) |
| 3.038 | H | H | OH | |
| 3.039 | Cl | $CF_3$ | OH | Fp. 135°C (Zers.) |
| 3.040 | $NO_2$ | H | OH | |
| 3.041 | H | $NO_2$ | OH | |
| 3.042 | $NO_2$ | Cl | OH | |
| 3.043 | $CF_3$ | H | OH | Fp. 129-131°C |
| 3.044 | H | $CF_3$ | OH | |

| Verb. Nr. | $R^1$ | $R^2$ | Y | phys. Daten |
|---|---|---|---|---|
| 3.045 | $OCH_3$ | H | OH | |
| 3.046 | CN | H | OH | |
| 3.047 | $OCH_3$ | Cl | OH | |
| 3.048 | CN | Cl | OH | |
| 3.049 | Br | Cl | OH | |
| 3.050 | $SCH_3$ | Cl | OH | |
| 3.051 | $SO_2CH_3$ | Cl | OH | Fp. >150°C (Zers.) |
| 3.052 | Cl | $SCH_3$ | OH | fest |
| 3.053 | Cl | $SOCH_3$ | OH | |
| 3.054 | Cl | $SO_2CH_3$ | OH | |
| 3.055 | $SOCH_3$ | Cl | OH | |
| 3.056 | $SO_2CH_3$ | H | OH | |
| 3.057 | H | $SO_2CH_3$ | OH | |
| 3.058 | H | $CH_3$ | OH | Fp. >135°C (Zers.) |
| 3.059 | Cl | F | OH | Fp. >143°C (Zers.) |
| 3.060 | H | $CF_3$ | OH | |
| 3.061 | F | F | OH | |
| 3.062 | F | $CF_3$ | OH | |
| 3.063 | $CF_3$ | F | OH | |
| 3.064 | H | $CCl_3$ | OH | |
| 3.065 | $CCl_3$ | H | OH | |
| 3.066 | Cl | $CCl_3$ | OH | |
| 3.067 | $CCl_3$ | Cl | OH | |
| 3.068 | $CH_3$ | H | OH | |
| 3.069 | Cl | Cl | Cl | Fp. 54-56°C |
| 3.070 | Cl | H | Cl | fest |
| 3.071 | H | Cl | Cl | fest |
| 3.072 | H | H | Cl | |
| 3.073 | Cl | $CF_3$ | Cl | Oel |
| 3.074 | $NO_2$ | H | Cl | |
| 3.075 | H | $NO_2$ | Cl | |
| 3.076 | $NO_2$ | Cl | Cl | |
| 3.077 | $CF_3$ | H | Cl | Oel |
| 3.078 | H | $CF_3$ | Cl | Oel |
| 3.079 | $OCH_3$ | H | Cl | |
| 3.080 | CN | H | Cl | |
| 3.081 | $OCH_3$ | Cl | Cl | |
| 3.082 | CN | Cl | Cl | |
| 3.083 | Br | Cl | Cl | |
| 3.084 | $SCH_3$ | Cl | Cl | |
| 3.085 | $SO_2CH_3$ | Cl | Cl | |
| 3.086 | Cl | $SCH_3$ | Cl | fest |
| 3.087 | Cl | $SOCH_3$ | Cl | |
| 3.088 | Cl | $SO_2CH_3$ | Cl | |
| 3.089 | $SOCH_3$ | Cl | Cl | |
| 3.090 | $SO_2CH_3$ | H | Cl | |
| 3.091 | H | $SO_2CH_3$ | Cl | |
| 3.092 | H | $CH_3$ | Cl | Oel |
| 3.093 | Cl | F | Cl | Oel |
| 3.094 | H | $CF_3$ | Cl | |

| Verb. Nr. | $R^1$ | $R^2$ | Y | phys. Daten |
|---|---|---|---|---|
| 3.095 | F | F | Cl | |
| 3.096 | F | $CF_3$ | Cl | |
| 3.097 | $CF_3$ | F | Cl | |
| 3.098 | H | $CCl_3$ | Cl | |
| 3.099 | $CCl_3$ | H | Cl | |
| 3.100 | Cl | $CCl_3$ | Cl | |
| 3.101 | $CCl_3$ | Cl | Cl | |
| 3.102 | $CH_3$ | H | Cl | |
| 3.103 | Cl | $OCH_3$ | $OC_2H_5$ | |
| 3.104 | Cl | $OCH_3$ | OH | fest |
| 3.105 | Cl | $OCH_3$ | Cl | fest |
| 3.106 | H | $OCH_3$ | $OC_2H_5$ | |
| 3.107 | H | $OCH_3$ | OH | |
| 3.108 | H | $OCH_3$ | Cl | |
| 3.109 | H | $OC_3H_7(i)$ | $OC_2H_5$ | |
| 3.110 | H | $OC_3H_7(i)$ | OH | |
| 3.111 | H | $OC_3H_7(i)$ | Cl | |
| 3.112 | H | $OCH_3$ | $OCH_3$ | Fp. 27-74°C |
| 3.113 | H | $SCH_3$ | $OC_2H_5$ | Fp. 46-48°C |
| 3.114 | H | $SCH_3$ | OH | Fp. 159-160°C |
| 3.115 | H | $SCH_3$ | Cl | fest |
| 3.116 | Cl | $SC_2H_5$ | OH | |
| 3.117 | Cl | $SC_2H_5$ | $OC_2H_5$ | |
| 3.118 | Cl | $SC_2H_5$ | Cl | |
| 3.119 | Cl | $SOC_2H_5$ | OH | |
| 3.120 | Cl | $SOC_2H_5$ | $OC_2H_5$ | |
| 3.121 | Cl | $SOC_2H_5$ | Cl | |
| 3.122 | Cl | $SO_2C_3H_7(i)$ | OH | |
| 3.123 | Cl | $SOC_3H_7(i)$ | OH | |
| 3.124 | Cl | $SC_3H_7(i)$ | OH | |
| 3.125 | Cl | $SO_2C_3H_7(i)$ | $OC_2H_5$ | |
| 3.126 | Cl | $SOC_3H_7(i)$ | $OC_2H_5$ | |
| 3.127 | Cl | $SC_3H_7(i)$ | $OC_2H_5$ | |
| 3.128 | Cl | $SO_2C_3H_7(i)$ | Cl | |
| 3.129 | Cl | $SOC_3H_7(i)$ | Cl | |
| 3.130 | Cl | $SC_3H_7(i)$ | Cl | |
| 3.131 | Cl | $OC_3H_7(i)$ | OH | |
| 3.132 | Cl | $OC_3H_7(i)$ | $OC_2H_5$ | |
| 3.133 | Cl | $OC_3H_7(i)$ | Cl | |
| 3.134 | $SCH_3$ | $SCH_3$ | $OC_2H_5$ | Fp. 60-70°C |
| 3.135 | Cl | Br | OH | |
| 3.136 | Cl | Br | $OC_2H_5$ | |
| 3.137 | Cl | Br | Cl | |
| 3.138 | Cl | $OCH_3$ | $OCH_3$ | Fp. 72-74°C |
| 3.139 | Cl | Cl | $COOC_2H_5$ | Oel |

F. Formulierungsbeispiele

Beispiel F 1.: Formulierungsbeispiele für Wirkstoffe der Formel I
(% = Gewichtsprozent)

| a) Emulsionskonzentrate | a) | b) | c) |
|---|---|---|---|
| Wirkstoff gemäss Tabelle 1 oder 2 | 20 % | 40 % | 50 % |
| Ca-Dodecylbenzolsulfonat | 5 % | 8 % | 5,8 % |
| Ricinusöl-polyethylenglykolether (36 Mol EO) | 5 % | - | - |
| Tributylphenoyl-polyethylenglykolether (30 Mol EO) | - | 12 % | 4,2 % |
| Cyclohexanon | - | 15 % | 20 % |
| Xylolgemisch | 70 % | 25 % | 20 % |

Aus solchen Konzentraten können durch Verdünnen mit Wasser Emulsionen jeder gewünschten Konzentration hergestellt werden.

| b) Lösungen | a) | b) | c) |
|---|---|---|---|
| Wirkstoff gemäss Tabelle 1 oder 2 | 80 % | 10 % | 5 % |
| Ethylenglykol-monomethylether | 20 % | - | - |
| Polyethylenglykol MG 400 | - | 70 % | - |
| N-Methyl-2-pyrrolidon | - | 20 % | 5 % |
| Epoxidiertes Kokosnussöl | - | - | 90 % |

Die Lösungen sind zur Anwendung in Form kleinster Tropfen geeignet.

| c) Granulate | a) | b) |
|---|---|---|
| Wirkstoff gemäss Tabelle 1 oder 2 | 5 % | 10 % |
| Kaolin | 94 % | - |
| Hochdisperse Kieselsäure | 1 % | |
| Attapulgit | - | 90 % |

Der Wirkstoff wird gelöst, auf den Träger aufgesprüht und das Lösungsmittel anschliessend im Vakuum abgedampft.

| d) Stäubemittel | a) | b) | c) |
|---|---|---|---|
| Wirkstoff gemäss Tabelle 1 oder 2 | 2 % | 5 % | 8 % |
| Hochdisperse Kieselsäure | 1 % | 5 % | 5 % |
| Talkum | 97 % | - | 10 % |
| Kaolin | - | 90 % | 77 % |

Durch inniges Vermischen der Trägerstoffe mit dem Wirkstoff erhält man gebrauchsfertiges Stäubemittel.

e) Spritzpulver

| | a) | b) |
|---|---|---|
| Wirkstoff gemäss Tabelle 1 oder 2 | 20 % | 60 % |
| Na-Ligninsulfonat | 5 % | 5 % |
| Na-Laurylsulfat | 3 % | 6 % |
| Octylphenolpolyethylen-glykolether (7-8 Mol EO) | - | 2 % |
| Hochdisperse Kieselsäure | 5 % | 27 % |
| Kaolin | 67 % | - |

Der Wirkstoff wird mit den Zusatzstoffen gut vermischt und in einer geeigneten Mühle gut vermahlen. Man erhält Spritzpulver, die sich mit Wasser zur Suspension jeder gewünschten Konzentration verdünnen lassen.

f) Extruder Granulat

| | |
|---|---|
| Wirkstoff gemäss Tabelle 1 oder 2 | 10 % |
| Na-Ligninsulfonat | 2 % |
| Carboxymethylcellulose | 1 % |
| Kaolin | 87 % |

Der Wirkstoff wird mit den Zusatzstoffen vermischt, vermahlen und mit Wasser angefeuchtet. Dieses Gemisch wird extrudiert und anschliessend im Luftstrom getrocknet.

g) Umhüllungs-Granulat

| | |
|---|---|
| Wirkstoff gemäss Tabelle 1 oder 2 | 3 % |
| Polyethylenglykol (MG 200) | 3 % |
| Kaolin | 94 % |

Der fein gemahlene Wirkstoff wird in einem Mischer auf das mit Polyäthylenglykol angefeuchtete Kaolin gleichmässig aufgetragen. Auf diese Weise erhält man staubfreie Umhüllungs-Granulate.

h) Suspensions-Konzentrat

| | |
|---|---|
| Wirkstoff gemäss Tabelle 1 oder 2 | 40 % |
| Ethylenglykol | 10 % |
| Nonylphenolpolyethylenglykolether (15 Mol EO) | 6 % |
| Na-Ligninsulfonat | 10 % |
| Carboxymethylcellulose | 1 % |
| 37 %ige wässrige Formaldehyd-Lösung | 0,2 % |
| Silikonöl in Form einer 75 %igen wässrigen Emulsion | 0,8 % |
| Wasser | 32 % |

Der fein gemahlene Wirkstoff wird mit den Zusatzstoffen innig vermischt. Man erhält so ein Suspensions-Konzentrat, aus welchem durch Verdünnen mit Wasser Suspensionen jeder gewünschten Konzentration hergestellt werden können.

B. Biologische Beispiele

Beispiel B 1: Pre-emergente Herbizid-Wirkung

Im Gewächshaus wird unmittelbar nach der Einsaat der Versuchspflanzen in Saatschalen die Erdoberfläche mit einer wässrigen Dispersion der Wirkstoffe, erhalten aus einem 25%igen Emulsionskonzentrat behandelt. Es werden unterschiedliche Aufwandmengen Wirksubstanz/Hektar getestet. Die Saatschalen werden im Gewächshaus bei 22-25° C und 50-70 % relativer Luftfeuchtigkeit gehalten und der Versuch nach 3 Wochen ausgewertet.

Die Herbizidwirkung wird dabei in einem neunstufigen (1 = vollständige Schädigung der Versuchpflanze, 9 = keine Herbizidwirkung an der Versuchspflanze) Boniturschema im Vergleich zur unbehandelten Kontrollgruppe ausgewertet.

Boniturnoten von 1 bis 4 (insbesondere von 1 bis 3) weisen auf eine gute bis sehr gute Herbizidwirkung hin. Boniturnoten von 6 bis 9 (insbesondere von 7 bis 9) weisen auf eine gute Toleranz (insbesondere bei

Kulturpflanzen) hin. Die Testresultate für Verbindung Nr. 1.005 sind in Tabelle 4 zusammengefasst.

Tabelle 4

| Testpflanze | Aufwandmenge [g/ha] | | | | | |
|---|---|---|---|---|---|---|
| | 2000 | 1000 | 5000 | 250 | 125 | 60 |
| Gerste | 7 | 7 | 9 | 9 | 9 | 9 |
| Weizen | 8 | 9 | 9 | 9 | 9 | 9 |
| Mais | 8 | 9 | 9 | 9 | 9 | 9 |
| Sorghum | 7 | 8 | 9 | 9 | 9. | 9 |
| Abutilon | 1 | 1 | 2 | 2 | 2 | 5 |
| Chenopodium Sp. | 1 | 1 | 1 | 1 | 1 | 1 |
| Solanum nigrum | 1 | 1 | 1 | 1 | 2 | 3 |
| Veronica Sp. | 1 | 1 | 1 | 1 | 4 | 6 |

Beispiel B 2: Post-emergente Herbizid-Wirkung

Eine Anzahl Unkräuter, sowohl monokotyle wie dikotyle, werden nach dem Auflaufen (im 4- bis 6-Blattstadium) mit einer wässrigen Wirkstoffdispersion in einer Dosierung von 250 g bis 2 kg Wirksubstanz pro Hektar auf die Pflanzen gespritzt und diese bei 24°-26°C und 45-60 % relativer Luftfeuchtigkeit gehalten. 15 Tage nach der Behandlung wird der Versuch nach dem in Beispiel B1 beschriebenen Boniturschema ausgewertet.

Die Testresultate für Verbindung 1.005 sind in Tabelle 5 zusammengestellt.

Tabelle 5

| Testpflanze | Aufwandmenge [g/ha] | | | |
|---|---|---|---|---|
| | 2000 | 1000 | 5000 | 250 |
| Gerste | 9 | 9 | 9 | 9 |
| Weizen | 8 | 9 | 9 | 9 |
| Mais | 8 | 9 | 9 | 9 |
| Sorghum | 7 | 8 | 9 | 9 |
| Reis (trocken) | 7 | 8 | 9 | 9 |
| Abutilon | 1 | 1 | 2 | 3 |
| Chenopodium Sp. | 1 | 1 | 1 | 2 |
| Solanum nigrum | 1 | 1 | 1 | 2 |
| Sinapis | 2 | 2 | 3 | 3 |

Beispiel B 3: Herbizidwirkung für Wasserreis (paddy)

Die Wasserunkräuter Echinochloa crus galli und Monocharia vag. werden in Plastikbechern (60 $cm^2$ Oberfläche, 500 ml Volumen) ausgesät. Nach der Saat wird bis zur Erdoberfläche mit Wasser aufgefüllt. 3 Tage nach der Saat wird der Wasserspiegel bis leicht über die Erdoberfläche erhöht (3-5 mm). Die Applikation erfolgt 3 Tage nach der Saat mit einer wässrigen Emulsion der Prüfsubstanzen durch eine Spritzung auf die Gefässe mit einer Aufwandmenge von 60 bis 250 g AS pro Hektar. Die Pflanzenbecher werden dann im Gewächshaus unter optimalen Wachstumsbedingungen für die Reisunkräuter aufgestellt, d.h. bei 25°-30°C und hoher Luftfeuchtigkeit. Die Auswertung der Versuche findet 3 Wochen nach Applikation statt. Die Verbindungen gemäss Tabelle 1 schädigen dabei die Unkräuter, nicht aber den Reis.

**Patentansprüche**

1. Cyclohexan-1,3-dione der Formel I oder I′

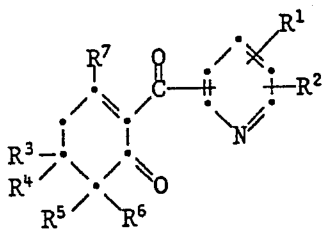

oder

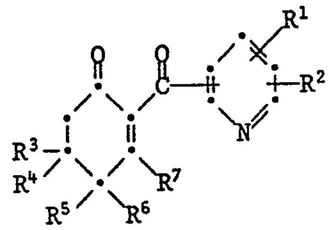

worin
$R^1$ und $R^2$ unabhängig voneinander Wasserstoff; Halogen; Nitro; Cyano; $C_1$-$C_4$-Alkyl; $C_1$-$C_4$-Alkoxy; $C_1$-$C_4$-Alkyl-$S(O)_n$-; $COR^8$; $C_1$-$C_4$-Halogenalkoxy; oder $C_1$-$C_4$-Halogenalkyl;
$R^3$, $R^4$ und $R^5$ unabhängig voneinander Wasserstoff; $C_1$-$C_4$-Alkyl; oder gegebenenfalls bis zu dreifach gleich oder verschieden durch Halogen, Nitro, Cyano, $C_1$-$C_4$-Alkyl, $C_1$-$C_4$-Alkoxy, $C_1$-$C_4$-Alkyl-$S(O)_n$-, $C_1$-$C_4$-Halogenalkyl, $C_1$-$C_4$-Halogenalkyl-$S(O)_n$- oder $C_1$-$C_4$-Halogenalkoxy substituiertes Phenyl oder Benzyl;
$R^6$ Wasserstoff; $C_1$-$C_4$-Alkyl, $C_1$-$C_4$-Alkoxycarbonyl; oder Cyano;
$R^7$ OH; oder $O^{\ominus}M^{\oplus}$;
$R^8$ OH; $C_1$-$C_4$-Alkoxy; $NH_2$; $C_1$-$C_4$-Alkylamino; oder di-$C_1$-$C_4$-Alkylamino;
n 0, 1 oder 2;
$M^{\oplus}$ ein Kationenäquivalent eines Metallions oder eines gegebenenfalls bis zu dreifach durch $C_1$-$C_4$-Alkyl, $C_1$-$C_4$-Hydroxyalkyl-, oder $C_1$-$C_4$-Alkoxy-$C_1$-$C_4$-alkyl-gruppen substituierten Ammoniumions
bedeutet.

2. Cyclohexandion gemäss Anspruch 1 der Formel I oder I′

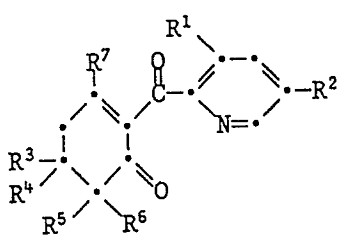

I

oder

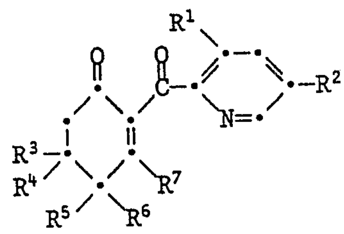

I'

worin die Reste $R^1$ und $R^2$ in den Positionen 3 und 5 des Pyridinringes und das Pyridincarbonylsystem über die Position 2 des Pyridinringes gebunden sind.

3. Cyclohexandione der Formel I oder I′ worin
$R^1$ Wasserstoff; Fluor; Chlor; Brom; Nitro; Cyano; Methyl; Trifluormethyl; Trichlormethyl; Methoxy; Methylthio; Methylsulfinyl Methylsulfonyl Carboxy; Carbamoyl; Methoxycarbonyl; oder Ethoxycarbonyl;
$R^2$ Wasserstoff; Fluor; Chlor; Nitro; Trifluormethyl; Trichlormethyl; Methylthio; Methylsulfinyl; oder Methylsulfonyl;
$R^3$ Wasserstoff; $C_1$-$C_3$-Alkyl, Phenyl; Benzyl; oder Chlorphenyl;
$R^4$ Wasserstoff; oder Methyl;
$R^5$ Wasserstoff; oder Methyl;
$R^6$ Wasserstoff; Cyano; Methyl; oder $C_1$-$C_2$-Alkoxycarbonyl;
$R^7$ OH; oder $O^{\ominus}M^{\oplus}$
$M^{\oplus}$ ein Kationenäquivalent des Natrium-, Lithium-, Calcium-, Trimethylammonium- oder Triethanolammoniumions
bedeuten, gemäss Anspruch 1 oder 2.

4. 2-(3-Chlor-5-trifluormethyl-pyridin-2-yl-carbonyl)-cyclohex-1-en-1-ol-3-on oder 2-(3-Chlor-5-methylsulfonyl-pyridin-2-yl-carbonyl)-cyclohex-1-en-1-ol-3-on als Verbindung der Formel I gemäss Anspruch 1.

5. Verfahren zur Herstellung von Cyclohexandionenen der Formel I oder I′, worin die Reste $R^1$ bis $R^6$ wie in einem der Ansprüche 1 bis 3 definiert sind und $R^7$ OH bedeutet, gekennzeichnet durch

a) die Umsetzung von Cyclohexandionen der Formel II, worin die Reste $R^3$ bis $R^6$ wie zuvor definiert sind, mit einem Pyridin der Formel III, worin $R^1$ und $R^2$ wie zuvor definiert sind und X Halogen, vorzugsweise Chlor oder Brom, den Rest

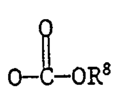

, oder

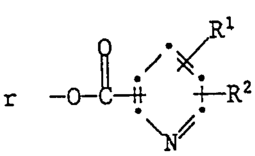

und $R^8$ $C_1$-$C_4$-Alkyl, Phenyl oder Benzyl bedeutet, in Gegenwart einer Base

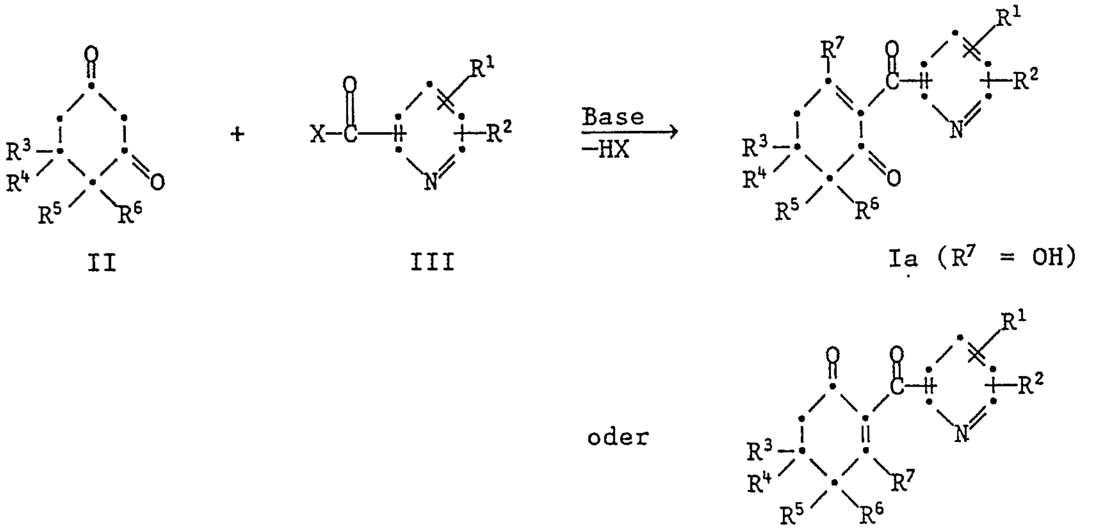


II III Ia ($R^7$ = OH)

oder

Ia' ($R^7$ = OH)

oder

b) die thermische Umlagerung eines Esters der Formel IV oder IV', worin die Reste $R^1$ bis $R^6$ wie zuvor definiert sind

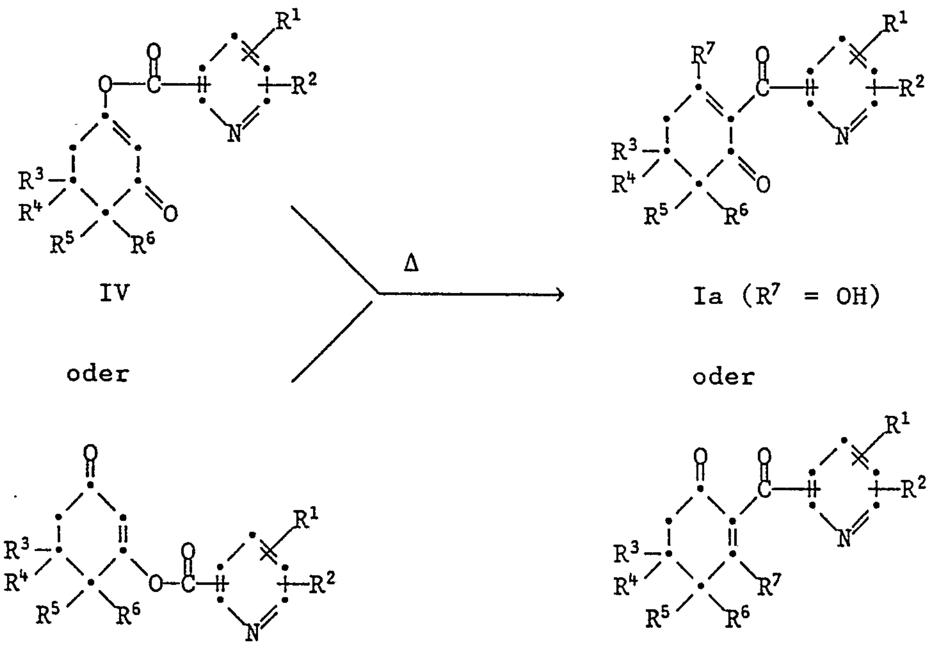


IV Ia ($R^7$ = OH)

oder oder

IV' Ia' ($R^7$ = OH)

6. Verfahren zur Herstellung von Salzen der Cyclohexandione der Formel I oder I', worin die Reste $R^1$ bis $R^6$ und M wie in einem der Ansprüche 1 bis 3 definiert sind und $R^7$ $O^{\ominus}M^{\oplus}$ bedeutet, gekennzeichnet durch die Umsetzung eines Cyclohexandions Ia oder Ia', worin $R^1$ bis $R^6$ wie zuvor definiert sind, und $R^7$ OH bedeutet mit einer Base V, worin B $OH^{\ominus}M^{\oplus}$ und $M^{\oplus}$ wie zuvor definiert ist

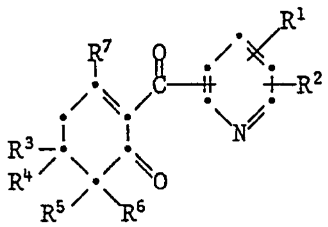

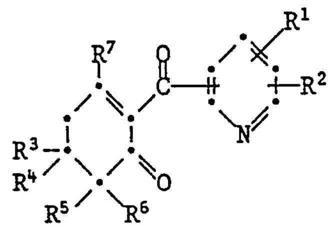

Ia ($R^7$ = OH) + B → Ib ($R^7$ = $O^{\ominus}M^{\oplus}$)

V

oder

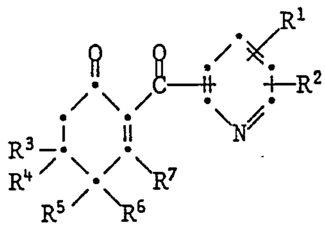

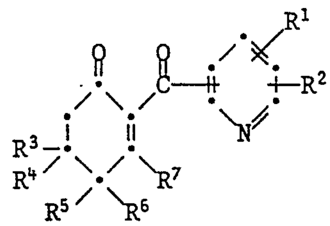

Ia' ($R^7$ = OH) Ib' ($R^7$ = $O^{\ominus}M^{\oplus}$)

7. Verfahren zur Herstellung von Verbindungen der Formel Ic oder Ic′ gemäss Anspruch 1, worin einer oder mehrere der Reste $R^1$ bis $R^6$ für $C_1$-$C_4$-Alkyl-S(O)$_n$- mit n = 1 oder 2 steht, und die übrigen Reste wie zuvor definiert sind, gekennzeichnet durch die Oxidation eines Thioethers der Formel Id oder Id′, worin der zu oxidierende Rest aus der Gruppe $R^1$ bis $R^6$ $C_1$-$C_4$-Alkyl-S(O)$_n$- mit n = 0 bedeutet und die übrigen Reste wie zuvor definiert sind

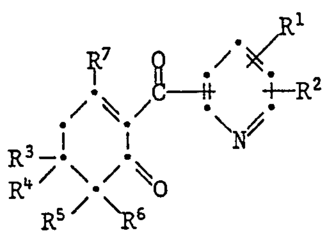

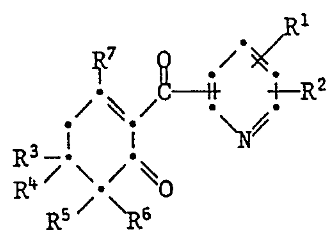

Id (Thioether mit n = 0) Oxidation Ic (Sulfinyl- oder Sulfonylverbindung mit n = 1 oder 2)

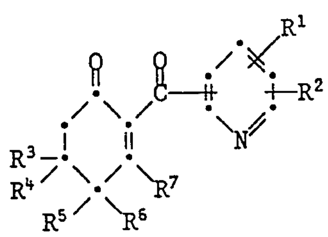

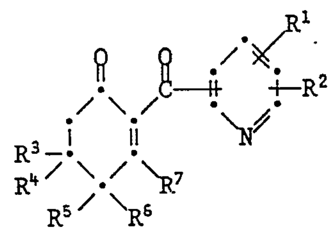

Id' (Thioether mit n = 0) Ic' (Sulfinyl- oder Sulfonylverbindung mit n = 1 oder 2)

8. Verfahren zur Herstellung von Pyridin-2-carbonsäureestern der Formel XI, worin $R^1$ und $R^2$ wie in einem der Ansprüche 1 bis 4 definiert ist und R′ $C_1$-$C_4$-Alkyl bedeutet, gekennzeichnet durch die Umsetzung eines Halogenpyridins der Formel X, worin Hal Halogen bedeutet und $R^1$ und $R^2$ wie zuvor definiert sind mit Kohlenmonoxid und einem Alkohol R′OH in Gegenwart eines Pd-Katalysators

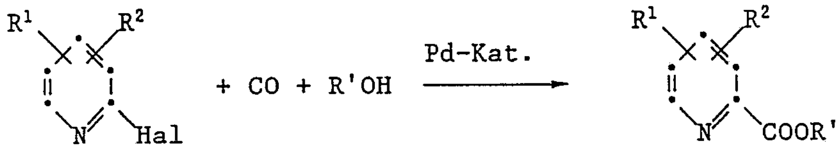


X XI

9. Verbindungen der Formel IV oder IV',

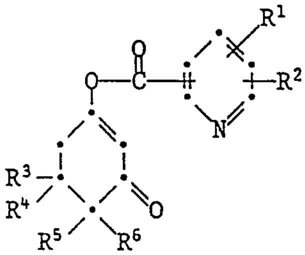

oder

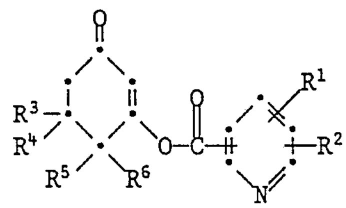

IV IV'

worin die Reste $R^1$ bis $R^6$ wie in einem der Ansprüche 1 bis 3 definiert sind.

10. Picolinsäurederivate der Formel XV

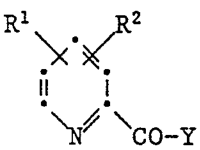

XV,

worin
Y OH; $C_1$-$C_4$-Alkoxy; oder Halogen; und
$R^1$ und $R^2$ unabhängig voneinander Halogen, Nitro; Cyano; $C_1$-$C_4$-Halogenalkyl; $C_1$-$C_4$-Alkyl; $C_1$-$C_4$-Alkoxy; oder $C_1$-$C_4$-Alkyl-$S(O)_n$; und
n 0; 1; oder 2;
bedeutet,
mit der Massgabe, dass
wenn Y Chlor bedeutet und der Rest $R^1$ in Position 3 und der Rest $R^2$ in Position 5 gebunden ist, $R^1$ und $R^2$ nicht beide zusammen Chlor oder beide zusammen Methyl oder wenn $R^1$ für Nitro steht $R^2$ nicht Methyl bedeutet.

11. Verfahren zur Herstellung von Verbindungen der Formel IV oder IV' gemäss Anspruch 9 durch die O-Acylierung eines Cyclohexandions der Formel II, worin $R^3$ bis $R^6$ wie zuvor definiert sind, mit einem Pyridin der Formel III, worin $R^1$ und $R^2$ wie zuvor definiert sind und X Halogen, vorzugsweise Chlor oder Brom, den Rest

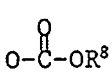

oder

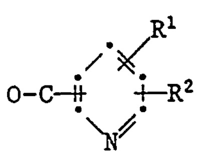

und $R^8$ $C_1$-$C_4$-Alkyl, Phenyl oder Benzyl bedeutet,

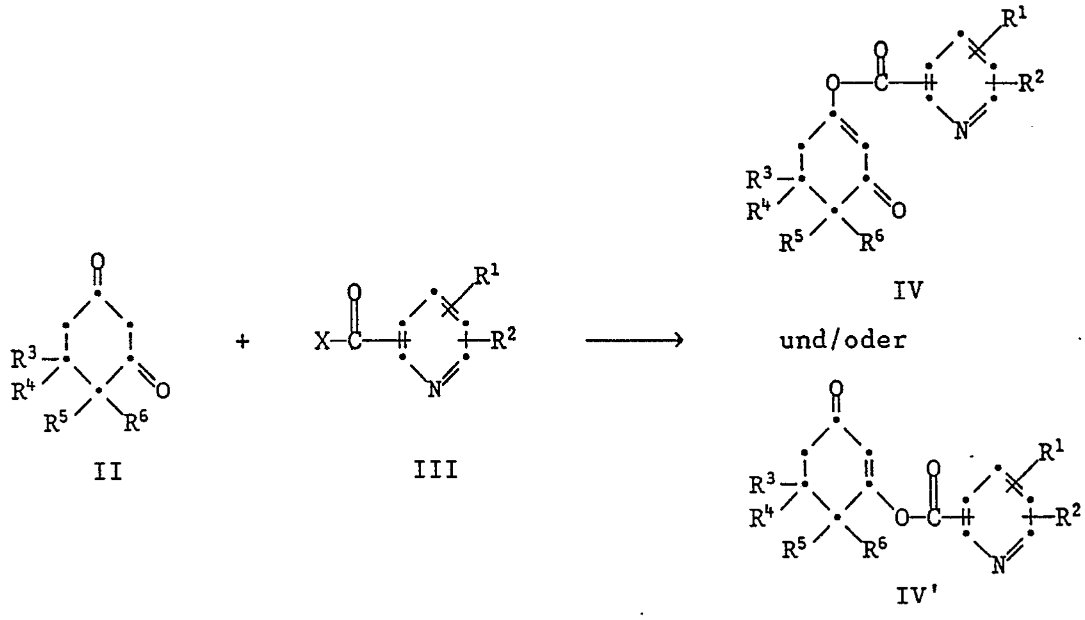

II + III → IV und/oder IV'

12. Herbizides Mittel, enthaltend eine Verbindung der Formel I gemäss einem der Ansprüche 1 bis 4 neben weiteren Hilfs- und/oder Trägerstoffen.

13. Verfahren zur Bekämpfung unerwünschten Pflanzenwuchses, dadurch gekennzeichnet, dass man eine Verbindung der Formel I gemäss Anspruch 1 bis 4 oder ein Mittel gemäss Anspruch 12 auf die zu bekämpfende Pflanze oder deren Lebensraum einwirken lässt.

14. Saatgut gekennzeichnet durch einen herbizid wirksamen Gehalt an einer Verbindung der Formel I gemäss einem der Ansprüche 1 bis 4.